(19) Europäisches Patentamt
European Patent Office
Office européen des brevets

(11) Veröffentlichungsnummer: **0 152 378**
**A2**

(12) **EUROPÄISCHE PATENTANMELDUNG**

(21) Anmeldenummer: 85810048.0

(22) Anmeldetag: 08.02.85

(51) Int. Cl.[4]: C 07 D 405/12
C 07 D 409/12, C 07 D 403/12
C 07 D 401/12, C 07 D 239/42
C 07 D 251/14, A 01 N 47/36

(30) Priorität: 14.02.84 CH 720/84
12.04.84 CH 1841/84

(43) Veröffentlichungstag der Anmeldung: 21.08.85 Patentblatt 85/34

(84) Benannte Vertragsstaaten: BE CH DE FR GB IT LI NL

(71) Anmelder: CIBA-GEIGY AG
Postfach
CH-4002 Basel(CH)

(72) Erfinder: Töpfl, Werner, Dr.
Dorneckstrasse 68
CH-4143 Dornach(CH)

(72) Erfinder: Meyer, Willy
Talweg 49
CH-4125 Riehen(CH)

(72) Erfinder: Schneider, Hans-Dieter, Dr.
Hauptstrasse 244
D-7858 Weil am Rhein(DE)

(54) **Herbizide Sulfonylharnstoffe.**

(57) Neue substituierte N-Sulfoynl-N'-pyrimidinyl- und -triazinyl-harnstoffe der Formel I haben gute pre- und postemergent-selektive herbizide und wuchsregulierende Eigenschaften

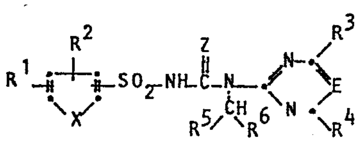

(I).

In dieser Formel bedeuten
E Stickstoff oder −CH=,
die Gruppe

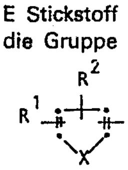

einen gemäss den Definitionen von $R^1$ und $R^2$ substituierten Phenyl-, Naphthyl-, Furyl-, Thiophenyl-, Pyrrol oder Pyridinylrest,

$R_3$ und $R_4$ Wasserstoff, Niederalkyl oder Niederalkoxy, oder eines davon auch Halogen, Niederalkenyl, Niederhalogenalkyl, Niederhalogenalkoxy, Amin, Niederalkylamin, einen Rest $-CH(OCH_3)_2$ eine ringgeschlossene Ketalgruppe oder einen über das Stickstoffatom gebundenen 5-7 gliedrigeren gesättigten Heterocyclus,

$R_5$ Wasserstoff, Niederalkyl, Niederhalogenalkyl, oder einen gegebenenfalls substituierten Phenyl-, Benzyl- oder Naphthylrest,

$R_6$ ist ein in der Beschreibung näher definiertes, über Schwefel, eine Sulfinyl- oder Sulfonylgruppe gebundener Alkyl, Acyl oder Acylamidorest oder eine über das Stickstoffatom gebundener 5-7 gliedriger heterocyclischer Aminorest.

Diese Verbindungen eignen sich vor allem zur selektiven Unkrautbekämpung in Getreidekulturen.

EP 0 152 378 A2

Croydon Printing Company Ltd.

CIBA-GEIGY AG
Basel (Schweiz)

5-14769/1+2/=

## Herbizide Sulfonylharnstoffe

Die vorliegende Erfindung betrifft neue, herbizid wirksame und pflanzenwuchsregulierende substituierte N-Sulfonyl-N'-pyridimidinyl- und triazinyl-harnstoffe und ihre Salze, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue Pyrimidin-2-yl- und Triazinyl-alkylamine.

Die erfindungsgemässen substituierten N-Sulfonyl-N'-pyrimidinyl- und triazinyl-harnstoffe entsprechen der Formel I

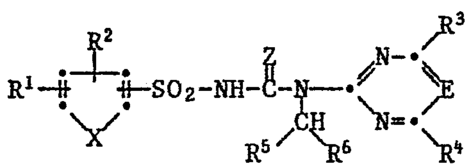

(I).

In dieser Formel bedeuten

E Stickstoff oder -CH=

X Sauerstoff, Schwefel, $-NR^7$, $-N=CR^7-$, -CH=CH- oder

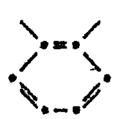

,

Y Sauerstoff oder einen Rest $S(O)_m$

m Null oder eine Zahl 1 bis 2,

Z Sauerstoff oder Schwefel,

$R^1$ Wasserstoff, Halogen, Nitro, $C_1$-$C_4$-Alkyl, $C_1$-$C_4$-Haloalkyl, $C_2$-$C_4$-Alkenyl, $C_2$-$C_4$-Haloalkenyl, $C_2$-$C_4$-Alkinyl, ein Aminorest $NR^{10}R^{11}$, ein Ketalrest $-CR^7(OC_1$-$C_4$-Alkyl$)_2$ oder $R^7COC_2$-$C_6$-Alkylen (O), einen RestTetrahydrofuran-2-yloxy oder Tetrahydropyran-2-yloxy, $-OSO_2R^8$, $-COR^8$, $-C(R^7)$=N-$OR^8$, $-SO_2NR^{10}R^{11}$, $SO_2R^8$, $YR^8$, Phenyl, Benzyl oder Phenoxy, wobei der Phenylkern unsubstituiert oder ein- oder mehrfach durch Halogen, Nitro, $C_1$-$C_4$-Alkyl, $C_1$-$C_4$-Halogenalkyl oder $C_1$-$C_4$-Alkoxy substituiert sein kann,

$R^2$ Wasserstoff, Halogen, $C_1$-$C_4$-Alkyl, $C_1$-$C_4$-Alkoxy, $C_1$-$C_4$-Halogenalkyl oder Nitro,

$R^3$ und $R^4$ unabhängig voneinander Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_2$-$C_4$-Alkoxyalkyl, $C_2$-$C_4$-Alkoxyalkoxy, Cyclopropyl, $NH_2$, $C_1$-$C_4$-Alkylamino, Di-$C_1$-$C_4$-alkylamino oder einen über das Stickstoffatom gebundenen gesättigten 5- bis 7-gliedrigen Stickstoffheterocyclus, der noch ein weiteres Heteroatom enthalten kann, einen Rest

$-CH(OCH_3)_2$ oder $R^7$-CHOC$_2$-C$_6$-Alkyl (O),

$R^5$ Wasserstoff, $C_1$-$C_4$-Alkyl, $C_1$-$C_4$-Halogenalkyl, oder einen Phenyl-, Benzyl-, Furyl-, Thienyl- oder Naphthylrest, der unsubstituiert oder ein oder mehrfach durch Halogen, Nitro, $C_1$-$C_4$-Alkyl, $C_1$-$C_4$-Halogenalkyl oder $C_1$-$C_4$-Alkoxy, substituiert ist,

$R^6$ ist ein Rest $-S(O)_mR^9$ oder eine über ein Stickstoffatom gebundener 5-7 gliedriger heterocyclischer Rest, welcher noch weitere Heteroatome im Ring haben kann, benzanneliert sein kann und welcher unsubstituiert oder durch Nitro, Halogen und/oder $C_1$-$C_4$ Alkylreste ein- oder mehrfach substituiert sein kann

$R^7$ Wasserstoff, $C_1$-$C_4$-Alkyl, $C_1$-$C_4$-Halogenalkyl, $C_5$-$C_6$-Cycloalkyl, $C_4$-$C_7$-Cycloalkylalkyl, $C_2$-$C_4$-Alkoxyalkyl, $C_2$-$C_4$-Alkenyl, $C_2$-$C_4$-Halogenalkenyl $C_5$-$C_6$-Cycloalkenyl, $C_3$-$C_4$-Alkinyl, $C_1$-$C_4$-Cyanoalkyl, $C_1$-$C_4$-Alkyl-$NR^9R^{10}$ Benzyl oder durch Halogen substituiertes Benzyl.

$R^8$ dasselbe wie $R^7$, aber nicht Wasserstoff oder $C_1$-$C_4$ Alkoxy

$R^9$ einen $C_1$-$C_4$-Alkylrest, der durch Halogen, $C_2$-$C_4$-Alkoxy, Carboxyl, $C_1$-$C_4$-Alkoxycarbonyl oder $-CONR^{10}R^{11}$ substituiert sein kann, einen Phenyl-, Benzyl- oder Naphthylrest, welcher einfach oder mehrfach durch Halogen, Nitro, Carboxyl, $C_1$-$C_4$-Alkyl, $C_1$-$C_4$-Halogenalkyl ,$C_1$-$C_4$-Alkoxy oder $C_1$-$C_4$-Halogenalkoxy substituiert sein kann, und in einem Rest $-SR^9$ kann $R^9$ auch einen Rest $-CZC_1$-$C_4$-Alkyl, $-CZOC_1$-$C_4$-Alkyl, $-CZNR^{10}R^{11}$, -CZ-Phenyl, CZ-Benzyl oder CZ-Naphthyl bedeuten, wobei diese Phenylring wie oben angegeben substituiert sein können,

$R^{10}$ und $R^{11}$ unabhängig voneinander je Wasserstoff $C_1$-$C_4$-Alkyl, $C_3$-$C_4$-Alkenyl, $C_3$-$C_4$-Alkinyl, $C_1$-$C_4$-Cyanoalkyl oder eines davon auch $C_1$-$C_4$-Alkoxy oder $C_2$-$C_4$-Alkoxyalkyl, Phenyl oder Benzyl wobei der Phenylring ein- oder mehrfach durch Halogen, Nitro, $C_1$-$C_4$-Alkyl, $C_1$-$C_4$-Halogenalkyl oder $C_1$-$C_4$-Alkoxy substituiert sein kann,

$R^{10}$ und $R^{11}$ zusammen mit dem Stickstoffatom, an das sie gebunden sind auch einen 5-7 gliedrigen gesättigten oder teilweise gesättigten Heterocyclus, der im Ring noch ein Stickstoff oder Schwefel und/oder ein oder zwei weitere Stickstoffatome enthalten kann und der durch Halogen und/oder $C_1$-$C_4$-Alkyl substituiert sein kann.

Die Erfindung umfasst auch die Salze dieser Sulfonylharnstoffe mit organischen und anorganischen Basen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldungen 44211, 44807 und 44808 sowie den britischen Patentanmeldungen 2 112 783 und 2 112 784 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl.

Unter Alkoxy ist zu verstehen: Methoxy, Aethoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Aethoxy oder i-Propyloxy.

Unter Halogen selbst in den Definition sowie Halogen als Teil in Halogenalkoxy, Halogenalkyl oder Halogenalkylthio sind Fluor, Chlor und Brom, vorzugsweise jedoch Fluor und Chlor zu verstehen.

Die unter $R^6$ definierten Reste umfassen im Rahmen der vorliegenden Erfindung beispielsweise folgende Radikale:

| | | | |
|---|---|---|---|
| $-S-C_1-C_4$-Alkyl | $-S-CH_2COOC_1-C_4$-Alkyl | Phenylthio | $-SCH_2COOH$ |
| $-SO-C_1-C_4$-Alkyl | $-S-CH_2CONHC_1-C_4$-Alkyl | Benzylthio | $-SCH(CH_3)COOH$ |
| $-SO_2C_1-C_4$-Alkyl | $-S-CH_2CON(C_1-C_4$-Alykl$)_2$ | Benzoylthio | |
| $-S-CO-C_1-C_4$-Alkyl | $-S-CS-OC_1-C_4$-Alkyl | $-SCSN(C_1-C_4$-Alkyl$)_2$ | |

ferner beispielsweise die folgenden heterocyclischen Aminoreste: 1,2,4-Triazolyl, 1,2,3,4-Tetrazol-1-yl, Pyrazol-1-yl, Imidazol-1-yl, Benztriazol-1-yl, Benzimidazol-1-yl, Indol-1-yl, Indazol-1-yl, Pyrimidin-1-yl, Pyridazin-1-yl, Pyrazin-1-yl, welche Ringe ein- bis dreifach durch Nitro, Halogen, insbesondere Chlor und Brom und/oder $C_1-C_4$-Alykl-, besonders Methylreste substituiert sein können.

Die vom Aminorest $NR^9R^{10}$ umfassten 5-7 gliedrigen gesättigten oder teilweise gesättigten heterocyclischen Reste umfassen beispielsweise folgende Radikale Pyrrolidino, Piperidino, Morpholino, Thiomorpholin, Azepino, Imdiazol-1-yl, Triazol-1-yl, 1,3-Oxazol-3-yl, 1,3-Thiazol-1-yl, Piperazin-1-yl, welche Reste unsubstituiert oder durch Halogen, insbesondere Chlor oder Brom und/oder durch $C_1-C_4$-Alkyl-, besonders Methylreste substituiert sein können.

Alkoxyalkylreste werden repräsentiert durch Methoxymethyl, Aethoxymethyl, Methoxyäthyl und Aethoxyäthyl, insbesondere aber Methoxyäthyl, Alkoxyalkoxyreste sind im Rahmen der vorliegenden Erfindung Methoxymethoxy, Alkoxymethoxy, Methoxyäthoxy und Aethoxyäthoxy. Halogenalkyl als Substituent selbst oder als Teil eines anderen Substituenten wie Halogenalkoxy oder Halogenalkylthio steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Unter den Verbindungen der Formel 1 sind diejenigen bevorzugt, in denen entweder

- Z ein Sauerstoffatom darstellt,
- der Rest 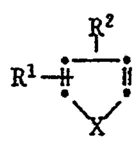 einen in der Ortho-Stellung durch $R^1$ substituierten Phenylrest darstellt,
- $R^6$ einen Rest $-S(O)_mR_8$ bedeutet,
- $R^6$ einen über das Stickstoffatom gebundenen 5-7 gliedrigen Heterocyclus, welcher noch weitere Heteroatome im Ring enthalten kann, welcher benzanneliert sein kann und welcher unsubstituiert oder durch Nitro, Halogen und/oder $C_1$-$C_4$-Alkylreste ein- oder mehrfach substituiert sein kann.

Bevorzugt sind diejenigen Verbindungen, welche z.B. der allgemeinen Formel Ic entsprechen

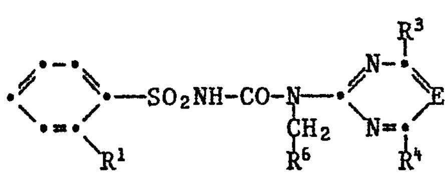

(Ic)

worin E, $R^1$, $R^3$, $R^4$ und $R^6$ die unter Formel I gegebene Bedeutung haben; ferner solche, die der allgemeinen Formel Id entsprechen

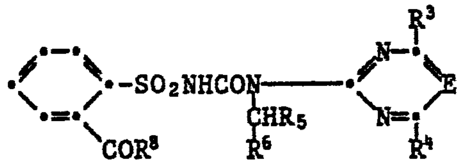

Id

worin E, $R^3$, $R^4$, $R^5$, $R^6$ und $R^8$ die unter Formel I gegebene Bedeutung haben.

Die Herstellung der Verbindungen der Formel I erfolgt beispielsweise gemäss den folgenden Methoden.

Nach einem ersten Verfahren werden die Verbindungen der Formel I erhalten, indem man ein Sulfonylisocyanat der Formel II

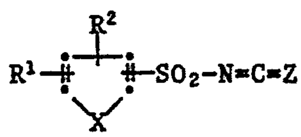

(II)

worin $R^1$, $R^2$, X und Z die unter Formel I gegebene Bedeutung haben, in einem inerten organischen Lösungs- oder Verdünnungsmittel, mit der äquivalenten Menge eines 2-Pyrimidin- oder 2-Triazinamines der Formel III umsetzt

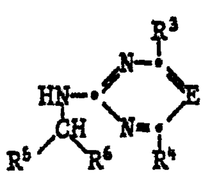

(III)

worin E, $R^3$, $R^4$, $R^5$ und $R^6$ die unter Formel I gegebene Bedeutung haben und gewünschtenfalls den Harnstoff als Salz isoliert oder ihn mit einer anorganischen oder organischen Base in ein Salz überführt.

Nach einem zweiten Verfahren erhält man die Verbindungen der Formel I, indem man ein Sulfonylamid der Formel IV

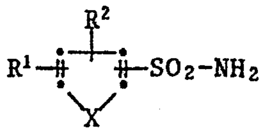

(IV)

worin $R^1$, $R^2$ und X die unter Formel I gegebene Bedeutung haben, in einem inerten organischen Lösungs- oder Verdünnungsmittel, in Gegenwart einer Base mit der äquivalenten Menge eines 2-Pyrimidinyl- oder 2-Triazinylcarbamoylhalides der Formel V umsetzt

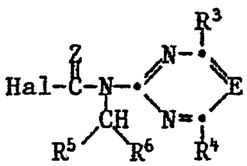

(V)

worin Hal für ein Halogenatom, vorzugsweise Chlor oder Brom steht und E, $R^3$, $R^4$, $R^5$, $R^6$ und Z die unter Formel I gegebene Bedeutung hat und gewünschtenfalls den Harnstoff als Salz isoliert oder ihn mit einer anorganischen oder organischen Base in ein Salz überführt.

Gemäss einem dritten Verfahren stellt man die Verbindungen der Formel I her, indem man einen Sulfonylharnstoff der Formel VI

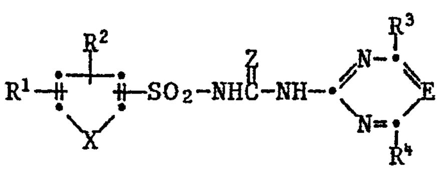

(VI)

worin E, $R^1$, $R^2$, $R^3$, $R^4$, X und Z die unter Formel I gegebene Bedeutung haben, in einem inerten organischen Lösungs- oder Verdünnungsmittel, in Gegenwart einer Base, mit der äquivalenten Menge eines Methylhalides der Formel VII umsetzt

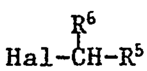

(VII)

worin Hal ein Halogenatom, vorzugsweise Chlor oder Brom bedeutet und $R^5$ und $R^6$ die unter Formel I gegebene Bedeutung haben und gewünschtenfalls den Harnstoff als Salz isoliert oder ihn mit einer anorganischen oder organischen Base in ein Salz überführt.

Die erhaltenen Harnstoffe der Formel I können gegebenenfalls mittels Aminen, Alkalimetall- oder Erdalkalimetallhydroxiden oder quaternären Ammoniumbasen in basische Additionssalze übergeführt werden. Dies geschieht beispielsweise durch Umsetzen mit der äquimolaren Menge Base und Verdampfen des Lösungsmittels. Solche Umsetzungen sind bekannt und beispielsweise in den US Patentschriften Nr. 2 384 757 und 3 410 887 beschrieben.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo-(2,2,2)-octan. 1,5-Diazabicyclo(4,3,0)non-5-en oder 1,5-Diazabicyclo(5,4,0)undec-7-en geeignet. Als Basen können aber auch anorganische Basen wie Hydride wie Natrium- oder Calciumhydrid, Hydroxyide wie Natrium- und

Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Neben diesen allgemeinen Methoden zur Herstellung der Verbindungen der Formel I kommen je nach der Bedeutung der Substituenten $R^5$ und $R^6$ auch weitere Synthesewege in Betracht, die sich jeweils nur für eine spezifische Gruppe von Endprodukten eignet. Solche Synthesen werden in den folgenden Schemata beispielsweise wiedergegeben. In den nachstehenden Formeln haben E, m, $R^1$, $R^2$, $R^3$, $R^4$, $R^5$, $R^6$, $R^8$, X und Z die unter Formel I gegebene Bedeutung. Die römischen Zahlen I, II und IV stehen für die unter diesen Zahlen oben gegebenen Formeln.

<u>Schema 1</u>

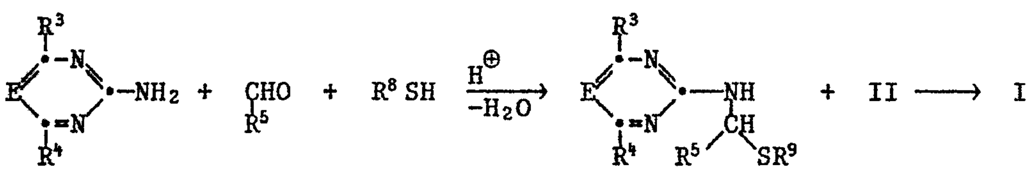


VIII                                   IIIa

Ein 2-Aminopyrimidin resp. ein 2-Amino-1,3,5-triazin der Formel VIII wird mit einem Aldehyd zur Schiff'schen Base kondensiert und diese in situ mit der Merkaptoverbindung umgesetzt. Die Reaktion wird bei erhöhter Temperatur z.B. dem Siedepunkt des Reaktionsgemisches in einem organischen Lösungsmittel oder Lösungsmittelgemisch wie z.B. einem Alkanol und/oder einem aromatischen Kohlenwasserstoff durchgeführt werden. Zur Beschleunigung der Reaktion wird eine kleine Menge eines Säurekatalysators zugegeben. Das Beispiel 1 beschreibt die Herstellung einer Verbindung der Formel IIIa, in der Tabelle 1 werden weitere aufgeführt. Diese Zwischenprodukte sind neu und

bilden einen Bestandteil dieser Erfindung. Die Kondensationsprodukte der Formel IIIa werden dann gemäss dem ersten Herstellungsverfahren mit einem Sulfonylisocyanat der Formel II umgesetzt wie in den Beispielen 9-11 beschrieben.

Schema 2

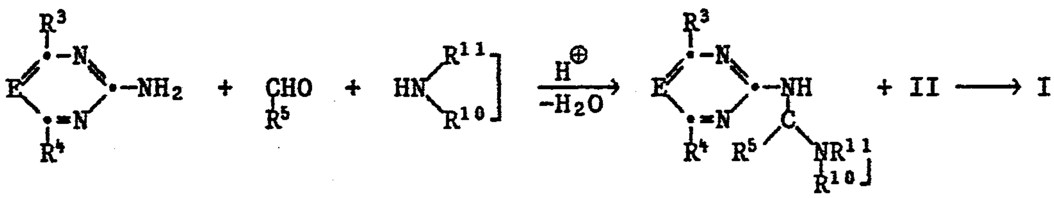


Auch hier wird das 2-Aminopyrimidin resp. -triazin der Formel VIII mit einem Aldehyd zur Schiff'schen Base kondensiert und diese in situ mit einem cyclischen sekundären Amin umgesetzt. Die Reaktion geschieht in Gegenwart einer kleinen Menge Säure in einem organischen Lösungsmittel. Die Beispiele 5-8 beschrieben solche Umsetzungen zu Verbindungen der Formel IIIb. Diese können dann mit einem Sulfonylisocyanat der Formel II zum Harnstoff der Formel I weiter umgesetzt werden, wie in den Beispielen 12 und 13 beschrieben.

Schema 3

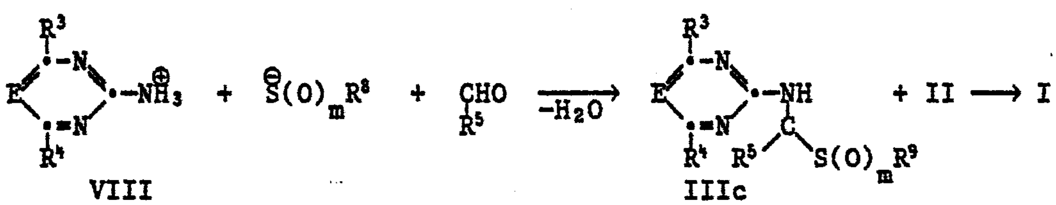


Das 2-Aminopyrimidin resp. -triazin der Formel VIII wird mit einer Sulfinsäure in einem organischen Lösungs- oder Verdünnungsmittel vorgelegt, wozu man dann unter Erwärmen den Aldehyd gibt. Das Kondensationsprodukt kann durch Verdünnen des Reaktionsgemisches mit Wasser ausgefällt werden. Eine solche Reaktion ist im Beispiel 2 beschrieben. Weitere Verbindungen sind in der Tabelle 2 aufgeführt. Die Zwischenprodukte der Formel IIIc bilden ebenfalls Gegenstand

dieser Erfindung. Die weitere Reaktion der Verbindung der Formel IIIc mit einem Sulfonylisocyanat der Formel II zum Harnstoff der Formel I erfolgt in bekannter Weise.

Eine Verbindung der Formel IIIa kann auch durch Oxidation in eine Verbindung der Formel IIIc überführt werden

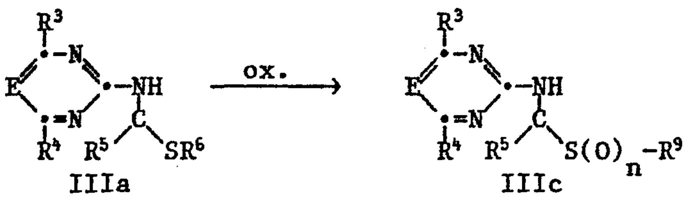


IIIa IIIc

In diesen Formeln bedeutet n die Zahl 1 oder 2.

Die Oxidation findet in einem inerten organischen Lösungs- oder Verdünnungsmittel statt bei Temperaturen die zwischen 0°C und dem Siedepunkt des Reaktionsgemisches liegen. Als Oxidationsmittel kommen Wasserstoffperoxid, organische Persäuren wie z.B. Perbenzoesäure oder Peressigsäure oder auch Kaliumpermanganat oder Chromoxid in Frage. Die Dauer der Oxidation und die Menge des zu verwendenden Oxidationsmittels hängt von der gewünschten Oxidationsstufe der Verbindung der Formel IIIc ab.

Schema 4

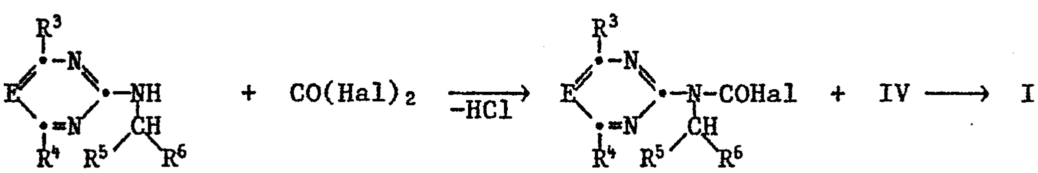


III Va

Dieses Schema entspricht einigermassen dem oben beschriebenen zweiten Verfahren zur Herstellung der Sulfonylharnstoffe der Formel I, ausser, dass man in einer Vorstufe in einem inerten organischen Lösungsmittel durch Kondensation der Verbindung der Formel III mit einem Kohlensäuredihalogenid (Phosgen) zuerst ein

N-(2-Pyrimidinyl)-N-methyl-carbamoylhalid oder N-(2-Triazinyl)-N-methyl-carbamoylhalid der Formel Va herstellt, und dieses dann mit dem Sulfonamid der Formel IV zum Harnstoff der Formel I umsetzt.

Schema 5

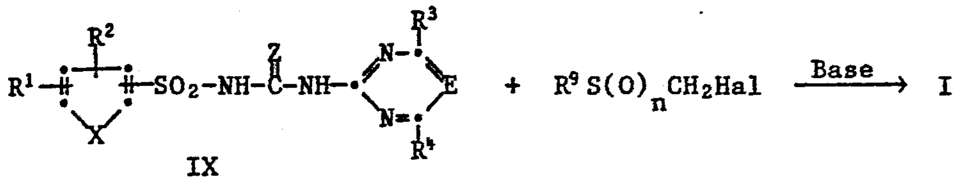


IX

Ein Sulfonylharnstoff der Formel IV wird in einem inerten organischen Lösungsmittel in Gegenwart einer Base mit einem Methylhalid zum Harnstoff der Formel I umgesetzt. Die Reaktion findet bei Raumtemperatur statt. Zur Beschleunigung kann man sie für kurze Zeit aufwärmen, bis zum Siedepunkt des Reaktionsgemisches.

Schema 6

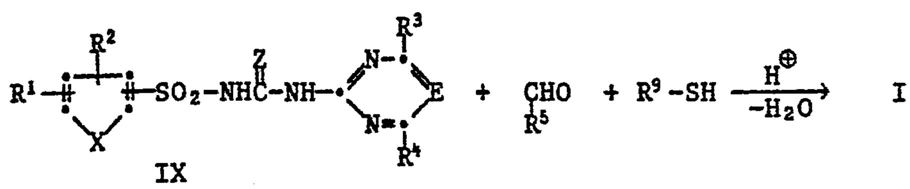


IX

Dieses Schema gleicht dem Schema 1 ausser dass man als Ausgangsprodukt einen Sulfonylharnstoff der Formel IX verwendet, diesen dann in einem inerten organischen Lösungs- oder Verteilungsmittel in Gegenwart einer kleinen Menge Säure mit einem Aldehyd und einem Mercaptan umsetzt. Der so erhaltene Sulfonylharnstoff der Formel Ia kann durch eine Oxydation analog derjenigen welche anschliessend an das Schema III beschrieben ist zur entsprechenden Sulfinyl- oder Sulfonylverbindung Ib oxydiert werden.

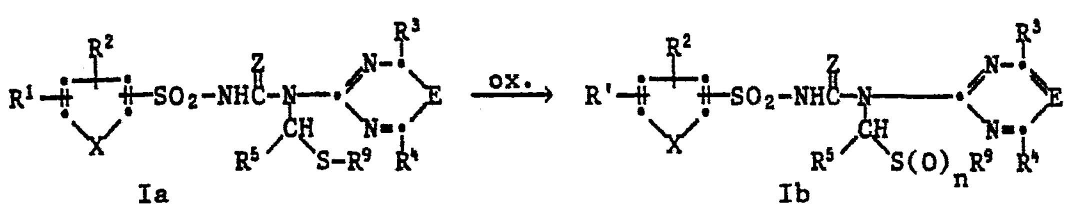


Ia Ib

Schema 7

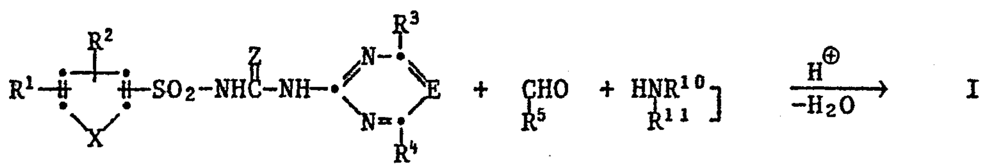

Dieses Schema ist analog dem Schema 2 ausser dass als Ausgangsstoff ein Sulfonylharnstoff der Formel IX verwendet wird, der dann in einem inerten organischen Lösungs- oder Verteilungsmittel mit einem Aldehyd und einem cyclischen sekundären Amin, in Gegenwart einer geringen Menge einer Säure und bei erhöhter Temperatur zum Sulfonylharnstoff der Formel I umsetzt.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglichen Massnahme.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter

bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I oder ein Salz davon enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I und ihre Salze werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzgut die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen

wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und

einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder

niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die herbiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

<u>Emulgierbare Konzentrate</u>:

| | | |
|---|---|---|
| Aktiver Wirkstoff: | 1 bis 20 %, bevorzugt | 5 bis 10 % |
| oberflächenaktive Mittel: | 5 bis 30 %, vorzugsweise | 10 bis 20 % |
| flüssiges Trägermittel: | 50 bis 94 %, vorzugsweise | 70 bis 85 % |

<u>Stäube</u>:

| | | |
|---|---|---|
| Aktiver Wirkstoff: | 0,1 bis 10 %, vorzugsweise | 0,1 bis 1 % |
| festes Trägermittel: | 99,9 bis 90 %, vorzugsweise | 99,9 bis 99 % |

<u>Suspensions-Konzentrate</u>:

| | | |
|---|---|---|
| Aktiver Wirkstoff: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 % |

Benetzbares Pulver:

| | | |
|---|---|---|
| Aktiver Wirkstoff: | 0,5 bis 90 %, | vorzugsweise 1 bis 80 % |
| oberflächenaktives Mittel: | 0,5 bis 20 %, | vorzugsweise 1 bis 15 % |
| festes Trägermittel: | 5 bis 95 %, | vorzugsweise 15 bis 90 % |

Granulate:

| | | |
|---|---|---|
| Aktiver Wirkstoff: | 0,5 bis 30 %, | vorzugsweise 3 bis 15 % |
| festes Trägermittel: | 99,5 bis 70 %, | vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den nachfolgenden Beispielen sind die Temperaturen in Celsiusgraden angegeben, Prozente und Angaben von Teilen beziehen sich auf das Gewicht.

Beispiel 1: Herstellung von 2-[(4-Chlorphenylthio)methylamino]-4-methoxy-6-methylpyrimidin (Zwischenprodukt)

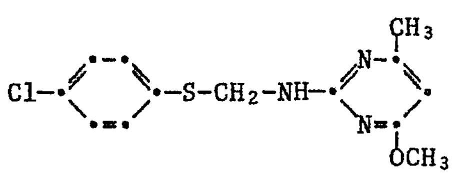

Verbindung 1.02

Ein Gemisch von 13,9 g 2-Amino-4-methoxy-6-methylpyrimidin (0,1 Mol), 14,8 g 4-Chlor-thiophenol (0,1 Mol), 9,4 g wässriges Formaldehyd 35 % (0,11 Mol) und 1 g Essigsäure werden in 100 ml

Methanol während einer Stunde am Rückfluss erhitzt. Die dabei entstehende Lösung wird klarfiltriert, mit 150 ml Toluol verdünnt und dann werden die Lösungsmittel am Rotationsverdampfer entfernt. Der Rückstand kristallisiert nach längerem Stehen. Der Schmelzpunkt ist 78-82° nach Umkristallisation aus Hexan. Die Ausbeute beträgt 24 g (81 % der Theorie).

Gemäss diesem Beispiel 1 werden folgende Verbindungen hergestellt

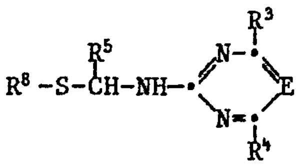

(IIIa)

Tabelle 1

| No. | $R^3$ | $R^4$ | $R^5$ | E | $R^8$ | Schmelzpunkt |
|---|---|---|---|---|---|---|
| 1.01 | $CH_3$ | $CH_3$ | H | CH | 4-Chlorphenyl | 110-113° |
| 1.02 | $OCH_3$ | $CH_3$ | H | CH | 4-Chlorphenyl | 78-82° |
| 1.03 | Cl | $OCH_3$ | H | CH | 4-Chlorphenyl | 102-105° |
| 1.04 | $OCH_3$ | $OCH_3$ | H | CH | 4-Chlorphenyl | 112-114° |
| 1.05 | $CH_3$ | $CH_3$ | H | CH | 4-Fluorphenyl | 96-98° |
| 1.06 | $OCH_3$ | $CH_3$ | H | CH | 2,4,5-Trichlorphenyl | 117-119° |
| 1.07 | $OCH_3$ | $CH_3$ | H | CH | 4-Acetylaminophenyl | 182-183° |
| 1.08 | $CH_3$ | $CH_3$ | H | CH | ß-Naphthyl | 115-118° |
| 1.09 | $OCH_3$ | $CH_3$ | H | CH | ß-Naphthyl | 101-102° |
| 1.10 | $CH_3$ | $CH_3$ | H | CH | Carbanilidomethyl | 128-130° |
| 1.11 | Cl | $OCH_3$ | H | CH | p-Tolyl | 106-109° |
| 1.12 | $CH_3$ | $CH_3$ | H | CH | $-CH_2-COOH$ | 123-125° (Zers.) |
| 1.13 | $CH_3$ | $OCH_3$ | H | CH | $-CH_2-COOH$ | 160-164° |
| 1.14 | $CH_3$ | $CH_3$ | H | CH | $-CH(CH_3)COOH$ | 141-145° |
| 1.15 | $CH_3$ | $CH_3$ | H | CH | $-CH_2CH_2-COOH$ | 150-153° |
| 1.16 | $CH_3$ | $CH_3$ | H | CH | $-CH_2-COOCH_3$ | 71-73° |
| 1.17 | $CH_3$ | $OCH_3$ | H | CH | $-CH_2-COOCH_3$ | 85-87° |
| 1.18 | $CH_3$ | $OCH_3$ | H | CH | N-Phenylcarbamoyl-methyl | 161-163° |
| 1.19 | $OCH_3$ | $OCH_3$ | H | CH | 4-Tolyl | 84-91° |

Tabelle 1 (Fortsetzung)

| No. | $R^3$ | $R^4$ | $R^5$ | E | $R^8$ | Schmelzpunkt |
|---|---|---|---|---|---|---|
| 1.20 | $OCH_3$ | $CH_2-OCH_3$ | H | CH | 2,4,5-Trichlorphenyl | 124-126° |
| 1.21 | $CH_3$ | $CH_3$ | $CH_3$ | CH | 2-Carboxylphenyl | 162-163° |
| 1.22 | $CH_3$ | $CH_3$ | $C_3H_7(i)$ | CH | 2-Carboxylphenyl | 138-141° |
| 1.23 | $CH_3$ | $CH_3$ | $-CH_2-Cl$ | CH | 2-Carboxylphenyl | 220-222° (Zers.) |
| 1.24 | $CH_3$ | $CH_3$ | -COOH | CH | 2-Carboxylphenyl | 180-182° (Zers.) |
| 1.25 | $CH_3$ | $CH_3$ | 2-Furyl | CH | 2-Carboxylphenyl | 169-171° (Zers.) |
| 1.26 | $CH_3$ | $CH_3$ | 4-Chlor-phenyl | CH | 2-Carboxylphenyl | 193-195° (Zers.) |
| 1.27 | $CH_3$ | $CH_3$ | H | CH | 2-Carboxylphenyl | 226-228° (Zers.) |

Beispiel 2: Herstellung von 4-Dimethylamino-6-methoxy-2-(4-tolyl-sulfonyl)methylamino-1,3,5-triazin

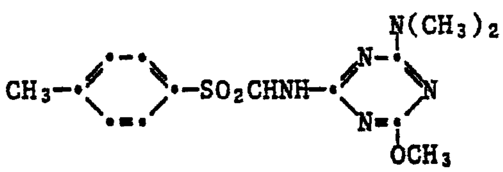

Verbindung 2.05

Man suspendiert in 100 ml Methanol 8,5 g 2-Amino-4-dimethylamino-6-methoxy-1,3,5-triazin (0,05 Mol) und 9,1 g Toluol-4-sulfinsäure-Natriumsalz (0,05 Mol) und gibt zur gerührten Suspension nacheinander 4,8 g Methansulfonsäure (0,05 Mol) und 4.7 g wässriges Formaldehyd 35 % (0,055 Mol) zu. Das Ganze wird dann während 30 Minuten bei Raumtemperatur und weitere 90 Minuten unter Rückfluss

gerührt. Nach dem Erkalten wird mit 1 l Wasser verdünnt, und das kristallin anfallende Produkt abgenutscht und getrocknet. Schmelzpunkt 188-190°, Ausbeute 10 g (59 % der Theorie).

Beispiel 3: Herstellung von 4,6-Dimethyl-2-(4-Tolylsulfonylmethylamino)-pyrimidin

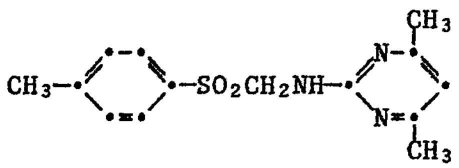

Verbindung 2.02

Man suspendiert 12,7 g 2-Amino-4,6-dimethylpyrimidin (0,1 Mol) in 200 ml Wasser und gibt unter Rühren 9,7 g Methansulfonsäure (0,1 g) zu wobei sich alles löst. Zur gerührten Lösung werden nacheinander 18,2 g Toluol-3-sulfonsäure-Natriumsalz (0,1 Mol) und 9,4 g wässriges Formaldehyd 35 % (0,11 Mol) gegeben. Danach wird 2 Stunden bei Raumtemperatur und weitere 30 Minuten bei 90° weitergerührt. Das Produkt fällt dabei als farbloser Niederschlag aus, der nach dem Erkalten abgenutscht und getrocknet wird. Schmelzpunkt 191-194°, Ausbeute 25 g (86 % der Theorie).

In analoger Weise wie in den Beispielen 2 und 3 werden folgende Verbindungen erhalten.

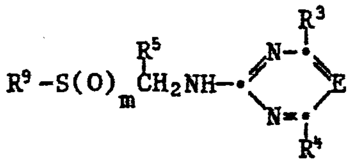

(IIIc)

Tabelle 2

| No. | $R^3$ | $R^4$ | $R^5$ | m | E | $R^9$ | Schmelzpunkt |
|---|---|---|---|---|---|---|---|
| 2.01 | $CH_3$ | $CH_3$ | H | 2 | CH | $CH_3$ | 145-148 |
| 2.02 | $CH_3$ | $CH_3$ | H | 2 | CH | p-Tolyl | 191-194° |
| 2.03 | $OCH_3$ | $CH_3$ | H | 2 | CH | p-Tolyl | 136-138° |
| 2.04 | $OCHF_2$ | $CH_3$ | H | 2 | CH | p-Tolyl | 165-166° |
| 2.05 | $N(CH_3)_2$ | $OCH_3$ | H | 2 | N | p-Tolyl | 188-190° |
| 2.06 | $OCH_3$ | $OCH_3$ | H | 2 | CH | p-Tolyl | 135-140° |
| 2.07 | $OCH_3$ | $CH_2COH_3$ | H | 2 | CH | p-Tolyl | 142-144° |
| 2.08 | Cl | $OCH_3$ | H | 2 | CH | p-Tolyl | |
| 2.09 | Cl | $CH_3$ | H | 2 | CH | p-Tolyl | |
| 2.10 | $CH_3$ | $CH_3$ | H | 2 | CH | 4-Chlorphenyl | 178-180° |
| 2.11 | $OCH_3$ | $CH_3$ | H | 2 | CH | 4-Chlorphenyl | |
| 2.12 | $CH_3$ | $CH_3$ | H | 2 | CH | 4-Bromphenyl | 184-186° |
| 2.13 | $CH_3$ | $OCH_3$ | H | 2 | CH | $CH_3$ | 149-150° |
| 2.14 | $CH_3$ | $CH_3$ | H | 2 | CH | Benzyl | 154-159° |
| 2.15 | $CH_3$ | $OCH_3$ | H | 2 | CH | ß-Naphthyl | 167-169° |
| 2.16 | $CH_3$ | $CH_3$ | $CH_3$ | 2 | CH | p-Tolyl | 125-127° |
| 2.17 | $CH_3$ | $CH_3$ | $C_3H_7i$ | 2 | CH | p-Tolyl | 116-118° |
| 2.18 | $OCH_3$ | $OCH_3$ | H | 2 | CH | $CH_3$ | 149-151° |
| 2.19 | $OCH_3$ | $OCH_3$ | H | 2 | CH | $C_2H_5$ | 145-147° |
| 2.20 | $OCH_3$ | $OCH_3$ | H | 2 | CH | $n-C_3H_7$ | 117-120° |

Tabelle 2 (Fortsetzung)

| No. | $R^3$ | $R^4$ | $R^5$ | m | E | $R^9$ | Schmelzpunkt |
|---|---|---|---|---|---|---|---|
| 2.21 | $OCH_3$ | $OCH_3$ | H | 2 | CH | Benzyl | 138-139° |
| 2.22 | $OCH_3$ | $OCH_3$ | H | 2 | CH | 2-Methoxy-carbonylbenzyl | 135-136° |
| 2.23 | $OCH_3$ | $OCH_3$ | H | 2 | CH | Phenyl | 135-139° |
| 2.24 | $OCH_3$ | $OCH_3$ | H | 2 | CH | 2-Difluor-methoxyphenyl | 137-138° |
| 2.25 | $OCH_3$ | $OCH_3$ | H | 2 | CH | 2,4,6-Tri-methylphenyl | 131-133° |
| 2.26 | $OCH_3$ | $OCH_3$ | H | 2 | CH | 4-Bromphenyl | 137-138° |
| 2.27 | $OCH_3$ | $OCH_3$ | H | 2 | CH | 4-Chlorphenyl | 140-141° |
| 2.28 | $OCH_3$ | $OCH_3$ | H | 2 | CH | β-Naphthyl | 139-140° |
| 2.29 | $OCHF_2$ | $CH_3$ | H | 2 | CH | $CH_3$ | 162-163°(Z) |
| 2.30 | $N(CH_3)_2$ | $OCH_3$ | H | 2 | CH | $CH_3$ | 184-187°(Z) |

Beispiel 4: Herstellung von 4,6-Dimethyl-2-(morpholin-4-ylthio-carbonyl-thiomethylamino)-pyrimidin

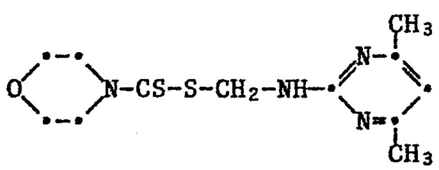

Verbindung 3.02

Man löst 6,6 g 85 % Kaliumhydroxyd (0,1 Mol) und 8,9 g Morpholin (0,1 Mol) in 200 ml Wasser und gibt unter Kühlen 7,7 g Schwefelkohlenstoff (0,1 Mol) zu und rührt so lange, bis eine gelbliche homogene Lösung entsteht. Dazu gibt man nacheinander unter Kühlen und Rühren 9,4 g wässriges Formaldehyd 35 % (0,11 Mol) und eine Lösung von 12,7 g 2-Amino-4,6-dimethylpyrimidin (0,1 Mol) und 9,7 g Methansulfonsäure (0,1 Mol) in 50 ml Wasser. Das Reaktionsgemisch wird bei Raumtemperatur weitergerührt worauf sich zuerst ein Oel abscheidet, das nach kurzer Zeit kristallisiert. Nach 24 Stunden

wird mit 1 l Wasser verdünnt, der kristalline Niederschlag abgenutscht und getrocknet. Man erhält 24 g Titelprodukt (80 % der Theorie) welches bei 119-121° schmilzt.

Gemäss dem Beispiel 4 wurden folgende Verbindungen hergestellt:

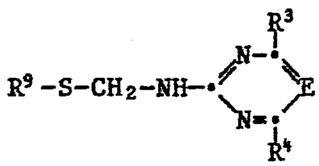

Tabelle 3

| No. | $R^3$ | $R^4$ | E | $R^9$ | Schmelzpunkt |
|---|---|---|---|---|---|
| 3.01 | $CH_3$ | $CH_3$ | CH | Pyrrolidinothiocarbonyl | 116-118° |
| 3.02 | $CH_3$ | $CH_3$ | CH | Morpholinothiocarbonyl | 119-121° |
| 3.03 | Cl | $OCH_3$ | CH | Pyrrolidinothiocarbonyl | 136-139° |
| 3.04 | $CH_3$ | $CH_3$ | CH | Azepinylthiocarbonyl | 90-92° |
| 3.05 | $CH_3$ | $CH_3$ | CH | Methoxythiocarbonyl | |
| 3.06 | $CH_3$ | $CH_3$ | CH | Dimethylthiocarbamoyl | 105-115° |
| 3.07 | $CH_3$ | $CH_3$ | CH | Acetyl | |
| 3.08 | $CH_3$ | $CH_3$ | CH | Benzoyl | > 110° Z |
| 3.09 | $OCHF_2$ | $CH_3$ | CH | Benzoyl | 125-128° Z |
| 3.10 | $CH_3$ | $OCH_3$ | CH | Benzoyl | 107-109° |

Beispiel 5: Herstellung von 4-Methoxy-6-methyl-2-(1,2,4-triazol-1-ylmethylamino)-pyrimidin

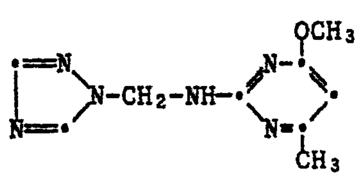

Verbindung 4.02

Ein Gemisch von 13,9 g 2-Amino-4-methoxy-6-methylpyrimidin (0,1 Mol), 7 g 1,2,4-Triazol (0,1 Mol), 9,4 g wässriges Formaldehyd 35 % (0,11 Mol) und 1 g Essigsäure in 100 ml Methanol wird unter Rühren während 1 1/2 Stunden am Rückfluss gekocht. Zuerst entsteht eine klare Lösung, aus der nach einiger Zeit das Reaktionsprodukt kristallin ausfällt. Nach dem Erhalten des Reaktionsgemisches wird das kristalline Titelprodukt abgenutscht und getrocknet. Ausbeute 14 g (63 % der Theorie) Schmelzpunkt 191-193°.

Beispiel 6: Herstellung von 2-[(2,6-Dichlorphenyl)-(1,2,4-triazol-1-yl)-methyl]-amino-4,6-dimethylpyrimidin

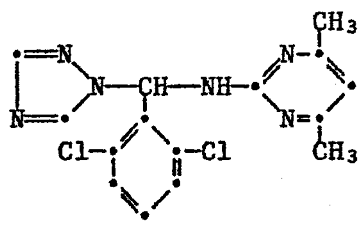

Ein Gemisch von 6,3 g 2-Amino-4,6-dimethylpyrimidin (0,05 Mol), 8,8 g 2,6-Dichlorbenzaldehyd (0,05 Mol), 3,5 g 1,2,4-Triazol (0,05 Mol) und 1 g Essigsäure in 50 ml Methanol wird unter Rühren während 2 Stunden am Rückfluss erhitzt. Dabei entsteht eine klare gelbliche Lösung. Diese wird am Rotationsverdampfer eingeeengt und der Rückstand aus Methanol kristallisiert. Man erhält so 6 g Titelprodukt (34 % der Theorie) welches sich bei 140-145° umwandelt um dann bei 158-159° zu schmelzen.

Beispiel 7: Herstellung von 2-(Benztriazol-1-yl-methylamino)-4-dimethylamino-6-methoxy-1,3,5-triazin

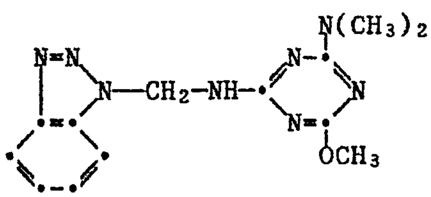

Verbindung 4.14

Ein Gemisch von 16,9 g 2-Amino-4-dimethylamino-6-methoxy-1,3,5-triazin (0,1 Mol), 12 g Benztriazol (0,1 Mol), 9,4 g wässrigem Formaldehyd 35 % (0,11 Mol) und 1 g Essigsäure in 100 ml Aethanol wird unter Rühren während 2 Stunden am Rückfluss erhitzt. Die noch heisse Lösung wird klarfiltriert und das Titelprodukt fällt beim Erhalten der Lösung kristallin aus. Nach Abnutschen und Trocknen erhält man 25 g kristallines Produkt (83 % der Theorie) mit einem Schmelzpunkt 222-224°.

<u>Beispiel 8</u>: Herstellung von 4,6-Dimethyl-2-[(2-Methylbenzimidazol-1-yl)-methylamino]-pyrimidin

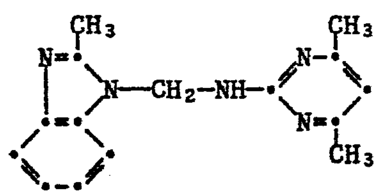

Verbindung 4.16

Ein Gemisch von 6,3 g 2-Amino-4,6-dimethylpyrimidin (0,05 Mol) , 6,7 g 2-Methylbenzimidazol (0,05 Mol), 4,7 g wässriges Formaldehyd 35 % (0, 055 Mol) 1 g Essigsäure in 50 ml Methanol wird während 1 1/2 Stunden unter Rühren am Rückfluss erhitzt. Dabei entsteht eine farblose Lösung. Man gibt 150 ml Toluol dazu und entfernt dann die Lösungsmittel am Rotationsverdmapfer. Der Rückstand fällt dabei kristallin aus. Man kristallisiert ihn aus Essigester/Methanol um und erhält so 10 g Titelprodukt (70 % der Theorie) welches bei 207-212° schmilzt.

In analoger Weise zu den Beispielen 5 bis 8 werden folgende Verbindungen erhalten

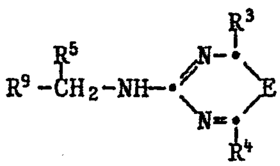

Tabelle 4

| No. | $R^3$ | $R^4$ | $R^5$ | E | $R^9$ | Schmelzpunkt |
|---|---|---|---|---|---|---|
| 4.01 | $CH_3$ | $CH_3$ | H | CH | 1,2,4-Triazol 1-yl | 162-164° |
| 4.02 | $OCH_3$ | $CH_3$ | H | CH | 1,2,4-Triazol-1-yl | 191-193° |
| 4.03 | $OCH_3$ | $OCH_3$ | H | CH | 1,2,4-Triazol-1-yl | 166-168° |
| 4.04 | $OCHF_2$ | $CH_3$ | H | CH | 1,2,4-Triazol-1-yl | 187-189° |
| 4.05 | $OCH_3$ | $CH_2OCH_3$ | H | CH | 1,2,4-Triazol-1-yl | 180-181° |
| 4.06 | Cl | $OCH_3$ | H | CH | 1,2,4-Triazol-1-yl | 157-159° |
| 4.07 | $OCH_3$ | $OCH_3$ | H | N | 1,2,4-Triazol-1-yl | 197-200° (Z) |
| 4.08 | $N(CH_3)_2$ | $OCH_3$ | H | N | 1,2,4-Triazol-1-yl | 206-208° |
| 4.09 | $CH_3$ | $CH_3$ | H | CH | Benztriazol-1-yl | 204-205° |
| 4.10 | $OCH_3$ | $CH_3$ | H | CH | Benztriazol-1-yl | 165-167° |
| 4.11 | $OCH_3$ | $OCH_3$ | H | CH | Benztriazol-1-yl | 168-170° |
| 4.12 | $OCH_3$ | $CH_2OCH_3$ | H | CH | Benztriazol-1-yl | 132-134° |
| 4.13 | $OCHF_2$ | $CH_3$ | H | CH | Benztriazol-1-yl | 142-144° |

| No. | $R^3$ | $R^4$ | $R^5$ | E | $R^8$ | Schmelzpunkt |
|---|---|---|---|---|---|---|
| 4.14 | $CH_3$ | $OCH_3$ | H | CH | 5(6)-Nitro-benz-triazol-1-yl | 190-193° *) |
| 4.15 | $OCH_3$ | $OCH_3$ | H | CH | 5(6)-Nitro-benz-triazol-1-yl | 173-175° *) |
| 4.16 | $CH_3$ | $OCH_3$ | H | CH | 5(6)-Chlor-benz-triazol-1-yl | 156-168° *) |
| 4.17 | $N(CH_3)_2$ | $OCH_3$ | H | N | Benztriazol-1-yl | 222-224° |
| 4.18 | $CH_3$ | $CH_3$ | H | CH | 4aH-Carbazol-1-yl | 153-155° |
| 4.19 | $CH_3$ | $CH_3$ | H | CH | 2-Methyl-benz-imidazol-1-yl | 207-212° |
| 4.20 | $CH_3$ | $CH_3$ | H | CH | 2,3-Dimethylindol-1-yl | |
| 4.21 | $CH_3$ | $OCH_3$ | H | N | 2,3-Dimethylindol-1-yl | |
| 4.22 | $OCHF_2$ | $CH_3$ | H | CH | 2,3-Dimethylindol-1-yl | |
| 4.23 | $CH_3$ | $CH_3$ | H | CH | 2,4,5-Tribromimid-azol-1-yl | |
| 4.24 | $CH_3$ | $CH_3$ | H | CH | 2,4,5-Tribromimid-azol-1-yl | |
| 4.25 | $N(CH_3)_2$ | $OCH_3$ | H | N | 2,4,5-Tribromimid-azol-1-yl | |
| 4.26 | $CH_3$ | $OCH_3$ | H | CH | Pyrazol-1-yl | |
| 4.27 | $OCHF_2$ | $CH_3$ | H | CH | Pyrazol-1-yl | |
| 4.28 | Cl | $OCH_3$ | H | N | Pyrazol-1-yl | |
| 4.29 | $CH_3$ | $OCH_3$ | $CH_3$ | CH | 1,2,4-Triazol-1-yl | |

*) Isomeren-Gemisch

Beispiel 9; Herstellung von N-2-Chlorphenylsulfonyl-N'-(4-chlorphenylthiomethyl)-N'-(4,6-dimethylpyrimidin-2-yl)-harnstoff

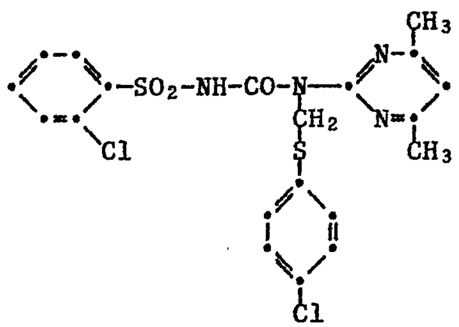

Verbindung 5.124

Man suspendiert 7.5 g 2-(4-Chlorphenylthio)-methylamino-4,6-dimethylpyrimidin (0,027 Mol) in 50 ml absolutem Acetonitril und gibt gibt 5.8 g 2-Chlorphenylisocyanat (0,027 Mol) dazu. Das Ganze wird 24 Stunden bei Raumtemperatur gerührt, anschliessend wird das Lösungsmittel am Rotationsverdampfer entfernt und der Rückstand in Essigester aufgenommen und zur Reinigung mit Essigester/Cyclohexan über eine Silicagelkolonne chromatographiert. Das Titelprodukt fällt nach Verdampfen der Lösungsmittel kristallin aus. Ausbeute 6.0 g (45 % der Theorie), Schmelzpunkt 132-135°.

Beispiel 10: Herstellung von N-(2-Difluormethoxyphenylsulfonyl)-N'-(p-tolylsulfonylmethyl)-N'-(4-difluormethoxy-6-methylpyrimidin-2-yl)-harnstoff

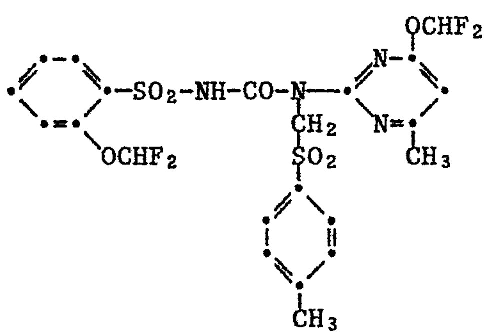

Verbindung 8.025

Man suspendiert 5,5 g 4-Difluormethoxy-6-methyl-2-p-tolylsulfonylmethylamino-pyrimidin (0,016 Mol) in 50 ml absolutem Dioxan und gibt unter Rühren 4 g 2-Difluormethoxyphenylisocyanat (0,016 Mol) zu und rührt 3 Stunden bei Raumtemperatur weiter. Dabei entsteht eine klare Lösung, die man im Rotationsverdampfer eineengt. Der Rückstand kristallisiert spontan, wenn man ihn mit Aether verrührt. Nach Abnutschen und Trocknen erhält man 8 g kristallines Titelprodukt (84 % der Theorie) vom Schmelzpunkt 159-164° (Z).

Beispiel 11: Herstellung von N-(2-Chlorphenylsulfonyl)-N'-(pyrrolidinothiocarbonylthiomethyl)-N'-(4,6-dimethylpyrimidin-2-yl)-harnstoff

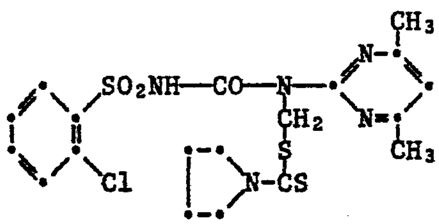

Verbindung 5.165

Man suspendiert 5,5 g 2-(Pyrrolidinothiocarbonylthiomethyl)-4,6-dimethylpyrimidin (0,019 Mol) in 30 ml absolutem Acetonitril und gibt unter Rühren 4,3 g 2-Chlorphenylsulfonylisocyanat (0,019 Mol) dazu. Das Ganze wird während 5 Stunden bei Raumtemperatur gerührt, wobei zunächst eine klare Lösung entsteht aus der nach einiger Zeit das Endprodukt ausfällt. Das Reaktionsgemisch wird gekühlt und dann abgenutscht. Man erhält so 6 g Titelprodukt (63 % der Theorie) welches bei 131-133° unter Zersetzung schmilzt.

Beispiel 12: Herstellung von N-(2-Chlorphenylsulfonyl)-N'-(1,2,4-Triazol-1-ylmethyl)-N'-(4,6-dimethylpyrimidin-2-yl)-harnstoff

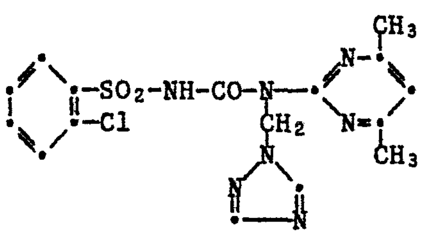

Verbindung 10.006

Man suspendiert 4 g 2-(1,2,4-Triazol-1-ylmethylamino)-4,6-dimethyl-pyrimidin (0,019 Mol) in 100 ml absolutem Acetonitril und gibt unter Rühren 4,3 g 2-Chlorphenylsulfonylisocyanat (0.019 Mol) zu. Das Ganze wird während 24 Stunden bei Raumtemperatur gerührt und die entstandene Lösung schliesslich am Rotationsverdampfer eingeengt. Das Titelprodukt fällt dabei kristallin an und wird aus Essigester umkristalliseirt. Man erhält so 5 g des obigen Harnstoffes (62 % der Theorie) welcher bei 152-157° schmilzt.

Beispiel 13: Herstellung von N-(2-Methoxycarbonylphenylsulfonyl)-N'-(benztriazol-1-ylmethyl)-N'-(4,6-dimethylpyrimidin-2-yl)-harnstoff

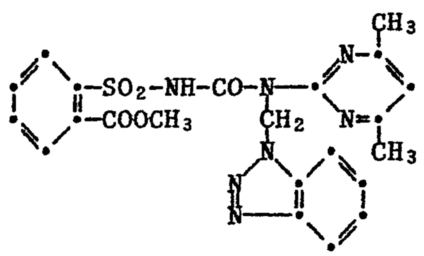

Verbindung 10.001

Man suspendiert 6,5 g 2-(Benztriazol-1-yl-methylamino)-4,6-dimethyl-pyrimidin (0,026 Mol) in 70 ml absolutem Dioxan und gibt unter Rühren 6,2 g 2-Methoxycarbonylphenylsulfonylisocyanat (0,026 Mol) zu. Das Ganze wird während 24 Stunden bei Raumtemperatur gerührt. Die entstandene Lösung wird am Rotationsverdampfer eingeengt und der Rückstand in Essigester aufgenommen und durch Chromatographie mit Essigester/Cyclohexan über eine Silikagelkolonne gereinigt. Nach Verdampfen des Lösungsmittels erhält man 7.5 g kristallines Titelprodukt (58 % der Theorie) vom Schmelzpunkt 178-180° (Z).

In analoger Weise zu den Beispielen 9-13 werden folgende Verbindungen hergestellt:

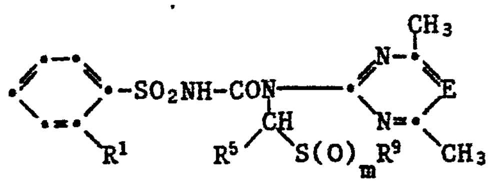

Tabelle 5

| No | $R^1$ | $R^5$ | m | $R^9$ | E | Smp. |
|---|---|---|---|---|---|---|
| 5.001 | $COOCH_3$ | H | 2 | p-Tolyl | CH | 159-160° |
| 5.002 | $COOCH_3$ | H | 0 | p-Tolyl | CH | |
| 5.003 | $COOCH_3$ | H | 2 | 4-Chlorphenyl | CH | |
| 5.004 | $COOCH_3$ | H | 0 | 4-Chlorphenyl | CH | 141-144° |
| 5.005 | $COOCH_3$ | H | 0 | Acetyl | CH | |
| 5.006 | $COOCH_3$ | H | 0 | Benzoyl | CH | |
| 5.007 | $COOCH_3$ | H | 2 | $CH_3$ | CH | 130-135°(Z) |
| 5.008 | $COOCH_3$ | H | 2 | $CH_2CH_2CH_2CH_3$ | CH | |
| 5.009 | $COOCH_3$ | H | 2 | $CH(CH_3)_2$ | CH | |
| 5.010 | $COOCH_3$ | H | 2 | Benzyl | CH | |
| 5.011 | $COOCH_3$ | H | 2 | Phenyl | CH | |
| 5.012 | $COOCH_3$ | H | 0 | Phenyl | CH | |
| 5.013 | $COOCH_3$ | H | 2 | 4-Bromphenyl | CH | |
| 5.014 | $COOCH_3$ | H | 0 | 4-Bromphenyl | CH | |
| 5.015 | $COOCH_3$ | H | 2 | 2-Chlorphenyl | CH | |
| 5.016 | $COOCH_3$ | H | 0 | 2-Chlorphenyl | CH | |
| 5.017 | $COOCH_3$ | H | 0 | $CH_3$ | CH | |
| 5.018 | $COOCH_3$ | H | 0 | $CH_2CH_2CH_2CH_3$ | CH | |
| 5.019 | $COOCH_3$ | H | 0 | $CH(CH_3)_2$ | CH | |

Tabelle 5 (Fortsetzung)

| No | $R^1$ | $R^5$ | m | $R^9$ | E | Smp. |
|---|---|---|---|---|---|---|
| 5.020 | $COOCH_3$ | H | 0 | Benzyl | CH | |
| 5.021 | $COOCH_3$ | H | 2 | ß-Naphtyl | CH | |
| 5.022 | $COOCH_3$ | H | 0 | ß-Naphtyl | CH | 178-183°(Z) |
| 5.023 | $COOCH_3$ | H | 2 | 1,2,4-Trichlor-phenyl | CH | |
| 5.024 | $COOCH_3$ | H | 0 | 1,2,4-Trichlor-phenyl | CH | |
| 5.025 | $COOCH_3$ | H | 2 | α-Naphtyl | CH | |
| 5.026 | $COOCH_3$ | H | 2 | α-Naphtyl | CH | |
| 5.027 | $COOCH_3$ | H | 0 | $-CH_2COOCH_3$ | CH | |
| 5.028 | $COOCH_3$ | H | 2 | 4-Fluorphenyl | CH | |
| 5.029 | $COOCH_3$ | H | 0 | 4-Fluorphenyl | CH | |
| 5.030 | $COOCH_3$ | H | 0 | 4-Nitrophenyl | CH | |
| 5.031 | $COOCH_3$ | H | 0 | 4-Nitrophenyl | CH | |
| 5.032 | $COOCH_3$ | H | 2 | 2-Nitrophenyl | CH | |
| 5.033 | $COOCH_3$ | H | 0 | 2-Nitrophenyl | CH | |
| 5.034 | $COOCH_3$ | H | 2 | 2-Trifluormethyl | CH | |
| 5.035 | $COOCH_3$ | H | 0 | 2-Trifluormethyl | CH | |
| 5.036 | $COOCH_3$ | H | 2 | 4-Methoxyphenyl | CH | |
| 5.037 | $COOCH_3$ | H | 0 | 4-Methoxyphenyl | CH | |
| 5.038 | $COOCH_3$ | H | 2 | 2-Methoxyphenyl | CH | |
| 5.039 | $COOCH_3$ | H | 0 | 2-Methoxyphenyl | CH | |
| 5.040 | $COOCH_3$ | H | 2 | 2,4,6-Trimethyl-phenyl | CH | |
| 5.041 | $COOCH_3$ | H | 0 | 2,4,6-Trimethyl-phenyl | CH | |

Tabelle 5 (Fortsetzung)

| No | $R^1$ | $R^5$ | m | $R^9$ | E | Smp. |
|---|---|---|---|---|---|---|
| 5.042 | $COOCH_3$ | H | 0 | $CS-N(CH_3)_2$ | CH | |
| 5.043 | $COOCH_3$ | H | 0 | $CSN(CH_2CH_3)_2$ | CH | |
| 5.044 | $COOCH_3$ | H | 0 | $CSN(CH_2CH_3)_2$ | CH | |
| 5.045 | $COOCH_3$ | H | 0 | Pyrrolidinothio-carbonyl | CH | |
| 5.046 | $COOCH_3$ | H | 0 | Piperidinothio-carbonyl | CH | |
| 5.047 | $COOCH_3$ | H | 0 | Anilidoacetyl | CH | |
| 5.048 | $COOCH_3$ | H | 0 | 2,4-Dichlorphenyl | CH | |
| 5.049 | $COOCH_3$ | H | 2 | 2,4-Dichlorphenyl | CH | |
| 5.050 | $COOCH_3$ | H | 0 | $CH_2COOH$ | CH | |
| 5.051 | $COOCH_3$ | H | 0 | $CH_2COO\ CH_3$ | CH | 154-156° |
| 5.052 | $COOCH_3$ | H | 0 | $CH_2CON(CH_3)_2$ | CH | |
| 5.053 | $COOCH_3$ | H | 0 | 4-Chlorphenyl | N | |
| 5.054 | $COOCH_3$ | H | 0 | p-Tolyl | N | |
| 5.055 | $COOCH_3$ | Furyl-2- | 0 | 2-Hydroxy-carbonylphenyl | CH | |
| 5.056 | $COOCH_3$ | Furyl-2- | 0 | 2-Methoxy-carbonylphenyl | CH | |
| 5.057 | $COOCH_3$ | Furyl-2- | 0 | Phenyl | CH | |
| 5.058 | $COOCH_3$ | 4-Chlor-phenyl | 0 | 2-Hydroxy-carbonylphenyl | CH | |
| 5.059 | $COOCH_3$ | 4-Chlor-phenyl | 0 | Phenyl | CH | |
| 5.060 | $COOCH_3$ | $CH_3$ | 0 | 4-Chlorphenyl | CH | |

Tabelle 5 (Fortsetzung)

| No | $R^1$ | $R^5$ | m | $R^9$ | E | Smp. |
|---|---|---|---|---|---|---|
| 5.061 | $OCHF_2$ | H | 2 | p-Tolyl | CH | 152-157°(Z) |
| 5.062 | $OCHF_2$ | H | 0 | p-Tolyl | CH | |
| 5.063 | $OCHF_2$ | H | 2 | 4-Chlorphenyl | CH | |
| 5.064 | $OCHF_2$ | H | 0 | 4-Chlorphenyl | CH | 109-111° |
| 5.065 | $OCHF_2$ | H | 0 | Acetyl | CH | |
| 5.066 | $OCHF_2$ | H | 0 | Benzoyl | CH | 155-158° Zers. |
| 5.067 | $OCHF_2$ | H | 2 | $CH_3$ | CH | |
| 5.068 | $OCHF_2$ | H | 2 | $CH_2CH_2CH_2CH_3$ | CH | |
| 5.069 | $OCHF_2$ | H | 2 | $CH(CH_3)_2$ | CH | |
| 5.070 | $OCHF_2$ | H | 2 | Benzyl | CH | |
| 5.071 | $OCHF_2$ | H | 2 | Phenyl | CH | |
| 5.072 | $OCHF_2$ | H | 0 | Phenyl | CH | |
| 5.073 | $OCHF_2$ | H | 2 | 4-Bromphenyl | CH | |
| 5.074 | $OCHF_2$ | H | 0 | 4-Bromphenyl | CH | |
| 5.075 | $OCHF_2$ | H | 2 | 2-Chlorphenyl | CH | |
| 5.076 | $OCHF_2$ | H | 0 | 2-Chlorphenyl | CH | |
| 5.077 | $OCHF_2$ | H | 0 | $CH_3$ | CH | |
| 5.078 | $OCHF_2$ | H | 0 | $CH_2CH_2CH_2CH_3$ | CH | |
| 5.079 | $OCHF_2$ | H | 0 | $CH(CH_3)_2$ | CH | |
| 5.080 | $OCHF_2$ | H | 0 | Benzyl | CH | |
| 5.081 | $OCHF_2$ | H | 2 | ß-Naphtyl | CH | |
| 5.082 | $OCHF_2$ | H | 0 | ß-Naphtyl | CH | 162-164° |
| 5.083 | $OCHF_2$ | H | 2 | 1,2,4-Trichlor-phenyl | CH | |

Tabelle 5 (Fortsetzung)

| No | $R^1$ | $R^5$ | m | $R^9$ | E | Smp. |
|---|---|---|---|---|---|---|
| 5.084 | $OCHF_2$ | H | 0 | 1,2,4-Trichlor-phenyl | CH | |
| 5.085 | $OCHF_2$ | H | 2 | α-Naphtyl | CH | |
| 5.086 | $OCHF_2$ | H | 2 | α-Naphtyl | CH | |
| 5.087 | $OCHF_2$ | H | 0 | $-CH_2COOCH_3$ | CH | |
| 5.088 | $OCHF_2$ | H | 2 | 4-Fluorphenyl | CH | |
| 5.089 | $OCHF_2$ | H | 0 | 4-Fluorphenyl | CH | 125-127° |
| 5.090 | $OCHF_2$ | H | 0 | 4-Nitrophenyl | CH | |
| 5.091 | $OCHF_2$ | H | 0 | 4-Nitrophenyl | CH | |
| 5.092 | $OCHF_2$ | H | 2 | 2-Nitrophenyl | CH | |
| 5.093 | $OCHF_2$ | H | 0 | 2-Nitrophenyl | CH | |
| 5.094 | $OCHF_2$ | H | 2 | 2-Trifluormethyl | CH | |
| 5.095 | $OCHF_2$ | H | 0 | 2-Trifluormethyl | CH | |
| 5.096 | $OCHF_2$ | H | 2 | 4-Methoxyphenyl | CH | |
| 5.097 | $OCHF_2$ | H | 0 | 4-Methoxyphenyl | CH | |
| 5.098 | $OCHF_2$ | H | 2 | 2-Methoxyphenyl | CH | |
| 5.099 | $OCHF_2$ | H | 0 | 2-Methoxyphenyl | CH | |
| 5.100 | $OCHF_2$ | H | 2 | 2,4,6-Trimethyl-phenyl | CH | |
| 5.101 | $OCHF_2$ | H | 0 | 2,4,6-Trimethyl-phenyl | CH | |
| 5.102 | $OCHF_2$ | H | 0 | $CS-N(CH_3)_2$ | CH | 139-142° Zers. |
| 5.103 | $OCHF_2$ | H | 0 | $CSN(CH_2CH_3)_2$ | CH | |
| 5.104 | $OCHF_2$ | H | 0 | $CSN(CH_2CH_3)_2$ | CH | |
| 5.105 | $OCHF_2$ | H | 0 | Pyrrolidinothio-carbonyl | CH | 136-139° Zers. |

Tabelle 5 (Fortsetzung)

| No | $R^1$ | $R^5$ | m | $R^9$ | E | Smp. |
|---|---|---|---|---|---|---|
| 5.106 | $OCHF_2$ | H | 0 | Piperidinothio-carbonyl | CH | |
| 5.107 | $OCHF_2$ | H | 0 | N-Phenylcarbamoyl-methyl | CH | 110-112° |
| 5.108 | $OCHF_2$ | H | 0 | 2,4-Dichlorphenyl | CH | |
| 5.109 | $OCHF_2$ | H | 2 | 2,4-Dichlorphenyl | CH | |
| 5.110 | $OCHF_2$ | H | 0 | $CH_2COOH$ | CH | |
| 5.111 | $OCHF_2$ | H | 0 | $CH_2COO\ CH_3$ | CH | |
| 5.112 | $OCHF_2$ | H | 0 | $CH_2CON(CH_3)_2$ | CH | |
| 5.113 | $OCHF_2$ | H | 0 | 4-Chlorphenyl | N | |
| 5.114 | $OCHF_2$ | H | 0 | p-Tolyl | N | |
| 5.115 | $OCHF_2$ | Furyl-2- | 0 | 2-Hydroxy-carbonylphenyl | CH | |
| 5.116 | $OCHF_2$ | Furyl-2- | 0 | 2-Methoxy-carbonylphenyl | CH | |
| 5.117 | $OCHF_2$ | Furyl-2- | 0 | Phenyl | CH | |
| 5.118 | $OCHF_2$ | 4-Chlor-phenyl | 0 | 2-Hydroxy-carbonylphenyl | CH | |
| 5.119 | $OCHF_2$ | 4-Chlor-phenyl | 0 | Phenyl | CH | |
| 5.120 | $OCHF_2$ | $CH_3$ | 0 | 4-Chlorphenyl | CH | |
| 5.121 | Cl | H | 2 | p-Tolyl | CH | 153-155° |
| 5.122 | Cl | H | 0 | p-Tolyl | CH | 86-88(Z) |
| 5.123 | Cl | H | 2 | 4-Chlorphenyl | CH | |
| 5.124 | Cl | H | 0 | 4-Chlorphenyl | CH | 132-135° Zers. |
| 5.125 | Cl | H | 0 | Acetyl | CH | |
| 5.126 | Cl | H | 0 | Benzoyl | CH | |
| 5.127 | Cl | H | 2 | $CH_3$ | CH | |

Tabelle 5 (Fortsetzung)

| No | $R^1$ | $R^5$ | m | $R^9$ | E | Smp. |
|---|---|---|---|---|---|---|
| 5.128 | Cl | H | 2 | $CH_2CH_2CH_2CH_3$ | CH | |
| 5.129 | Cl | H | 2 | $CH(CH_3)_2$ | CH | |
| 5.130 | Cl | H | 2 | Benzyl | CH | 146-147°(Z) |
| 5.131 | Cl | H | 2 | Phenyl | CH | |
| 5.132 | Cl | H | 0 | Phenyl | CH | |
| 5.133 | Cl | H | 2 | 4-Bromphenyl | CH | 150-153°(Z) |
| 5.134 | Cl | H | 0 | 4-Bromphenyl | CH | |
| 5.135 | Cl | H | 2 | 2-Chlorphenyl | CH | |
| 5.136 | Cl | H | 0 | 2-Chlorphenyl | CH | |
| 5.137 | Cl | H | 0 | $CH_3$ | CH | |
| 5.138 | Cl | H | 0 | $CH_2CH_2CH_2CH_3$ | CH | |
| 5.139 | Cl | H | 0 | $CH(CH_3)_2$ | CH | |
| 5.140 | Cl | H | 0 | Benzyl | CH | |
| 5.141 | Cl | H | 2 | ß-Naphtyl | CH | |
| 5.142 | Cl | H | 0 | ß-Naphtyl | CH | 156-158° |
| 5.143 | Cl | H | 2 | 1,2,4-Trichlor-phenyl | CH | |
| 5.144 | Cl | H | 0 | 1,2,4-Trichlor-phenyl | CH | |
| 5.145 | Cl | H | 2 | α-Naphtyl | CH | |
| 5.146 | Cl | H | 2 | α-Naphtyl | CH | |
| 5.147 | Cl | H | 0 | $-CH_2COOCH_3$ | CH | |
| 5.148 | Cl | H | 2 | 4-Fluorphenyl | CH | |
| 5.149 | Cl | H | 0 | 4-Fluorphenyl | CH | |
| 5.150 | Cl | H | 0 | 4-Nitrophenyl | CH | |
| 5.151 | Cl | H | 0 | 4-Nitrophenyl | CH | |

Tabelle 5 (Fortsetzung)

| No | $R^1$ | $R^5$ | m | $R^9$ | E | Smp. |
|---|---|---|---|---|---|---|
| 5.152 | Cl | H | 2 | 2-Nitrophenyl | CH | |
| 5.153 | Cl | H | 0 | 2-Nitrophenyl | CH | |
| 5.154 | Cl | H | 2 | 2-Trifluormethyl | CH | |
| 5.155 | Cl | H | 0 | 2-Trifluormethyl | CH | |
| 5.156 | Cl | H | 2 | 4-Methoxyphenyl | CH | |
| 5.157 | Cl | H | 0 | 4-Methoxyphenyl | CH | |
| 5.158 | Cl | H | 2 | 2-Methoxyphenyl | CH | |
| 5.159 | Cl | H | 0 | 2-Methoxyphenyl | CH | |
| 5.160 | Cl | H | 2 | 2,4,6-Trimethyl-phenyl | CH | |
| 5.161 | Cl | H | 0 | 2,4,6-Trimethyl-phenyl | CH | |
| 5.162 | Cl | H | 0 | $CS-N(CH_3)_2$ | CH | |
| 5.163 | Cl | H | 0 | $CSN(CH_2CH_3)_2$ | CH | |
| 5.164 | Cl | H | 0 | $CSN(CH_2CH_3)_2$ | CH | |
| 5.165 | Cl | H | 0 | Pyrrolidinothio-carbonyl | CH | 131-133° Zers. |
| 5.166 | Cl | H | 0 | Piperidinothio-carbonyl | CH | |
| 5.167 | Cl | H | 0 | N-Phenylcarbamoyl-methyl | CH | 110-112° |
| 5.168 | Cl | H | 0 | 2,4-Dichlorphenyl | CH | |
| 5.169 | Cl | H | 2 | 2,4-Dichlorphenyl | CH | |
| 5.170 | Cl | H | 0 | $CH_2COOH$ | CH | 123-125°(Z) |
| 5.171 | Cl | H | 0 | $CH_2COO\ CH_3$ | CH | |
| 5.172 | Cl | H | 0 | $CH_2CON(CH_3)_2$ | CH | |

Tabelle 5 (Fortsetzung)

| No | $R^1$ | $R^5$ | m | $R^9$ | E | Smp. |
|---|---|---|---|---|---|---|
| 5.173 | Cl | H | 0 | 4-Chlorphenyl | N | |
| 5.174 | Cl | H | 0 | p-Tolyl | N | |
| 5.175 | Cl | Furyl-2- | 0 | 2-Hydroxy-carbonylphenyl | CH | |
| 5.176 | Cl | Furyl-2- | 0 | 2-Methoxy-carbonylphenyl | CH | |
| 5.177 | Cl | Furyl-2- | 0 | Phenyl | CH | |
| 5.178 | Cl | 4-Chlor-phenyl | 0 | 2-Hydroxy-carbonylphenyl | CH | |
| 5.179 | Cl | 4-Chlor-phenyl | 0 | Phenyl | CH | |
| 5.180 | Cl | $CH_3$ | 0 | 4-Chlorphenyl | CH | |
| 5.181 | $OCH_3$ | H | 0 | Phenyl | CH | |
| 5.182 | $OCH_3$ | H | 2 | Phenyl | CH | |
| 5.183 | $OCH_3$ | H | 0 | $CSN(CH_3)_2$ | CH | |
| 5.184 | $OCH_3$ | H | 0 | Pyrrolidinothio-carbonyl | CH | |
| 5.185 | $OCH_3$ | H | 0 | $CH_3$ | CH | |
| 5.186 | $OCH_3$ | H | 2 | $CH_3$ | CH | |
| 5.187 | $NO_2$ | H | 0 | Phenyl | CH | |
| 5.188 | $NO_2$ | H | 2 | Phenyl | CH | |
| 5.189 | $NO_2$ | H | 0 | $CSN(CH_3)_2$ | CH | |
| 5.190 | $NO_2$ | H | 0 | Pyrrolidinothio-carbonyl | CH | |
| 5.191 | $NO_2$ | H | 0 | $CH_3$ | CH | |
| 5.192 | $NO_2$ | H | 2 | $CH_3$ | CH | |
| 5.193 | $CF_3$ | H | 0 | Phenyl | CH | |

Tabelle 5 (Fortsetzung)

| No | $R^1$ | $R^5$ | m | $R^9$ | E | Smp. |
|---|---|---|---|---|---|---|
| 5.194 | $CF_3$ | H | 2 | Phenyl | CH | |
| 5.195 | $CF_3$ | H | 0 | $CSN(CH_3)_2$ | CH | |
| 5.196 | $CF_3$ | H | 0 | Pyrrolidino-thiocarbonyl | CH | |
| 5.197 | $CF_3$ | H | 0 | $CH_3$ | CH | |
| 5.198 | $CF_3$ | H | 2 | $CH_3$ | CH | |
| 5.199 | $OSO_2CH_3$ | H | 0 | Phenyl | CH | |
| 5.200 | $OSO_2CH_3$ | H | 2 | Phenyl | CH | |
| 5.201 | $OSO_2CH_3$ | H | 0 | $CSN(CH_3)_2$ | CH | |
| 5.202 | $OSO_2CH_3$ | H | 0 | Pyrrolidino-thiocarbonyl | CH | |
| 5.203 | $OSO_2CH_3$ | H | 0 | $CH_3$ | CH | |
| 5.204 | $OSO_2CH_3$ | H | 2 | $CH_3$ | CH | |
| 5.205 | $OSO_2C_3H_7n$ | H | 2 | Phenyl | CH | |
| 5.206 | $OSO_2C_3H_7n$ | H | 2 | Phenyl | CH | |
| 5.207 | $OSO_2C_3H_7n$ | H | 0 | $CSN(CH_3)_2$ | CH | |
| 5.208 | $OSO_2C_3H_7n$ | H | 0 | Pyrrolidino-thiocarbonyl | CH | |
| 5.209 | $OSO_2C_3H_7n$ | H | 2 | $CH_3$ | CH | |
| 5.210 | $OSO_2C_3H_7n$ | H | 0 | $CH_3$ | CH | |
| 5.211 | $SO_2N(CH_3)_2$ | H | 0 | Phenyl | CH | |
| 5.212 | $SO_2N(CH_3)_2$ | H | 2 | Phenyl | CH | |
| 5.213 | $SO_2N(CH_3)_2$ | H | 0 | $CSN(CH_3)_2$ | CH | |
| 5.214 | $SO_2N(CH_3)_2$ | H | 0 | Pyrrolidino-thiocarbonyl | CH | |
| 5.215 | $SO_2N(CH_3)_2$ | H | 0 | $CH_3$ | CH | |

Tabelle 5 (Fortsetzung)

| No | $R^1$ | $R^5$ | m | $R^9$ | E | Smp. |
|---|---|---|---|---|---|---|
| 5.216 | $SO_2N(CH_3)_2$ | H | 2 | $CH_3$ | CH | |
| 5.217 | $CH_3$ | H | 0 | Phenyl | CH | |
| 5.218 | $CH_3$ | H | 2 | Phenyl | CH | |
| 5.219 | $CH_3$ | H | 0 | $CSN(CH_3)_2$ | CH | |
| 5.220 | $CH_3$ | H | 0 | Pyrrolidino-thiocarbonyl | CH | |
| 5.221 | $CH_3$ | H | 0 | $CH_3$ | CH | |
| 5.222 | $CH_3$ | H | 2 | $CH_3$ | CH | |
| 5.223 | $SO_2CH_3$ | H | 0 | Phenyl | CH | |
| 5.224 | $SO_2CH_3$ | H | 2 | Phenyl | CH | |
| 5.225 | $SO_2CH_3$ | H | 0 | $CSN(CH_3)_2$ | CH | |
| 5.226 | $SO_2CH_3$ | H | 0 | Pyrrolidino-thiocarbonyl | CH | |
| 5.227 | $SO_2CH_3$ | H | 0 | $CH_3$ | CH | |
| 5.228 | $SO_2CH_3$ | H | 2 | $CH_3$ | CH | |
| 5.229 | $CH_3$ | H | 2 | p-Tolyl | CH | 148-150° Zers. |
| 5.230 | $CH_3$ | H | 0 | Morpholino-thiocarbonyl | CH | 145-146° Zers. |
| 5.231 | $OCHF_2$ | H | 0 | Morpholino-thiocarbonyl | CH | 132-134° Zers. |
| 5.232 | $OCHF_2$ | H | 0 | 4-Fluorphenyl | CH | 125-127° |
| 5.233 | $COOCH_3$ | H | 0 | 4-Fluorphenyl | CH | |
| 5.234 | $COOCH_3$ | H | 2 | 4-Fluorphenyl | CH | |

Tabelle 5 (Fortsetzung)

| No. | $R^1$ | $R^5$ | m | $R^9$ | E | Smp. |
|---|---|---|---|---|---|---|
| 5.235 | $CH_3$ | H | 2 | Benzyl | CH | 146-148° Zers. |
| 5.236 | $CH_3$ | H | 2 | 4-Bromphenyl | CH | 141-143° Zers. |
| 5.237 | Cl | $CH_3$ | 2 | p-Tolyl | CH | 126-129° Zers. |
| 5.238 | Cl | H | 0 | 2-Carboxyphenyl | CH | 134-138° Zers. |
| 5.239 | 2,6-$Cl_2$ | H | 2 | Benzyl | CH | 147-151° Zers. |
| 5.240 | 2,6-$Cl_2$ | H | 0 | ß-Naphthyl | CH | 149-152° |
| 5.241 | 2,6-$Cl_2$ | H | 2 | p-Tolyl | CH | 163-166 Zers. |
| 5.242 | Phenyl | H | 2 | $CH_3$ | CH | 162-166° Zers. |
| 5.243 | Phenyl | H | 2 | p-Tolyl | CH | 154-157° Zers. |
| 5.244 | Phenyl | H | 0 | $-CS-N(CH_3)_2$ | CH | 143-145° Zers. |
| 5.245 | Phenyl | H | 0 | $-CH_2-COOCH_3$ | CH | 172-174° Zers. |
| 5.246 | Phenyl | H | 0 | $-CH_2-CH_2-COOH$ | CH | 155-157 Zers. |
| 5.247 | Phenyl | H | 0 | $-CH(CH_3)COOH$ | CH | 150-152° Zers. |
| 5.248 | Cl | H | 2 | Phenyl | N | |
| 5.249 | Cl | H | 2 | p-Tolyl | N | |
| 5.250 | $COOCH_3$ | H | 2 | $CH_3$ | N | |
| 5.251 | $OCHF_2$ | H | 2 | p-Tolyl | N | |
| 5.252 | $NO_2$ | H | 0 | $C_2H_4COOH$ | CH | 121-124° |
| 5.253 | $CF_3$ | H | 0 | 4-Chlorphenyl | CH | 118-121° |

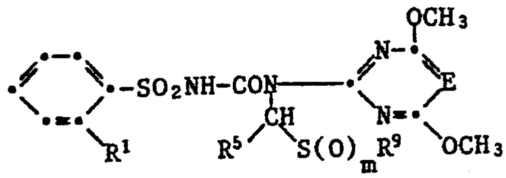

Tabelle 6

| No | $R^1$ | $R^5$ | m | $R^9$ | E | Smp. |
|---|---|---|---|---|---|---|
| 6.001 | $COOCH_3$ | H | 2 | p-Tolyl | CH | 148-150°(Z) |
| 6.002 | $COOCH_3$ | H | 0 | p-Tolyl | CH | |
| 6.003 | $COOCH_3$ | H | 2 | 4-Chlorphenyl | CH | |
| 6.004 | $COOCH_3$ | H | 0 | 4-Chlorphenyl | CH | |
| 6.005 | $COOCH_3$ | H | 0 | Acetyl | CH | |
| 6.006 | $COOCH_3$ | H | 0 | Benzoyl | CH | |
| 6.007 | $COOCH_3$ | H | 2 | $CH_3$ | CH | |
| 6.008 | $COOCH_3$ | H | 2 | $CH_2CH_2CH_2CH_3$ | CH | |
| 6.009 | $COOCH_3$ | H | 2 | $CH(CH_3)_2$ | CH | |
| 6.010 | $COOCH_3$ | H | 2 | Benzyl | CH | 136-137°(Z) |
| 6.011 | $COOCH_3$ | H | 2 | Phenyl | CH | 141-142°(Z) |
| 6.012 | $COOCH_3$ | H | 0 | Phenyl | CH | |
| 6.013 | $COOCH_3$ | H | 2 | 4-Bromphenyl | CH | 134-135°(Z) |
| 6.014 | $COOCH_3$ | H | 0 | 4-Bromphenyl | CH | |
| 6.015 | $COOCH_3$ | H | 2 | 2-Chlorphenyl | CH | |
| 6.016 | $COOCH_3$ | H | 0 | 2-Chlorphenyl | CH | |
| 6.017 | $COOCH_3$ | H | 0 | $CH_3$ | CH | |
| 6.018 | $COOCH_3$ | H | 0 | $CH_2CH_2CH_2CH_3$ | CH | |
| 6.019 | $COOCH_3$ | H | 0 | $CH(CH_3)_2$ | CH | |

Tabelle 6 (Fortsetzung)

| No | $R^1$ | $R^5$ | m | $R^9$ | E | Smp. |
|---|---|---|---|---|---|---|
| 6.020 | $COOCH_3$ | H | 0 | Benzyl | CH | |
| 6.021 | $COOCH_3$ | H | 2 | ß-Naphtyl | CH | 140-141°(Z) |
| 6.022 | $COOCH_3$ | H | 0 | ß-Naphtyl | CH | |
| 6.023 | $COOCH_3$ | H | 2 | 1,2,4-Trichlor-phenyl | CH | |
| 6.024 | $COOCH_3$ | H | 0 | 1,2,4-Trichlor-phenyl | CH | |
| 6.025 | $COOCH_3$ | H | 2 | α-Naphtyl | CH | |
| 6.026 | $COOCH_3$ | H | 2 | α-Naphtyl | CH | |
| 6.027 | $COOCH_3$ | H | 0 | $-CH_2COOCH_3$ | CH | |
| 6.028 | $COOCH_3$ | H | 2 | 4-Fluorphenyl | CH | |
| 6.029 | $COOCH_3$ | H | 0 | 4-Fluorphenyl | CH | |
| 6.030 | $COOCH_3$ | H | 0 | 4-Nitrophenyl | CH | |
| 6.031 | $COOCH_3$ | H | 0 | 4-Nitrophenyl | CH | |
| 6.032 | $COOCH_3$ | H | 2 | 2-Nitrophenyl | CH | |
| 6.033 | $COOCH_3$ | H | 0 | 2-Nitrophenyl | CH | |
| 6.034 | $COOCH_3$ | H | 2 | 2-Trifluormethyl-phenyl | CH | |
| 6.035 | $COOCH_3$ | H | 0 | 2-Trifluormethyl-phenyl | CH | |
| 6.036 | $COOCH_3$ | H | 2 | 4-Methoxyphenyl | CH | |
| 6.037 | $COOCH_3$ | H | 0 | 4-Methoxyphenyl | CH | |
| 6.038 | $COOCH_3$ | H | 2 | 2-Methoxyphenyl | CH | |
| 6.039 | $COOCH_3$ | H | 0 | 2-Methoxyphenyl | CH | |
| 6.040 | $COOCH_3$ | H | 2 | 2,4,6-Trimethyl-phenyl | CH | 133-134°(Z) |
| 6.041 | $COOCH_3$ | H | 0 | 2,4,6-Trimethyl-phenyl | CH | |

Tabelle 6 (Fortsetzung)

| No | $R^1$ | $R^5$ | m | $R^9$ | E | Smp. |
|---|---|---|---|---|---|---|
| 6.042 | $COOCH_3$ | H | 0 | $CS-N(CH_3)_2$ | CH | |
| 6.043 | $COOCH_3$ | H | 0 | $CSN(CH_2CH_3)_2$ | CH | 139-140°(Z) |
| 6.044 | $COOCH_3$ | H | 0 | $CSN(CH_2CH_3)_2$ | N | |
| 6.045 | $COOCH_3$ | H | 0 | Pyrrolidinothio-carbonyl | CH | |
| 6.046 | $COOCH_3$ | H | 0 | Piperidinothio-carbonyl | CH | |
| 6.047 | $COOCH_3$ | H | 0 | N-Phenylcarbamoyl-methyl | CH | |
| 6.048 | $COOCH_3$ | H | 0 | 2,4-Dichlorphenyl | CH | |
| 6.049 | $COOCH_3$ | H | 2 | 2,4-Dichlorphenyl | CH | |
| 6.050 | $COOCH_3$ | H | 0 | $CH_2COOH$ | CH | |
| 6.051 | $COOCH_3$ | H | 0 | $CH_2COO\ CH_3$ | CH | |
| 6.052 | $COOCH_3$ | H | 0 | $CH_2CON(CH_3)_2$ | CH | |
| 6.053 | $COOCH_3$ | H | 0 | 4-Chlorphenyl | N | |
| 6.054 | $COOCH_3$ | H | 0 | p-Tolyl | N | |
| 6.055 | $COOCH_3$ | Furyl-2- | 0 | 2-Hydroxy-carbonylphenyl | CH | |
| 6.056 | $COOCH_3$ | Furyl-2- | 0 | 2-Methoxy-carbonylphenyl | CH | |
| 6.057 | $COOCH_3$ | Furyl-2- | 0 | Phenyl | CH | |
| 6.058 | $COOCH_3$ | 4-Chlor-phenyl | 0 | 2-Hydroxy-carbonylphenyl | CH | |
| 6.059 | $COOCH_3$ | 4-Chlor-phenyl | 0 | Phenyl | CH | |
| 6.060 | $COOCH_3$ | $CH_3$ | 0 | 4-Chlorphenyl | CH | |

Tabelle 6 (Fortsetzung)

| No | $R^1$ | $R^5$ | m | $R^9$ | E | Smp. |
|---|---|---|---|---|---|---|
| 6.061 | $OCHF_2$ | H | 2 | p-Tolyl | CH | 142-143° |
| 6.062 | $OCHF_2$ | H | 0 | p-Tolyl | CH | |
| 6.063 | $OCHF_2$ | H | 2 | 4-Chlorphenyl | CH | |
| 6.064 | $OCHF_2$ | H | 0 | 4-Chlorphenyl | CH | |
| 6.065 | $OCHF_2$ | H | 0 | Acetyl | CH | |
| 6.066 | $OCHF_2$ | H | 0 | Benzoyl | CH | |
| 6.067 | $OCHF_2$ | H | 2 | $CH_3$ | CH | |
| 6.068 | $OCHF_2$ | H | 2 | $CH_2CH_2CH_2CH_3$ | CH | |
| 6.069 | $OCHF_2$ | H | 2 | $CH(CH_3)_2$ | CH | |
| 6.070 | $OCHF_2$ | H | 2 | Benzyl | CH | |
| 6.071 | $OCHF_2$ | H | 2 | Phenyl | CH | |
| 6.072 | $OCHF_2$ | H | 0 | Phenyl | CH | |
| 6.073 | $OCHF_2$ | H | 2 | 4-Bromphenyl | CH | |
| 6.074 | $OCHF_2$ | H | 0 | 4-Bromphenyl | CH | |
| 6.075 | $OCHF_2$ | H | 2 | 2-Chlorphenyl | CH | |
| 6.076 | $OCHF_2$ | H | 0 | 2-Chlorphenyl | CH | |
| 6.077 | $OCHF_2$ | H | 0 | $CH_3$ | CH | |
| 6.078 | $OCHF_2$ | H | 0 | $CH_2CH_2CH_2CH_3$ | CH | |
| 6.079 | $OCHF_2$ | H | 0 | $CH(CH_3)_2$ | CH | |
| 6.080 | $OCHF_2$ | H | 0 | Benzyl | CH | |
| 6.081 | $OCHF_2$ | H | 2 | ß-Naphtyl | CH | |
| 6.082 | $OCHF_2$ | H | 0 | ß-Naphtyl | CH | |
| 6.083 | $OCHF_2$ | H | 2 | 1,2,4-Trichlor-phenyl | CH | |

Tabelle 6 (Fortsetzung)

| No | $R^1$ | $R^5$ | m | $R^9$ | E | Smp. |
|---|---|---|---|---|---|---|
| 6.084 | $OCHF_2$ | H | 0 | 1,2,4-Trichlor-phenyl | CH | |
| 6.085 | $OCHF_2$ | H | 2 | α-Naphtyl | CH | |
| 6.086 | $OCHF_2$ | H | 2 | α-Naphtyl | CH | |
| 6.087 | $OCHF_2$ | H | 0 | $-CH_2COOCH_3$ | CH | |
| 6.088 | $OCHF_2$ | H | 2 | 4-Fluorphenyl | CH | |
| 6.089 | $OCHF_2$ | H | 0 | 4-Fluorphenyl | CH | |
| 6.090 | $OCHF_2$ | H | 0 | 4-Nitrophenyl | CH | |
| 6.091 | $OCHF_2$ | H | 0 | 4-Nitrophenyl | CH | |
| 6.092 | $OCHF_2$ | H | 2 | 2-Nitrophenyl | CH | |
| 6.093 | $OCHF_2$ | H | 0 | 2-Nitrophenyl | CH | |
| 6.094 | $OCHF_2$ | H | 2 | 2-Trifluormethyl | CH | |
| 6.095 | $OCHF_2$ | H | 0 | 2-Trifluormethyl | CH | |
| 6.096 | $OCHF_2$ | H | 2 | 4-Methoxyphenyl | CH | |
| 6.097 | $OCHF_2$ | H | 0 | 4-Methoxyphenyl | CH | |
| 6.098 | $OCHF_2$ | H | 2 | 2-Methoxyphenyl | CH | |
| 6.099 | $OCHF_2$ | H | 0 | 2-Methoxyphenyl | CH | |
| 6.100 | $OCHF_2$ | H | 2 | 2,4,6-Trimethyl-phenyl | CH | |
| 6.101 | $OCHF_2$ | H | 0 | 2,4,6-Trimethyl-phenyl | CH | |
| 6.102 | $OCHF_2$ | H | 0 | $CS-N(CH_3)_2$ | CH | |
| 6.103 | $OCHF_2$ | H | 0 | $CSN(CH_2CH_3)_2$ | CH | |
| 6.104 | $OCHF_2$ | H | 0 | $CSN(CH_2CH_3)_2$ | CH | |
| 6.105 | $OCHF_2$ | H | 0 | Pyrrolidinothio-carbonyl | CH | |

Tabelle 6 (Fortsetzung)

| No | $R^1$ | $R^5$ | m | $R^9$ | E | Smp. |
|---|---|---|---|---|---|---|
| 6.106 | $OCHF_2$ | H | 0 | Piperidinothio-carbonyl | CH | |
| 6.107 | $OCHF_2$ | H | 0 | N-Phenylcarbamoyl-methyl | CH | |
| 6.108 | $OCHF_2$ | H | 0 | 2,4-Dichlorphenyl | CH | |
| 6.109 | $OCHF_2$ | H | 2 | 2,4-Dichlorphenyl | CH | |
| 6.110 | $OCHF_2$ | H | 0 | $CH_2COOH$ | CH | |
| 6.111 | $OCHF_2$ | H | 0 | $CH_2COO\ CH_3$ | CH | |
| 6.112 | $OCHF_2$ | H | 0 | $CH_2CON(CH_3)_2$ | CH | |
| 6.113 | $OCHF_2$ | H | 0 | 4-Chlorphenyl | N | |
| 6.114 | $OCHF_2$ | H | 0 | p-Tolyl | N | |
| 6.115 | $OCHF_2$ | Furyl-2- | 0 | 2-Hydroxy-carbonylphenyl | CH | |
| 6.116 | $OCHF_2$ | Furyl-2- | 0 | 2-Methoxy-carbonylphenyl | CH | |
| 6.117 | $OCHF_2$ | Furyl-2- | 0 | Phenyl | CH | |
| 6.118 | $OCHF_2$ | 4-Chlor-phenyl | 0 | 2-Hydroxy-carbonylphenyl | CH | |
| 6.119 | $OCHF_2$ | 4-Chlor-phenyl | 0 | Phenyl | CH | |
| 6.120 | $OCHF_2$ | $CH_3$ | 0 | 4-Chlorphenyl | CH | |
| 6.121 | Cl | H | 2 | p-Tolyl | CH | |
| 6.122 | Cl | H | 0 | p-Tolyl | CH | |
| 6.123 | Cl | H | 2 | 4-Chlorphenyl | CH | |
| 6.124 | Cl | H | 0 | 4-Chlorphenyl | CH | |
| 6.125 | Cl | H | 0 | Acetyl | CH | |
| 6.126 | Cl | H | 0 | Benzoyl | CH | |
| 6.127 | Cl | H | 2 | $CH_3$ | CH | |

Tabelle 6 (Fortsetzung)

| No | $R^1$ | $R^5$ | m | $R^9$ | E | Smp. |
|---|---|---|---|---|---|---|
| 6.128 | Cl | H | 2 | $CH_2CH_2CH_2CH_3$ | CH | |
| 6.129 | Cl | H | 2 | $CH(CH_3)_2$ | CH | |
| 6.130 | Cl | H | 2 | Benzyl | CH | |
| 6.131 | Cl | H | 2 | Phenyl | CH | |
| 6.132 | Cl | H | 0 | Phenyl | CH | |
| 6.133 | Cl | H | 2 | 4-Bromphenyl | CH | |
| 6.134 | Cl | H | 0 | 4-Bromphenyl | CH | |
| 6.135 | Cl | H | 2 | 2-Chlorphenyl | CH | |
| 6.136 | Cl | H | 0 | 2-Chlorphenyl | CH | |
| 6.137 | Cl | H | 0 | $CH_3$ CH | | |
| 6.138 | Cl | H | 0 | $CH_2CH_2CH_2CH_3$ | CH | |
| 6.139 | Cl | H | 0 | $CH(CH_3)_2$ | CH | |
| 6.140 | Cl | H | 0 | Benzyl | CH | |
| 6.141 | Cl | H | 2 | ß-Naphtyl | CH | |
| 6.142 | Cl | H | 0 | ß-Naphtyl | CH | |
| 6.143 | Cl | H | 2 | 1,2,4-Trichlor-phenyl | CH | |
| 6.144 | Cl | H | 0 | 1,2,4-Trichlor-phenyl | CH | |
| 6.145 | Cl | H | 2 | α-Naphtyl | CH | |
| 6.146 | Cl | H | 2 | α-Naphtyl | CH | |
| 6.147 | Cl | H | 0 | $-CH_2COOCH_3$ | CH | |
| 6.148 | Cl | H | 2 | 4-Fluorphenyl | CH | |
| 6.149 | Cl | H | 0 | 4-Fluorphenyl | CH | |
| 6.150 | Cl | H | 0 | 4-Nitrophenyl | CH | |
| 6.151 | Cl | H | 0 | 4-Nitrophenyl | CH | |

Tabelle 6 (Fortsetzung)

| No | $R^1$ | $R^5$ | m | $R^9$ | E | Smp. |
|---|---|---|---|---|---|---|
| 6.152 | Cl | H | 2 | 2-Nitrophenyl | CH | |
| 6.153 | Cl | H | 0 | 2-Nitrophenyl | CH | |
| 6.154 | Cl | H | 2 | 2-Trifluormethyl | CH | |
| 6.155 | Cl | H | 0 | 2-Trifluormethyl | CH | |
| 6.156 | Cl | H | 2 | 4-Methoxyphenyl | CH | |
| 6.157 | Cl | H | 0 | 4-Methoxyphenyl | CH | |
| 6.158 | Cl | H | 2 | 2-Methoxyphenyl | CH | |
| 6.159 | Cl | H | 0 | 2-Methoxyphenyl | CH | |
| 6.160 | Cl | H | 2 | 2,4,6-Trimethyl-phenyl | CH | |
| 6.161 | Cl | H | 0 | 2,4,6-Trimethyl-phenyl | CH | |
| 6.162 | Cl | H | 0 | $CS-N(CH_3)_2$ | CH | |
| 6.163 | Cl | H | 0 | $CSN(CH_2CH_3)_2$ | CH | |
| 6.164 | Cl | H | 0 | $CSN(CH_2CH_3)_2$ | CH | |
| 6.165 | Cl | H | 0 | Pyrrolidinothio-carbonyl | CH | |
| 6.166 | Cl | H | 0 | Piperidinothio-carbonyl | CH | |
| 6.167 | Cl | H | 0 | N-Phenylcarbamoyl-methyl | CH | |
| 6.168 | Cl | H | 0 | 2,4-Dichlorphenyl | CH | |
| 6.169 | Cl | H | 2 | 2,4-Dichlorphenyl | CH | |
| 6.170 | Cl | H | 0 | $CH_2COOH$ | CH | |
| 6.171 | Cl | H | 0 | $CH_2COO\ CH_3$ | CH | |
| 6.172 | Cl | H | 0 | $CH_2CON(CH_3)_2$ | CH | |

Tabelle 6 (Fortsetzung)

| No | $R^1$ | $R^5$ | m | $R^9$ | E | Smp. |
|---|---|---|---|---|---|---|
| 6.173 | Cl | H | 0 | 4-Chlorphenyl | N | |
| 6.174 | Cl | H | 0 | p-Tolyl | N | |
| 6.175 | Cl | Furyl-2- | 0 | 2-Hydroxy-carbonylphenyl | CH | |
| 6.176 | Cl | Furyl-2- | 0 | 2-Methoxy-carbonylphenyl | CH | |
| 6.177 | Cl | Furyl-2- | 0 | Phenyl | CH | |
| 6.178 | Cl | 4-Chlor-phenyl | 0 | 2-Hydroxy-carbonylphenyl | CH | |
| 6.179 | Cl | 4-Chlor-phenyl | 0 | Phenyl | CH | |
| 6.180 | Cl | $CH_3$ | 0 | 4-Chlorphenyl | CH | |
| 6.181 | $OCH_3$ | H | 0 | Phenyl | CH | |
| 6.182 | $OCH_3$ | H | 2 | Phenyl | CH | |
| 6.183 | $OCH_3$ | H | 0 | $CSN(CH_3)_2$ | CH | |
| 6.184 | $OCH_3$ | H | 0 | Pyrrolidinothio-carbonyl | CH | |
| 6.185 | $OCH_3$ | H | 0 | $CH_3$ | CH | |
| 6.186 | $OCH_3$ | H | 2 | $CH_3$ | CH | |
| 6.187 | $NO_2$ | H | 0 | Phenyl | CH | |
| 6.188 | $NO_2$ | H | 2 | Phenyl | CH | |
| 6.189 | $NO_2$ | H | 0 | $CSN(CH_3)_2$ | CH | |
| 6.190 | $NO_2$ | H | 0 | Pyrrolidinothio-carbonyl | CH | |
| 6.191 | $NO_2$ | H | 0 | $CH_3$ | CH | |
| 6.192 | $NO_2$ | H | 2 | $CH_3$ | CH | |
| 6.193 | $CF_3$ | H | 0 | Phenyl | CH | |

Tabelle 6 (Fortsetzung)

| No | $R^1$ | $R^5$ | m | $R^9$ | E | Smp. |
|---|---|---|---|---|---|---|
| 6.194 | $CF_3$ | H | 2 | Phenyl | CH | |
| 6.195 | $CF_3$ | H | 0 | $CSN(CH_3)_2$ | CH | |
| 6.196 | $CF_3$ | H | 0 | Pyrrolidino-thiocarbonyl | CH | |
| 6.197 | $CF_3$ | H | 0 | $CH_3$ | CH | |
| 6.198 | $CF_3$ | H | 2 | $CH_3$ | CH | |
| 6.199 | $OSO_2CH_3$ | H | 0 | Phenyl | CH | |
| 6.200 | $OSO_2CH_3$ | H | 2 | Phenyl | CH | |
| 6.201 | $OSO_2CH_3$ | H | 0 | $CSN(CH_3)_2$ | CH | |
| 6.202 | $OSO_2CH_3$ | H | 0 | Pyrrolidino-thiocarbonyl | CH | |
| 6.203 | $OSO_2CH_3$ | H | 0 | $CH_3$ | CH | |
| 6.204 | $OSO_2CH_3$ | H | 2 | $CH_3$ | CH | |
| 6.205 | $OSO_2C_3H_7n$ | H | 2 | Phenyl | CH | |
| 6.206 | $OSO_2C_3H_7n$ | H | 2 | Phenyl | CH | |
| 6.207 | $OSO_2C_3H_7n$ | H | 0 | $CSN(CH_3)_2$ | CH | |
| 6.208 | $OSO_2C_3H_7n$ | H | 0 | Pyrrolidino-thiocarbonyl | CH | |
| 6.209 | $OSO_2C_3H_7n$ | H | 2 | $CH_3$ | CH | |
| 6.210 | $OSO_2C_3H_7n$ | H | 0 | $CH_3$ | CH | |
| 6.211 | $SO_2N(CH_3)_2$ | H | 0 | Phenyl | CH | |
| 6.212 | $SO_2N(CH_3)_2$ | H | 2 | Phenyl | CH | |
| 6.213 | $SO_2N(CH_3)_2$ | H | 0 | $CSN(CH_3)_2$ | CH | |
| 6.214 | $SO_2N(CH_3)_2$ | H | 0 | Pyrrolidino-thiocarbonyl | CH | |
| 6.215 | $SO_2N(CH_3)_2$ | H | 0 | $CH_3$ | CH | |

Tabelle 6 (Fortsetzung)

| No | $R^1$ | $R^5$ | m | $R^9$ | E | Smp. |
|---|---|---|---|---|---|---|
| 6.216 | $SO_2N(CH_3)_2$ | H | 2 | $CH_3$ | CH | |
| 6.217 | $CH_3$ | H | 0 | Phenyl | CH | |
| 6.218 | $CH_3$ | H | 2 | Phenyl | CH | |
| 6.219 | $CH_3$ | H | 0 | $CSN(CH_3)_2$ | CH | |
| 6.220 | $CH_3$ | H | 0 | Pyrrolidino-thiocarbonyl | CH | |
| 6.221 | $CH_3$ | H | 0 | $CH_3$ | CH | |
| 6.222 | $CH_3$ | H | 2 | $CH_3$ | CH | |
| 6.223 | $SO_2CH_3$ | H | 0 | Phenyl | CH | |
| 6.224 | $SO_2CH_3$ | H | 2 | Phenyl | CH | |
| 6.225 | $SO_2CH_3$ | H | 0 | $CSN(CH_3)_2$ | CH | |
| 6.226 | $SO_2CH_3$ | H | 0 | Pyrrolidino-thiocarbonyl | CH | |
| 6.227 | $SO_2CH_3$ | H | 0 | $CH_3$ | CH | |
| 6.228 | $SO_2CH_3$ | H | 2 | $CH_3$ | CH | |
| 6.229 | Phenyl | H | 0 | p-Tolyl | CH | 144-147° |
| 6.230 | Phenyl | H | 2 | p-Tolyl | CH | 141-144° Zers. |
| 6.231 | $COOCH_3$ | H | 2 | Benzyl | N | |
| 6.232 | $COOCH_3$ | H | 2 | Phenyl | N | |
| 6.233 | $OCF_2CHF_2$ | H | 2 | p-Tolyl | CH | 133-134° |
| 6.234 | $OCF_2CHF_2$ | H | 2 | p-Tolyl | CH | |
| 6.235 | $OCF_2CHF_2$ | H | 2 | $CH_3$ | CH | |
| 6.236 | $OCF_2CHF_2$ | H | 2 | Phenyl | CH | |
| 6.237 | $OCF_2CHF_2$ | H | 2 | 4-Chlorphenyl | CH | |
| 6.238 | $OCF_2CHF_2$ | H | 2 | ß-Naphthyl | CH | |

Tabelle 6 (Fortsetzung)

| No. | $R^1$ | $R^5$ | m | $R^9$ | E | Smp. |
|---|---|---|---|---|---|---|
| 6.239 | F | H | 0 | 4-Chlorphenyl | CH | |
| 6.240 | F | H | 0 | β-Naphthyl | CH | 158-154° |
| 6.241 | F | H | 2 | β-Naphthyl | CH | |
| 6.242 | $COOCH_3$ | H | 2 | 2-Methoxy-carbonylbenzyl | CH | 66-67° |
| 6.243 | $COOCH_3$ | H | 2 | $C_3H_7n$ | CH | |
| 6.244 | $COOCH_3$ | H | 2 | $C_2H_5$ | CH | |
| 6.245 | $COOCH_3$ | H | 2 | 2-Difluormethoxy-phenyl | CH | 145-147° |
| 6.246 | $COOCH_3$ | H | 2 | 3,4-Dichlorphenyl | CH | |
| 6.247 | $COOCH_3$ | H | 2 | 3,5-Dichlorphenyl | CH | |
| 6.248 | $COOCH_3$ | H | 2 | 3-Chlorphenyl | CH | |
| 6.249 | $COOCH_3$ | H | 2 | 2-Difluormethyl-thiophenyl | CH | |
| 6.250 | $COOCH_3$ | H | 2 | 3-Trifluormethyl-phenyl | CH | |
| 6.251 | $COOCH_3$ | H | 2 | 2,5-Dimethyl-phenyl | CH | |
| 6.252 | $COOC_2H_5$ | H | 2 | $CH_3$ | CH | |
| 6.253 | $COOC_2H_5$ | H | 2 | $C_3H_7n$ | CH | |
| 6.254 | $COOC_2H_5$ | H | 2 | Phenyl | CH | 149° |
| 6.255 | $COOC_2H_5$ | H | 2 | p-Tolyl | CH | 147° |
| 6.256 | $COOC_2H_5$ | H | 0 | $CSN(CH_3)_2$ | CH | |
| 6.257 | $NO_2$ | H | 2 | p-Tolyl | CH | 149-151°(Z) |
| 6.258 | $NO_2$ | H | 2 | 4-Chlorphenyl | CH | 164°(Z) |

Tabelle 6 (Fortsetzung)

| No. | $R^1$ ($R^2$) | $R^5$ | m | $R^9$ | E | Smp. |
|---|---|---|---|---|---|---|
| 6.259 | $NO_2$ | H | 2 | 4-Bromophenyl | CH | |
| 6.260 | $NO_2$ | H | 2 | β-Naphthyl | CH | |
| 6.261 | $NO_2$ | H | 2 | $C_2H_5$ | CH | |
| 6.262 | $NO_2$ | H | 2 | $C_3H_7n$ | CH | |
| 6.263 | $NO_2$ | H | 2 | Benzyl | CH | |
| 6.264 | $NO_2$ | H | 2 | 2-Methoxy-carbonylbenzyl | CH | |
| 6.265 | $NO_2$ | H | 2 | 4-Methoxy-phenyl | CH | |
| 6.266 | $NO_2$ | H | 2 | 2-Difluor-methoxyphenyl | CH | |
| 6.267 | $NO_2$ | H | 2 | Mesityl | CH | |
| 6.268 | $OCF_3$ | H | 2 | p-Tolyl | CH | 138-139°(Z) |
| 6.269 | $OC_2H_4OCH_3$ | H | 2 | p-Tolyl | CH | 135-137°(Z) |
| 6.270 | $OC_2H_4Cl$ | H | 2 | p-Tolyl | CH | 133-135°(Z) |
| 6.271 | OCCl=CHCl | | 2 | p-Tolyl | CH | 143-144°(Z) |
| 6.272 | $CF_3$ | | 2 | p-Tolyl | CH | 156-158°(Z) |
| 6.273 | $OSO_2CH_3$ | | 2 | p-Tolyl | CH | |
| 6.274 | $OSO_2N(CH_3)_2$ | | 2 | p-Tolyl | CH | |
| 6.275 | $OCH_3$ | | 2 | p-Tolyl | CH | 164°(Z) |
| 6.276 | $OCH_3$ | | 2 | 4-Bromphenyl | CH | 165°(Z) |
| 6.277 | $OCH_3$ | | 2 | β-Naphthyl | CH | 148-150°(Z) |
| 6.278 | $OCH_3$ | | 2 | Benzyl | CH | 126-128°(Z) |
| 6.279 | $CF_3$ | | 2 | p-Tolyl | CH | 156-158°(Z) |
| 6.280 | $CF_3$ | | 2 | 4-Chlorphenyl | CH | 147-154°(Z) |
| 6.281 | $CF_3$ | | 2 | β-Naphthyl | CH | 152-154°(Z) |

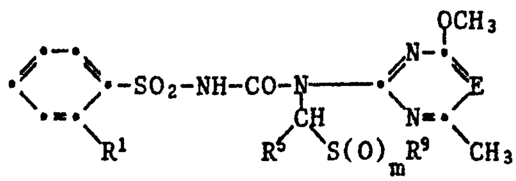

Tabelle 7

| No | $R^1$ | $R^5$ | m | $R^9$ | E | Smp. |
|---|---|---|---|---|---|---|
| 7.001 | $COOCH_3$ | H | 2 | p-Tolyl | CH | 153-158° Zers. |
| 7.002 | $COOCH_3$ | H | 0 | p-Tolyl | CH | |
| 7.003 | $COOCH_3$ | H | 2 | 4-Chlorphenyl | CH | |
| 7.004 | $COOCH_3$ | H | 0 | 4-Chlorphenyl | CH | |
| 7.005 | $COOCH_3$ | H | 0 | Acetyl | CH | |
| 7.006 | $COOCH_3$ | H | 0 | Benzoyl | CH | |
| 7.007 | $COOCH_3$ | H | 2 | $CH_3$ | CH | |
| 7.008 | $COOCH_3$ | H | 2 | $CH_2CH_2CH_2CH_3$ | CH | |
| 7.009 | $COOCH_3$ | H | 2 | $CH(CH_3)_2$ | CH | |
| 7.010 | $COOCH_3$ | H | 2 | Benzyl | CH | |
| 7.011 | $COOCH_3$ | H | 2 | Phenyl | CH | |
| 7.012 | $COOCH_3$ | H | 0 | Phenyl | CH | |
| 7.013 | $COOCH_3$ | H | 2 | 4-Bromphenyl | CH | |
| 7.014 | $COOCH_3$ | H | 0 | 4-Bromphenyl | CH | |
| 7.015 | $COOCH_3$ | H | 2 | 2-Chlorphenyl | CH | |
| 7.016 | $COOCH_3$ | H | 0 | 2-Chlorphenyl | CH | |
| 7.017 | $COOCH_3$ | H | 0 | $CH_3$ | CH | |
| 7.018 | $COOCH_3$ | H | 0 | $CH_2CH_2CH_2CH_3$ | CH | |
| 7.019 | $COOCH_3$ | H | 0 | $CH(CH_3)_2$ | CH | |

Tabelle 7 (Fortsetzung)

| No | $R^1$ | $R^5$ | m | $R^9$ | E | Smp. |
|---|---|---|---|---|---|---|
| 7.020 | $COOCH_3$ | H | 0 | Benzyl | CH | |
| 7.021 | $COOCH_3$ | H | 2 | ß-Naphtyl | CH | |
| 7.022 | $COOCH_3$ | H | 0 | ß-Naphtyl | CH | |
| 7.023 | $COOCH_3$ | H | 2 | 1,2,4-Trichlor-phenyl | CH | |
| 7.024 | $COOCH_3$ | H | 0 | 1,2,4-Trichlor-phenyl | CH | |
| 7.025 | $COOCH_3$ | H | 2 | α-Naphtyl | CH | |
| 7.026 | $COOCH_3$ | H | 2 | α-Naphtyl | CH | |
| 7.027 | $COOCH_3$ | H | 0 | $-CH_2COOCH_3$ | CH | |
| 7.028 | $COOCH_3$ | H | 2 | 4-Fluorphenyl | CH | |
| 7.029 | $COOCH_3$ | H | 0 | 4-Fluorphenyl | CH | |
| 7.030 | $COOCH_3$ | H | 0 | 4-Nitrophenyl | CH | |
| 7.031 | $COOCH_3$ | H | 0 | 4-Nitrophenyl | CH | |
| 7.032 | $COOCH_3$ | H | 2 | 2-Nitrophenyl | CH | |
| 7.033 | $COOCH_3$ | H | 0 | 2-Nitrophenyl | CH | |
| 7.034 | $COOCH_3$ | H | 2 | 2-Trifluormethyl | CH | |
| 7.035 | $COOCH_3$ | H | 0 | 2-Trifluormethyl | CH | |
| 7.036 | $COOCH_3$ | H | 2 | 4-Methoxyphenyl | CH | |
| 7.037 | $COOCH_3$ | H | 0 | 4-Methoxyphenyl | CH | |
| 7.038 | $COOCH_3$ | H | 2 | 2-Methoxyphenyl | CH | |
| 7.039 | $COOCH_3$ | H | 0 | 2-Methoxyphenyl | CH | |
| 7.040 | $COOCH_3$ | H | 2 | 2,4,6-Trimethyl-phenyl | CH | |
| 7.041 | $COOCH_3$ | H | 0 | 2,4,6-Trimethyl-phenyl | CH | |

Tabelle 7 (Fortsetzung)

| No | $R^1$ | $R^5$ | m | $R^9$ | E | Smp. |
|---|---|---|---|---|---|---|
| 7.042 | $COOCH_3$ | H | 0 | $CS-N(CH_3)_2$ | CH | |
| 7.043 | $COOCH_3$ | H | 0 | $CSN(CH_2CH_3)_2$ | CH | |
| 7.044 | $COOCH_3$ | H | 0 | $CSN(CH_2CH_3)_2$ | CH | |
| 7.045 | $COOCH_3$ | H | 0 | Pyrrolidinothio-carbonyl | CH | |
| 7.046 | $COOCH_3$ | H | 0 | Piperidinothio-carbonyl | CH | |
| 7.047 | $COOCH_3$ | H | 0 | N-Phenylcarbamoyl-methyl | CH | |
| 7.048 | $COOCH_3$ | H | 0 | 2,4-Dichlorphenyl | CH | |
| 7.049 | $COOCH_3$ | H | 2 | 2,4-Dichlorphenyl | CH | |
| 7.050 | $COOCH_3$ | H | 0 | $CH_2COOH$ | CH | |
| 7.051 | $COOCH_3$ | H | 0 | $CH_2COO\ CH_3$ | CH | |
| 7.052 | $COOCH_3$ | H | 0 | $CH_2CON(CH_3)_2$ | CH | |
| 7.053 | $COOCH_3$ | H | 0 | 4-Chlorphenyl | N | |
| 7.054 | $COOCH_3$ | H | 0 | p-Tolyl | N | |
| 7.055 | $COOCH_3$ | Furyl-2- | 0 | 2-Hydroxy-carbonylphenyl | CH | |
| 7.056 | $COOCH_3$ | Furyl-2- | 0 | 2-Methoxy-carbonylphenyl | CH | |
| 7.057 | $COOCH_3$ | Furyl-2- | 0 | Phenyl | CH | |
| 7.058 | $COOCH_3$ | 4-Chlor-phenyl | 0 | 2-Hydroxy-carbonylphenyl | CH | |
| .059 | $COOCH_3$ | 4-Chlor-phenyl | 0 | Phenyl | CH | |
| 7.060 | $COOCH_3$ | $CH_3$ | 0 | 4-Chlorphenyl | CH | |

Tabelle 7 (Fortsetzung)

| No | $R^1$ | $R^5$ | m | $R^9$ | E | Smp. |
|---|---|---|---|---|---|---|
| 7.061 | $OCHF_2$ | H | 2 | p-Tolyl | CH | 146-150° |
| 7.062 | $OCHF_2$ | H | 0 | p-Tolyl | CH | |
| 7.063 | $OCHF_2$ | H | 2 | 4-Chlorphenyl | CH | |
| 7.064 | $OCHF_2$ | H | 0 | 4-Chlorphenyl | CH | |
| 7.065 | $OCHF_2$ | H | 0 | Acetyl | CH | |
| 7.066 | $OCHF_2$ | H | 0 | Benzoyl | CH | |
| 7.067 | $OCHF_2$ | H | 2 | $CH_3$ | N | |
| 7.068 | $OCHF_2$ | H | 2 | $CH_2CH_2CH_2CH_3$ | CH | |
| 7.069 | $OCHF_2$ | H | 2 | $CH(CH_3)_2$ | CH | |
| 7.070 | $OCHF_2$ | H | 2 | Benzyl | CH | |
| 7.071 | $OCHF_2$ | H | 2 | Phenyl | CH | |
| 7.072 | $OCHF_2$ | H | 0 | Phenyl | CH | |
| 7.073 | $OCHF_2$ | H | 2 | 4-Bromphenyl | CH | |
| 7.074 | $OCHF_2$ | H | 0 | 4-Bromphenyl | CH | |
| 7.075 | $OCHF_2$ | H | 2 | 2-Chlorphenyl | CH | |
| 7.076 | $OCHF_2$ | H | 0 | 2-Chlorphenyl | CH | |
| 7.077 | $OCHF_2$ | H | 0 | $CH_3$ | N | |
| 7.078 | $OCHF_2$ | H | 0 | $CH_2CH_2CH_2CH_3$ | CH | |
| 7.079 | $OCHF_2$ | H | 0 | $CH(CH_3)_2$ | CH | |
| 7.080 | $OCHF_2$ | H | 0 | Benzyl | CH | |
| 7.081 | $OCHF_2$ | H | 2 | ß-Naphtyl | CH | |
| 7.082 | $OCHF_2$ | H | 0 | ß-Naphtyl | CH | 162-164° |
| 7.083 | $OCHF_2$ | H | 2 | 1,2,4-Trichlor-phenyl | CH | |

Tabelle 7 (Fortsetzung)

| No | $R^1$ | $R^5$ | m | $R^9$ | E | Smp. |
|---|---|---|---|---|---|---|
| 7.084 | $OCHF_2$ | H | 0 | 2,4,5-Trichlor-phenyl | CH | 104-107° |
| 7.085 | $OCHF_2$ | H | 2 | α-Naphtyl | CH | |
| 7.086 | $OCHF_2$ | H | 2 | α-Naphtyl | CH | |
| 7.087 | $OCHF_2$ | H | 0 | $-CH_2COOCH_3$ | CH | |
| 7.088 | $OCHF_2$ | H | 2 | 4-Fluorphenyl | CH | |
| 7.089 | $OCHF_2$ | H | 0 | 4-Fluorphenyl | CH | |
| 7.090 | $OCHF_2$ | H | 0 | 4-Nitrophenyl | CH | |
| 7.091 | $OCHF_2$ | H | 0 | 4-Nitrophenyl | CH | |
| 7.092 | $OCHF_2$ | H | 2 | 2-Nitrophenyl | CH | |
| 7.093 | $OCHF_2$ | H | 0 | 2-Nitrophenyl | CH | |
| 7.094 | $OCHF_2$ | H | 2 | 2-Trifluormethyl | CH | |
| 7.095 | $OCHF_2$ | H | 0 | 2-Trifluormethyl | CH | |
| 7.096 | $OCHF_2$ | H | 2 | 4-Methoxyphenyl | CH | |
| 7.097 | $OCHF_2$ | H | 0 | 4-Methoxyphenyl | CH | |
| 7.098 | $OCHF_2$ | H | 2 | 2-Methoxyphenyl | CH | |
| 7.099 | $OCHF_2$ | H | 0 | 2-Methoxyphenyl | CH | |
| 7.100 | $OCHF_2$ | H | 2 | 2,4,6-Trimethyl-phenyl | CH | |
| 7.101 | $OCHF_2$ | H | 0 | 2,4,6-Trimethyl-phenyl | CH | |
| 7.102 | $OCHF_2$ | H | 0 | $CS-N(CH_3)_2$ | CH | |
| 7.103 | $OCHF_2$ | H | 0 | $CSN(CH_2CH_3)_2$ | CH | |
| 7.104 | $OCHF_2$ | H | 0 | $CSN(CH_2CH_3)_2$ | CH | |
| 7.105 | $OCHF_2$ | H | 0 | Pyrrolidinothio-carbonyl | CH | |

Tabelle 7 (Fortsetzung)

| No | $R^1$ | $R^5$ | m | $R^9$ | E | Smp. |
|---|---|---|---|---|---|---|
| 7.106 | $OCHF_2$ | H | 0 | Piperidinothio-carbonyl | CH | |
| 7.107 | $OCHF_2$ | H | 0 | N-Phenylcarbamoyl-methyl | CH | |
| 7.108 | $OCHF_2$ | H | 0 | 2,4-Dichlorphenyl | CH | |
| 7.109 | $OCHF_2$ | H | 2 | 2,4-Dichlorphenyl | CH | |
| 7.110 | $OCHF_2$ | H | 0 | $CH_2COOH$ | CH | |
| 7.111 | $OCHF_2$ | H | 0 | $CH_2COO\ CH_3$ | CH | |
| 7.112 | $OCHF_2$ | H | 0 | $CH_2CON(CH_3)_2$ | CH | |
| 7.113 | $OCHF_2$ | H | 0 | 4-Chlorphenyl | N | |
| 7.114 | $OCHF_2$ | H | 0 | p-Tolyl | N | |
| 7.115 | $OCHF_2$ | Furyl-2- | 0 | 2-Hydroxy-carbonylphenyl | CH | |
| 7.116 | $OCHF_2$ | Furyl-2- | 0 | 2-Methoxy-carbonylphenyl | CH | |
| 7.117 | $OCHF_2$ | Furyl-2- | 0 | Phenyl | CH | |
| 7.118 | $OCHF_2$ | 4-Chlor-phenyl | 0 | 2-Hydroxy-carbonylphenyl | CH | |
| 7.119 | $OCHF_2$ | 4-Chlor-phenyl | 0 | Phenyl | CH | |
| 7.120 | $OCHF_2$ | $CH_3$ | 0 | 4-Chlorphenyl | CH | |
| 7.121 | Cl | H | 2 | p-Tolyl | CH | |
| 7.122 | Cl | H | 0 | p-Tolyl | CH | |
| 7.123 | Cl | H | 2 | 4-Chlorphenyl | CH | |
| 7.124 | Cl | H | 0 | 4-Chlorphenyl | CH | |
| 7.125 | Cl | H | 0 | Acetyl | CH | |
| 7.126 | Cl | H | 0 | Benzoyl | CH | |
| 7.127 | Cl | H | 2 | $CH_3$ | CH | |

Tabelle 7 (Fortsetzung)

| No | $R^1$ | $R^5$ | m | $R^9$ | E | Smp. |
|---|---|---|---|---|---|---|
| 7.128 | Cl | H | 2 | $CH_2CH_2CH_2CH_3$ | CH | |
| 7.129 | Cl | H | 2 | $CH(CH_3)_2$ | CH | |
| 7.130 | Cl | H | 2 | Benzyl | CH | |
| 7.131 | Cl | H | 2 | Phenyl | CH | |
| 7.132 | Cl | H | 0 | Phenyl | CH | |
| 7.133 | Cl | H | 2 | 4-Bromphenyl | CH | |
| 7.134 | Cl | H | 0 | 4-Bromphenyl | CH | |
| 7.135 | Cl | H | 2 | 2-Chlorphenyl | CH | |
| 7.136 | Cl | H | 0 | 2-Chlorphenyl | CH | |
| 7.137 | Cl | H | 0 | $CH_3$ CH | | |
| 7.138 | Cl | H | 0 | $CH_2CH_2CH_2CH_3$ | CH | |
| 7.139 | Cl | H | 0 | $CH(CH_3)_2$ | CH | |
| 7.140 | Cl | H | 0 | Benzyl | CH | |
| 7.141 | Cl | H | 2 | ß-Naphtyl | CH | |
| 7.142 | Cl | H | 0 | ß-Naphtyl | CH | 156-158° Zers. |
| 7.143 | Cl | H | 2 | 1,2,4-Trichlor-phenyl | CH | |
| 7.144 | Cl | H | 0 | 1,2,4-Trichlor-phenyl | CH | |
| 7.145 | Cl | H | 2 | α-Naphtyl | CH | |
| 7.146 | Cl | H | 2 | α-Naphtyl | CH | |
| 7.147 | Cl | H | 0 | $-CH_2COOCH_3$ | CH | |
| 7.148 | Cl | H | 2 | 4-Fluorphenyl | CH | |
| 7.149 | Cl | H | 0 | 4-Fluorphenyl | CH | |
| 7.150 | Cl | H | 0 | 4-Nitrophenyl | CH | |
| 7.151 | Cl | H | 0 | 4-Nitrophenyl | CH | |

Tabelle 7 (Fortsetzung)

| No | $R^1$ | $R^5$ | m | $R^9$ | E | Smp. |
|---|---|---|---|---|---|---|
| 7.152 | Cl | H | 2 | 2-Nitrophenyl | CH | |
| 7.153 | Cl | H | 0 | 2-Nitrophenyl | CH | |
| 7.154 | Cl | H | 2 | 2-Trifluormethyl | CH | |
| 7.155 | Cl | H | 0 | 2-Trifluormethyl | CH | |
| 7.156 | Cl | H | 2 | 4-Methoxyphenyl | CH | |
| 7.157 | Cl | H | 0 | 4-Methoxyphenyl | CH | |
| 7.158 | Cl | H | 2 | 2-Methoxyphenyl | CH | |
| 7.159 | Cl | H | 0 | 2-Methoxyphenyl | CH | |
| 7.160 | Cl | H | 2 | 2,4,6-Trimethyl-phenyl | CH | |
| 7.161 | Cl | H | 0 | 2,4,6-Trimethyl-phenyl | CH | |
| 7.162 | Cl | H | 0 | $CS-N(CH_3)_2$ | CH | |
| 7.163 | Cl | H | 0 | $CSN(CH_2CH_3)_2$ | CH | |
| 7.164 | Cl | H | 0 | $CSN(CH_2CH_3)_2$ | CH | |
| 7.165 | Cl | H | 0 | Pyrrolidinothio-carbonyl | CH | |
| 7.166 | Cl | H | 0 | Piperidinothio-carbonyl | CH | |
| 7.167 | Cl | H | 0 | N-Phenylcarbamoyl-methyl | CH | |
| 7.168 | Cl | H | 0 | 2,4-Dichlorphenyl | CH | |
| 7.169 | Cl | H | 2 | 2,4-Dichlorphenyl | CH | |
| 7.170 | Cl | H | 0 | $CH_2COOH$ | CH | |
| 7.171 | Cl | H | 0 | $CH_2COO\ CH_3$ | CH | |
| 7.172 | Cl | H | 0 | $CH_2CON(CH_3)_2$ | CH | |

Tabelle 7 (Fortsetzung)

| No | $R^1$ | $R^5$ | m | $R^9$ | E | Smp. |
|---|---|---|---|---|---|---|
| 7.173 | Cl | H | 0 | 4-Chlorphenyl | N | |
| 7.174 | Cl | H | 0 | p-Tolyl | N | |
| 7.175 | Cl | Furyl-2- | 0 | 2-Hydroxy-carbonylphenyl | CH | |
| 7.176 | Cl | Furyl-2- | 0 | 2-Methoxy-carbonylphenyl | CH | |
| 7.177 | Cl | Furyl-2- | 0 | Phenyl | CH | |
| 7.178 | Cl | 4-Chlor-phenyl | 0 | 2-Hydroxy-carbonylphenyl | CH | |
| 7.179 | Cl | 4-Chlor-phenyl | 0 | Phenyl | CH | |
| 7.180 | Cl | $CH_3$ | 0 | 4-Chlorphenyl | CH | |
| 7.181 | $OCH_3$ | H | 0 | Phenyl | CH | |
| 7.182 | $OCH_3$ | H | 2 | Phenyl | CH | |
| 7.183 | $OCH_3$ | H | 0 | $CSN(CH_3)_2$ | CH | |
| 7.184 | $OCH_3$ | H | 0 | Pyrrolidinothio-carbonyl | CH | |
| 7.185 | $OCH_3$ | H | 0 | $CH_3$ | CH | |
| 7.186 | $OCH_3$ | H | 2 | $CH_3$ | CH | |
| 7.187 | $NO_2$ | H | 0 | Phenyl | CH | |
| 7.188 | $NO_2$ | H | 2 | Phenyl | CH | |
| 7.189 | $NO_2$ | H | 0 | $CSN(CH_3)_2$ | CH | |
| 7.190 | $NO_2$ | H | 0 | Pyrrolidinothio-carbonyl | CH | |
| 7.191 | $NO_2$ | H | 0 | $CH_3$ | CH | |
| 7.192 | $NO_2$ | H | 2 | $CH_3$ | CH | |
| 7.193 | $CF_3$ | H | 0 | Phenyl | CH | |

Tabelle 7 (Fortsetzung)

| No | $R^1$ | $R^5$ | m | $R^9$ | E | Smp. |
|---|---|---|---|---|---|---|
| 7.194 | $CF_3$ | H | 2 | Phenyl | CH | |
| 7.195 | $CF_3$ | H | 0 | $CSN(CH_3)_2$ | CH | |
| 7.196 | $CF_3$ | H | 0 | Pyrrolidino-thiocarbonyl | CH | |
| 7.197 | $CF_3$ | H | 0 | $CH_3$ | CH | |
| 7.198 | $CF_3$ | H | 2 | $CH_3$ | CH | |
| 7.199 | $OSO_2CH_3$ | H | 0 | Phenyl | CH | |
| 7.200 | $OSO_2CH_3$ | H | 2 | Phenyl | CH | |
| 7.201 | $OSO_2CH_3$ | H | 0 | $CSN(CH_3)_2$ | CH | |
| 7.202 | $OSO_2CH_3$ | H | 0 | Pyrrolidino-thiocarbonyl | CH | |
| 7.203 | $OSO_2CH_3$ | H | 0 | $CH_3$ | CH | |
| 7.204 | $OSO_2CH_3$ | H | 2 | $CH_3$ | CH | |
| 7.205 | $OSO_2C_3H_7n$ | H | 2 | Phenyl | CH | |
| 7.206 | $OSO_2C_3H_7n$ | H | 2 | Phenyl | CH | |
| 7.207 | $OSO_2C_3H_7n$ | H | 0 | $CSN(CH_3)_2$ | CH | |
| 7.208 | $OSO_2C_3H_7n$ | H | 0 | Pyrrolidino-thiocarbonyl | CH | |
| 7.209 | $OSO_2C_3H_7n$ | H | 2 | $CH_3$ | CH | |
| 7.210 | $OSO_2C_3H_7n$ | H | 0 | $CH_3$ | CH | |
| 7.211 | $SO_2N(CH_3)_2$ | H | 0 | Phenyl | CH | |
| 7.212 | $SO_2N(CH_3)_2$ | H | 2 | Phenyl | CH | |
| 7.213 | $SO_2N(CH_3)_2$ | H | 0 | $CSN(CH_3)_2$ | CH | |
| 7.214 | $SO_2N(CH_3)_2$ | H | 0 | Pyrrolidino-thiocarbonyl | CH | |
| 7.215 | $SO_2N(CH_3)_2$ | H | 0 | $CH_3$ | CH | |

Tabelle 7 (Fortsetzung)

| No | $R^1$ | $R^5$ | m | $R^9$ | E | Smp. |
|---|---|---|---|---|---|---|
| 7.216 | $SO_2N(CH_3)_2$ | H | 2 | $CH_3$ | CH | |
| 7.217 | $CH_3$ | H | 0 | Phenyl | CH | |
| 7.218 | $CH_3$ | H | 2 | Phenyl | CH | |
| 7.219 | $CH_3$ | H | 0 | $CSN(CH_3)_2$ | CH | |
| 7.220 | $CH_3$ | H | 0 | Pyrrolidino-thiocarbonyl | CH | |
| 7.221 | $CH_3$ | H | 0 | $CH_3$ | CH | |
| 7.222 | $CH_3$ | H | 2 | $CH_3$ | CH | |
| 7.223 | $SO_2CH_3$ | H | 0 | Phenyl | CH | |
| 7.224 | $SO_2CH_3$ | H | 2 | Phenyl | CH | |
| 7.225 | $SO_2CH_3$ | H | 0 | $CSN(CH_3)_2$ | CH | |
| 7.226 | $SO_2CH_3$ | H | 0 | Pyrrolidino-thiocarbonyl | CH | |
| 7.227 | $SO_2CH_3$ | H | 0 | $CH_3$ | CH | |
| 7.228 | $SO_2CH_3$ | H | 2 | $CH_3$ | CH | |
| 7.229 | $CF_3$ | H | 2 | ß-Naphthyl | CH | 153-156° Zers. |
| 7.230 | 2,6-$Cl_2$ | H | 2 | ß-Naphthyl | CH | 154-158° Zers. |
| 7.231 | Phenyl | H | 2 | $CH_3$ | CH | 166-170° Zers. |
| 7.232 | Phenyl | H | 2 | p-Tolyl | CH | 156-159° Zers. |
| 7.233 | Phenyl | H | 0 | 4-Chlorphenyl | CH | 161-154° |
| 7.234 | F | H | 0 | 4-Chlorphenyl | CH | 126-127° |
| 7.235 | Phenyl | H | 0 | $CH_2COOCH_3$ | CH | 127-129° |
| 7.236 | $NO_2$ | H | 0 | Anilidocarbonyl-methyl | CH | 162-165°(Z) |
| 7.237 | 2-Cl,6-$CH_3$ | H | 2 | p-Tolyl | CH | 150-152°(Z) |

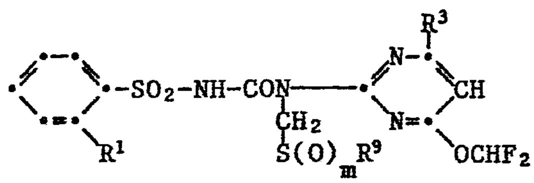

Tabelle 8

| No . | $R^1$ | m | $R^9$ | $R^3$ | Schmelzpunkt |
|---|---|---|---|---|---|
| 8.001 | $COOCH_3$ | 0 | p-Tolyl | $CH_3$ | |
| 8.002 | $COOCH_3$ | 0 | p-Tolyl | $OCH_3$ | |
| 8.003 | $COOCH_3$ | 0 | p-Tolyl | $OCHF_2$ | |
| 8.004 | $COOCH_3$ | 2 | p-Tolyl | $CH_3$ | |
| 8.005 | $COOCH_3$ | 2 | p-Tolyl | $OCH_3$ | |
| 8.006 | $COOCH_3$ | 2 | p-Tolyl | $OCHF_2$ | |
| 8.007 | $COOCH_3$ | 0 | $CSN(CH_3)_2$ | $CH_3$ | |
| 8.008 | $COOCH_3$ | 0 | $CSN(CH_3)_2$ | $OCH_3$ | |
| 8.009 | $COOCH_3$ | 0 | $CSN(CH_3)_2$ | $OCHF_2$ | |
| 8.010 | $COOCH_3$ | 2 | Aethyl | $CH_3$ | |
| 8.011 | $COOCH_3$ | 2 | Aethyl | $OCH_3$ | |
| 8.012 | $COOCH_3$ | 2 | Aethyl | $OCHF_2$ | |
| 8.013 | $COOCH_3$ | 0 | 4-Chlorphenyl | $CH_3$ | |
| 8.014 | COOCH | 0 | 4-Chlorphenyl | $OCHF_2$ | |
| 8.015 | $COOCH_3$ | 0 | 4-Chlorphenyl | $OCH_3$ | |
| 8.016 | $COOCH_3$ | 2 | 4-Chlorphenyl | $CH_3$ | |

Tabelle 8 (Fortsetzung)

| No | $R^1$ | m | $R^9$ | $R^3$ | Schmelzpunkt |
|---|---|---|---|---|---|
| 8.017 | $COOCH_3$ | 2 | 4-Chlorphenyl | $OCHF_2$ | |
| 8.018 | $COOCH_3$ | 2 | 4-Chlorphenyl | $OCH_3$ | |
| 8.019 | $COOCH_3$ | 0 | Pyrrolidino thiocarbonyl | $CH_3$ | |
| 8.020 | $COOCH_3$ | 0 | Pyrrolidino-thiocarbonyl | $OCH_2$ | |
| 8.021 | $COOCH_3$ | 0 | Pyrrolidino-thiocarbonyl | $OCHF_2$ | |
| 8.022 | $OCHF_2$ | 0 | p-Tolyl | $CH_3$ | |
| 8.023 | $OCHF_2$ | 0 | p-Tolyl | $OCH_3$ | |
| 8.024 | $OCHF_2$ | 0 | p-Tolyl | $OCHF_2$ | |
| 8.025 | $OCHF_2$ | 2 | p-Tolyl | $CH_3$ | 154-158° Zers. |
| 8.026 | $OCHF_2$ | 2 | p-Tolyl | $OCH_3$ | |
| 8.027 | $OCHF_2$ | 2 | p-Tolyl | $OCHF_2$ | |
| 8.028 | $OCHF_2$ | 0 | $CSN(CH_3)_2$ | $CH_3$ | |
| 8.029 | $OCHF_2$ | 0 | $CSN(CH_3)_2$ | $OCH_3$ | |
| 8.030 | $OCHF_2$ | 0 | $CSN(CH_3)_2$ | $OCHF_2$ | |
| 8.031 | $OCHF_2$ | 2 | Aethyl | $CH_3$ | |
| 8.032 | $OCHF_2$ | 2 | Aethyl | $OCH_3$ | |
| 8.033 | $OCHF_2$ | 2 | Aethyl | $OCHF_2$ | |
| 8.034 | $OCHF_2$ | 0 | 4-Chlorphenyl | $CH_3$ | |
| 8.035 | $OCHF_2$ | 0 | 4-Chlorphenyl | $OCHF_2$ | |
| 8.036 | $OCHF_2$ | 0 | 4-Chlorphenyl | $OCH_3$ | |
| 8.037 | $OCHF_2$ | 2 | 4-Chlorphenyl | $CH_3$ | |
| 8.038 | $OCHF_2$ | 2 | 4-Chlorphenyl | $OCHF_2$ | |

Tabelle 8 (Fortsetzung)

| No | $R^1$ | m | $R^9$ | $R^3$ | Schmelzpunkt |
|---|---|---|---|---|---|
| 8.039 | $OCHF_2$ | 2 | 4-Chlorphenyl | $OCH_3$ | |
| 8.040 | $OCHF_2$ | 0 | Pyrrolidino thiocarbonyl | $CH_3$ | |
| 8.041 | $OCHF_2$ | 0 | Pyrrolidino-thiocarbonyl | $OCH_2$ | |
| 8.042 | $OCHF_2$ | 0 | Pyrrolidino-thiocarbonyl | $OCHF_2$ | |
| 8.043 | Cl | 0 | p-Tolyl | $CH_3$ | |
| 8.044 | Cl | 0 | p-Tolyl | $OCH_3$ | |
| 8.045 | Cl | 0 | p-Tolyl | $OCHF_2$ | |
| 8.046 | Cl | 2 | p-Tolyl | $CH_3$ | |
| 8.047 | Cl | 2 | p-Tolyl | $OCH_3$ | |
| 8.048 | Cl | 2 | p-Tolyl | $OCHF_2$ | |
| 8.049 | Cl | 0 | $CSN(CH_3)_2$ | $CH_3$ | |
| 8.050 | Cl | 0 | $CSN(CH_3)_2$ | $OCH_3$ | |
| 8.051 | Cl | 0 | $CSN(CH_3)_2$ | $OCHF_2$ | |
| 8.052 | Cl | 2 | Aethyl | $CH_3$ | |
| 8.053 | Cl | 2 | Aethyl | $OCH_3$ | |
| 8.054 | Cl | 2 | Aethyl | $OCHF_2$ | |
| 8.055 | Cl | 0 | 4-Chlorphenyl | $CH_3$ | |
| 8.056 | Cl | 0 | 4-Chlorphenyl | $OCHF_2$ | |
| 8.057 | Cl | 0 | 4-Chlorphenyl | $OCH_3$ | |
| 8.058 | Cl | 2 | 4-Chlorphenyl | $CH_3$ | |
| 8.059 | Cl | 2 | 4-Chlorphenyl | $OCHF_2$ | |
| 8.060 | Cl | 2 | 4-Chlorphenyl | $OCH_3$ | |

Tabelle 8 (Fortsetzung)

| No | $R^1$ | m | $R^9$ | $R^3$ | Schmelzpunkt |
|---|---|---|---|---|---|
| 8.061 | Cl | 0 | Pyrrolidino thiocarbonyl | $CH_3$ | |
| 8.062 | Cl | 0 | Pyrrolidino-thiocarbonyl | $OCH_2$ | |
| 8.063 | Cl | 0 | Pyrrolidino-thiocarbonyl | $OCHF_2$ | |
| 8.064 | $NO_2$ | 0 | p-Tolyl | $CH_3$ | |
| 8.065 | $NO_2$ | 0 | p-Tolyl | $OCH_3$ | |
| 8.066 | $NO_2$ | 0 | p-Tolyl | $OCHF_2$ | |
| 8.067 | $NO_2$ | 2 | p-Tolyl | $CH_3$ | |
| 8.068 | $NO_2$ | 2 | p-Tolyl | $OCH_3$ | |
| 8.069 | $NO_2$ | 2 | p-Tolyl | $OCHF_2$ | |
| 8.070 | $NO_2$ | 0 | $CSN(CH_3)_2$ | $CH_3$ | |
| 8.071 | $NO_2$ | 0 | $CSN(CH_3)_2$ | $OCH_3$ | |
| 8.072 | $NO_2$ | 0 | $CSN(CH_3)_2$ | $OCHF_2$ | |
| 8.073 | $NO_2$ | 2 | Aethyl | $CH_3$ | |
| 8.074 | $NO_2$ | 2 | Aethyl | $OCH_3$ | |
| 8.075 | $NO_2$ | 2 | Aethyl | $OCHF_2$ | |
| 8.076 | $NO_2$ | 0 | 4-Chlorphenyl | $CH_3$ | |
| 8.077 | $NO_2$ | 0 | 4-Chlorphenyl | $OCHF_2$ | |
| 8.078 | $NO_2$ | 0 | 4-Chlorphenyl | $OCH_3$ | |
| 8.079 | $NO_2$ | 2 | 4-Chlorphenyl | $CH_3$ | |
| 8.080 | $NO_2$ | 2 | 4-Chlorphenyl | $OCHF_2$ | |
| 8.081 | $NO_2$ | 2 | 4-Chlorphenyl | $OCH_3$ | |
| 8.082 | $NO_2$ | 0 | Pyrrolidino-thiocarbonyl | $CH_3$ | |

Tabelle 8 (Fortsetzung)

| No | $R^1$ | m | $R^9$ | $R^3$ | Schmelzpunkt |
|---|---|---|---|---|---|
| 8.083 | $NO_2$ | 0 | Pyrrolidino-thiocarbonyl | $OCH_2$ | |
| 8.084 | $NO_2$ | 0 | Pyrrolidino-thiocarbonyl | $OCHF_2$ | |
| 8.085 | $COOCH_3$ | 0 | $CH_3$ | $CH_3$ | |
| 8.086 | $COOCH_3$ | 0 | $CH_3$ | $OCH_3$ | |
| 8.087 | $COOCH_3$ | 0 | $CH_3$ | $OCHF_2$ | |
| 8.088 | $COOCH_3$ | 2 | $CH_3$ | $CH_3$ | |
| 8.089 | $COOCH_3$ | 2 | $CH_3$ | $OCH_3$ | |
| 8.090 | $COOCH_3$ | 2 | $CH_3$ | $OCHF_2$ | |
| 8.091 | $OCHF_2$ | 0 | $CH_3$ | $CH_3$ | |
| 8.092 | $OCHF_2$ | 0 | $CH_3$ | $OCHF_2$ | |
| 8.093 | $OCHF_2$ | 0 | $CH_3$ | $OCH_3$ | |
| 8.094 | $OCHF_2$ | 2 | $CH_3$ | $CH_3$ | |
| 8.095 | $OCHF_2$ | 2 | $CH_3$ | $OCH_3$ | |
| 8.096 | $OCHF_2$ | 2 | $CH_3$ | $OCHF_2$ | |
| 8.097 | $NO_2$ | 2 | $CH_2COOH$ | $CH_3$ | 139-141°(Z) |
| 8.098 | $OCH_3$ | 2 | $CH_3$ | $CH_3$ | 148°(Z) |
| 8.099 | $CF_3$ | 2 | $CH_3$ | $CH_3$ | 159-160°(Z) |

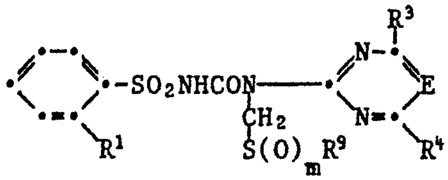

Tabelle 9

| No | $R^1$ | m | $R^9$ | $R^3$ | $R^4$ | E | Smp. |
|---|---|---|---|---|---|---|---|
| 9.001 | $COOCH_3$ | 0 | Pyrrolidino-thiocarbonyl | $CH_2OCH_3$ | $OCH_3$ | CH | |
| 9.002 | $COOCH_3$ | 0 | Pyrrolidino-thiocarbonyl | $OCH_2CH_3$ | $CH_3$ | CH | |
| 9.003 | $COOCH_3$ | 0 | Pyrrolidino-thiocarbonyl | Cl | $OCH_3$ | CH | |
| 9.004 | $COOCH_3$ | 0 | Pyrrolidino-thiocarbonyl | $N(CH_3)_2$ | $OCH_3$ | CH | |
| 9.005 | $COOCH_3$ | 0 | Pyrrolidino-thiocarbonyl | $CH(OCH_3)_2$ | $OCH_3$ | CH | |
| 9.006 | $COOCH_3$ | 0 | Pyrrolidino-thiocarbonyl | $CH_2Cl$ | $OCH_3$ | CH | |
| 9.007 | $COOCH_3$ | 0 | Dimethylthio-carbamoyl | $CH_2OCH_3$ | $OCH_3$ | CH | |
| 9.008 | $COOCH_3$ | 0 | Dimethylthio-carbamoyl | $OCH_2CH_3$ | $CH_3$ | CH | |
| 9.009 | $COOCH_3$ | 0 | Dimethylthio-carbamoyl | Cl | $OCH_3$ | CH | |
| 9.010 | $COOCH_3$ | 0 | Dimethylthio-carbamoyl | $N(CH_3)_2$ | $OCH_3$ | CH | |
| 9.011 | $COOCH_3$ | 0 | Dimethylthio-carbamoyl | $CH(OCH_3)$ | $OCH_3$ | CH | |
| 9.012 | $COOCH_3$ | 0 | Benzoyl | $CH_2OCH_3$ | $OCH_3$ | CH | |
| 9.013 | $COOCH_3$ | 0 | Benzoyl | $OCH_2CH_3$ | $CH_3$ | CH | |
| 9.014 | $COOCH_3$ | 0 | Benzoyl | Cl | $OCH_3$ | N | |

Tabelle 9 (Fortsetzung)

| No | $R^1$ | m | $R^9$ | $R^3$ | $R^4$ | E | Smp. |
|---|---|---|---|---|---|---|---|
| 9.015 | $COOCH_3$ | 0 | Benzoyl | $N(CH_3)_2$ | $OCH_3$ | N | |
| 9.016 | $COOCH_3$ | 0 | Benzoyl | $CH(OCH_3)_2$ | $OCH_3$ | CH | |
| 9.017 | $COOCH_3$ | 0 | Benzoyl | $CH_2Cl$ | $OCH_3$ | CH | |
| 9.018 | $COOCH_3$ | 0 | Acetyl | $CH_2OCH_3$ | $OCH_3$ | CH | |
| 9.019 | $COOCH_3$ | 0 | Acetyl | $OCH_2CH_3$ | $CH_3$ | CH | |
| 9.020 | $COOCH_3$ | 0 | Acetyl | Cl | $OCH_3$ | N | |
| 9.021 | $COOCH_3$ | 0 | Acetyl | $N(CH_3)_2$ | $OCH_3$ | N | |
| 9.022 | $COOCH_3$ | 0 | Acetyl | $CH(OCH_3)_2$ | $OCH_3$ | CH | |
| 9.023 | $COOCH_3$ | 0 | Acetyl | $CH_2Cl$ | $OCH_3$ | CH | |
| 9.024 | $OCHF_2$ | 0 | Pyrrolidino-thiocarbonyl | $CH_2OCH_3$ | $OCH_3$ | CH | |
| 9.025 | $OCHF_2$ | 0 | Pyrrolidino-thiocarbonyl | $OCH_2CH_3$ | $CH_3$ | CH | |
| 9.026 | $OCHF_2$ | 0 | Pyrrolidino-thiocarbonyl | Cl | $OCH_3$ | CH | 143-144° Zers. |
| 9.027 | $OCHF_2$ | 0 | Pyrrolidino-thiocarbonyl | $N(CH_3)_2$ | $OCH_3$ | CH | |
| 9.028 | $OCHF_2$ | 0 | Pyrrolidino-thiocarbonyl | $CH(OCH_3)_2$ | $OCH_3$ | CH | |
| 9.029 | $OCHF_2$ | 0 | Pyrrolidino-thiocarbonyl | $CH_2Cl$ | $OCH_3$ | CH | |
| 9.030 | $OCHF_2$ | 0 | Dimethylthio-carbamoyl | $CH_2OCH_3$ | $OCH_3$ | CH | |
| 9.031 | $OCHF_2$ | 0 | Dimethylthio-carbamoyl | $OCH_2CH_3$ | $CH_3$ | CH | |
| 9.032 | $OCHF_2$ | 0 | Dimethylthio-carbamoyl | Cl | $OCH_3$ | N | |
| 9.033 | $OCHF_2$ | 0 | Dimethylthio-carbamoyl | $N(CH_3)_2$ | $OCH_3$ | N | |

Tabelle 9 (Fortsetzung)

| No | $R^1$ | m | $R^9$ | $R^3$ | $R^4$ | E | Smp. |
|---|---|---|---|---|---|---|---|
| 9.034 | $OCHF_2$ | O | Dimethylthio-carbamoyl | $CH(OCH_3)$ | $OCH_3$ | CH | |
| 9.035 | $OCHF_2$ | O | Benzoyl | $CH_2OCH_3$ | $OCH_3$ | CH | |
| 9.036 | $OCHF_2$ | O | Benzoyl | $OCH_2CH_3$ | $CH_3$ | CH | |
| 9.037 | $OCHF_2$ | O | Benzoyl | Cl | $OCH_3$ | N | |
| 9.038 | $OCHF_2$ | O | Benzoyl | $N(CH_3)_2$ | $OCH_3$ | CH | |
| 9.039 | $OCHF_2$ | O | Benzoyl | $CH(OCH_3)_2$ | $OCH_3$ | CH | |
| 9.040 | $OCHF_2$ | O | Benzoyl | $CH_2Cl$ | $OCH_3$ | CH | |
| 9.041 | $OCHF_2$ | O | Acetyl | $CH_2OCH_3$ | $OCH_3$ | CH | |
| 9.042 | $OCHF_2$ | O | Acetyl | $OCH_2CH_3$ | $CH_3$ | CH | |
| 9.043 | $OCHF_2$ | O | Acetyl | Cl | $OCH_3$ | CH | |
| 9.044 | $OCHF_2$ | O | Acetyl | $N(CH_3)_2$ | $OCH_3$ | N | |
| 9.045 | $OCHF_2$ | O | Acetyl | $CH(OCH_3)_2$ | $OCH_3$ | CH | |
| 9.046 | $OCHF_2$ | O | Acetyl | $CH_2Cl$ | $OCH_3$ | CH | |
| 9.047 | Cl | O | Pyrrolidino-thiocarbonyl | $CH_2OCH_3$ | $OCH_3$ | CH | |
| 9.048 | Cl | O | Pyrrolidino-thiocarbonyl | $OCH_2CH_3$ | $CH_3$ | CH | |
| 9.049 | Cl | O | Pyrrolidino-thiocarbonyl | Cl | $OCH_3$ | CH | 141-143° Zers. |
| 9.050 | Cl | O | Pyrrolidino-thiocarbonyl | $N(CH_3)_2$ | $OCH_3$ | N | |
| 9.051 | Cl | O | Pyrrolidino-thiocarbonyl | $CH(OCH_3)_2$ | $OCH_3$ | CH | |
| 9.052 | Cl | O | Pyrrolidino-thiocarbonyl | $CH_2Cl$ | $OCH_3$ | CH | |
| 9.053 | Cl | O | Dimethylthio-carbamoyl | $CH_2OCH_3$ | $OCH_3$ | CH | |

Tabelle 9 (Fortsetzung)

| No | $R^1$ | m | $R^9$ | $R^3$ | $R^4$ | E | Smp. |
|---|---|---|---|---|---|---|---|
| 9.054 | Cl | 0 | Dimethylthio-carbamoyl | $OCH_2CH_3$ | $CH_3$ | CH | |
| 9.055 | Cl | 0 | Dimethylthio-carbamoyl | Cl | $OCH_3$ | CH | |
| 9.056 | Cl | 0 | Dimethylthio-carbamoyl | $N(CH_3)_2$ | $OCH_3$ | CH | |
| 9.057 | Cl | 0 | Dimethylthio-carbamoyl | $CH(OCH_3)_2$ | $OCH_3$ | CH | |
| 9.058 | Cl | 0 | Benzoyl | $CH_2OCH_3$ | $OCH_3$ | CH | |
| 9.059 | Cl | 0 | Benzoyl | $OCH_2CH_3$ | $CH_3$ | CH | |
| 9.060 | Cl | 0 | Benzoyl | Cl | $OCH_3$ | N | |
| 9.061 | Cl | 0 | Benzoyl | $N(CH_3)_2$ | $OCH_3$ | CH | |
| 9.062 | Cl | 0 | Benzoyl | $CH(OCH_3)_2$ | $OCH_3$ | CH | |
| 9.063 | Cl | 0 | Benzoyl | $CH_2Cl$ | $OCH_3$ | CH | |
| 9.064 | Cl | 0 | Acetyl | $CH_2OCH_3$ | $OCH_3$ | CH | |
| 9.065 | Cl | 0 | Acetyl | $OCH_2CH_3$ | $CH_3$ | CH | |
| 9.066 | Cl | 0 | Acetyl | Cl | $OCH_3$ | CH | |
| 9.067 | Cl | 0 | Acetyl | $N(CH_3)_2$ | $OCH_3$ | N | |
| 9.068 | Cl | 0 | Acetyl | $CH(OCH_3)_2$ | $OCH_3$ | CH | |
| 9.069 | Cl | 0 | Acetyl | $CH_2Cl$ | $OCH_3$ | CH | |
| 9.070 | $COOCH_3$ | 0 | 2-Chlorphenyl | $CH_2CH_3$ | $OCH_3$ | CH | |
| 9.071 | $COOCH_3$ | 2 | 2-Chlorphenyl | $CH_2CH_3$ | $OCH_3$ | CH | |
| 9.072 | $COOCH_3$ | 0 | 2-Chlorphenyl | Cl | $OCH_3$ | N | |
| 9.073 | $COOCH_3$ | 2 | 2-Chlorphenyl | Cl | $OCH_3$ | CH | |
| 9.074 | $COOCH_3$ | 0 | 2-Chlorphenyl | $N(CH_3)_2$ | $OCH_3$ | N | |

Tabelle 9 (Fortsetzung)

| No | $R^1$ | m | $R^9$ | $R^3$ | $R^4$ | E | Smp. |
|---|---|---|---|---|---|---|---|
| 9.075 | $COOCH_3$ | 2 | 2-Chlorphenyl | $N(CH_3)_2$ | $OCH_3$ | CH | |
| 9.076 | $COOCH_3$ | 0 | $CH_2CH_3$ | Cl | $OCH_3$ | N | |
| 9.077 | $COOCH_3$ | 2 | $CH_2CH_3$ | Cl | $OCH_3$ | CH | |
| 9.078 | $COOCH_3$ | 0 | $CH_2CH_3$ | $CH_2OCH_3$ | $OCH_3$ | CH | |
| 9.079 | $COOCH_3$ | 2 | $CH_2CH_3$ | $CH_2OCH_3$ | $OCH_3$ | CH | |
| 9.080 | $COOCH_3$ | 0 | p-Tolyl | $OCH_3$ | Cl | CH | |
| 9.081 | $COOCH_3$ | 2 | p-Tolyl | $OCH_3$ | Cl | N | |
| 9.082 | $COOCH_3$ | 0 | p-Tolyl | $CH_2OCH_3$ | $OCH_3$ | CH | |
| 9.083 | $COOCH_3$ | 2 | p-Tolyl | $CH_2OCH_3$ | $OCH_3$ | CH | |
| 9.084 | $OCHF_2$ | 0 | p-Tolyl | $CH_2OCH_3$ | $OCH_3$ | CH | |
| 9.085 | $OCHF_2$ | 2 | p-Tolyl | $CH_2OCH_3$ | $OCH_3$ | CH | 154-157° Zers. |
| 9.086 | $COOCH_3$ | 0 | Methyl | $CH_2OCH_3$ | $CH_3$ | CH | |
| 9.087 | $COOCH_3$ | 0 | Methyl | $OCH_2CH_3$ | $OCH_3$ | CH | |
| 9.088 | $COOCH_3$ | 0 | Methyl | Cl | $OCH_3$ | CH | |
| 9.089 | $COOCH_3$ | 0 | Methyl | $N(CH_3)_2$ | $OCH_3$ | CH | |
| 9.090 | $COOCH_3$ | 0 | Methyl | $CH(OCH_3)_2$ | $OCH_3$ | CH | |
| 9.091 | $COOCH_3$ | 0 | Methyl | $CH_2Cl$ | $OCH_3$ | CH | |
| 9.092 | $COOCH_3$ | 2 | Methyl | $CH_2OCH_3$ | $CH_3$ | CH | |
| 9.093 | $COOCH_3$ | 2 | Methyl | $OCH_2CH_3$ | $CH_3$ | CH | |
| 9.094 | $COOCH_3$ | 2 | Methyl | Cl | $OCH_3$ | N | |
| 9.095 | $COOCH_3$ | 2 | Methyl | $N(CH_3)_2$ | $OCH_3$ | N | |
| 9.096 | $COOCH_3$ | 2 | Methyl | $CH(OCH_3)$ | $OCH_3$ | CH | |
| 9.097 | $COOCH_3$ | 0 | 4-Chlorphenyl | $CH_2OCH_3$ | $CH_3$ | CH | |
| 9.098 | $COOCH_3$ | 0 | 4-Chlorphenyl | $OCH_2CH_3$ | $CH_3$ | CH | |

Tabelle 9 (Fortsetzung)

| No | $R^1$ | m | $R^9$ | $R^3$ | $R^4$ | E | Smp. |
|---|---|---|---|---|---|---|---|
| 9.099 | $COOCH_3$ | 0 | 4-Chlorphenyl | Cl | $OCH_3$ | N | |
| 9.100 | $COOCH_3$ | 0 | 4-Chlorphenyl | $N(CH_3)_2$ | $OCH_3$ | N | |
| 9.101 | $COOCH_3$ | 0 | 4-Chlorphenyl | $CH(OCH_3)_2$ | $OCH_3$ | CH | |
| 9.102 | $COOCH_3$ | 0 | 4-Chlorphenyl | $CH_2Cl$ | $OCH_3$ | CH | |
| 9.103 | $COOCH_3$ | 2 | 4-Chlorphenyl | $CH_2OCH_3$ | $CH_3$ | CH | |
| 9.104 | $COOCH_3$ | 2 | 4-Chlorphenyl | $OCH_2CH_3$ | $CH_3$ | CH | |
| 9.105 | $COOCH_3$ | 2 | 4-Chlorphenyl | Cl | $OCH_3$ | N | |
| 9.106 | $COOCH_3$ | 2 | 4-Chlorphenyl | $N(CH_3)_2$ | $OCH_3$ | N | |
| 9.107 | $COOCH_3$ | 2 | 4-Chlorphenyl | $CH(OCH_3)_2$ | $OCH_3$ | CH | |
| 9.108 | $COOCH_3$ | 2 | 4-Chlorphenyl | $CH_2Cl$ | $OCH_3$ | CH | |
| 9.109 | $OCHF_2$ | 0 | Methyl | $CH_2OCH_3$ | $CH_3$ | CH | |
| 9.110 | $OCHF_2$ | 0 | Methyl | $OCH_2CH_3$ | $OCH_3$ | CH | |
| 9.111 | $OCHF_2$ | 0 | Methyl | Cl | $OCH_3$ | N | |
| 9.112 | $OCHF_2$ | 0 | Methyl | $N(CH_3)_2$ | $OCH_3$ | CH | |
| 9.113 | $OCHF_2$ | 0 | Methyl | $CH(OCH_3)_2$ | $OCH_3$ | CH | |
| 9.114 | $OCHF_2$ | 0 | Methyl | $CH_2Cl$ | $OCH_3$ | CH | |
| 9.115 | $OCHF_2$ | 2 | Methyl | $CH_2OCH_3$ | $CH_3$ | CH | |
| 9.116 | $OCHF_2$ | 2 | Methyl | $OCH_2CH_3$ | $CH_3$ | CH | |
| 9.117 | $OCHF_2$ | 2 | Methyl | Cl | $OCH_3$ | N | |
| 9.118 | $OCHF_2$ | 2 | Methyl | $N(CH_3)_2$ | $OCH_3$ | N | |
| 9.119 | $OCHF_2$ | 2 | Methyl | $CH(OCH_3)$ | $OCH_3$ | CH | |
| 9.120 | $OCHF_2$ | 0 | 4-Chlorphenyl | $CH_2OCH_3$ | $CH_3$ | CH | |
| 9.121 | $OCHF_2$ | 0 | 4-Chlorphenyl | $OCH_2CH_3$ | $CH_3$ | CH | |
| 9.122 | $OCHF_2$ | 0 | 4-Chlorphenyl | Cl | $OCH_3$ | N | |

Tabelle 9 (Fortsetzung)

| No | $R^1$ | m | $R^9$ | $R^3$ | $R^4$ | E | Smp. |
|---|---|---|---|---|---|---|---|
| 9.123 | $OCHF_2$ | 0 | 4-Chlorphenyl | $N(CH_3)_2$ | $OCH_3$ | N | |
| 9.124 | $OCHF_2$ | 0 | 4-Chlorphenyl | $CH(OCH_3)_2$ | $OCH_3$ | CH | |
| 9.125 | $OCHF_2$ | 0 | 4-Chlorphenyl | $CH_2Cl$ | $OCH_3$ | CH | |
| 9.126 | $OCHF_2$ | 2 | 4-Chlorphenyl | $CH_2OCH_3$ | $CH_3$ | CH | |
| 9.127 | $OCHF_2$ | 2 | 4-Chlorphenyl | $OCH_2CH_3$ | $CH_3$ | CH | |
| 9.128 | $OCHF_2$ | 2 | 4-Chlorphenyl | Cl | $OCH_3$ | N | |
| 9.129 | $OCHF_2$ | 2 | 4-Chlorphenyl | $N(CH_3)_2$ | $OCH_3$ | N | |
| 9.130 | $OCHF_2$ | 2 | 4-Chlorphenyl | $CH(OCH_3)_2$ | $OCH_3$ | CH | |
| 9.131 | $OCHF_2$ | 2 | 4-Chlorphenyl | $CH_2Cl$ | $OCH_3$ | CH | |
| 9.132 | Cl | 0 | 4-Chlorphenyl | Cl | $OCH_3$ | CH | 142-144° |
| 9.133 | Cl | 2 | 4-Chlorphenyl | Cl | $OCH_3$ | CH | 140-142°(Z) |
| 9.134 | $COOC_2H_5$ | 0 | p-Tolyl | Cl | $OCH_3$ | CH | |
| 9.135 | $COOC_2H_5$ | 2 | p-Tolyl | Cl | $OCH_3$ | CH | |
| 9.136 | $COOCH_3$ | 0 | 2,4,5-Trichlorphenyl | $CH_2OCH_3$ | $OCH_3$ | CH | 128-130°(Z) |
| 9.137 | $COOCH_3$ | 0 | p-Tolyl | $N(CH_3)_2$ | $OCH_3$ | N | 138-140° |
| 9.138 | F | 0 | $CH_2COOCH_3$ | $N(CH_3)_2$ | $OCH_3$ | N | 130-133° |
| 9.139 | $NO_2$ | 0 | 2,4,5-Trichlorphenyl | $CH_2OCH_3$ | $OCH_3$ | CH | 118-120° |
| 9.140 | Cl | 0 | $CH_2COOCH_3$ | $N(CH_3)_2$ | $OCH_3$ | N | 135-138° |
| 9.141 | $COOCH_3$ | 0 | $CH_2COOCH_3$ | $N(CH_3)_2$ | $OCH_3$ | N | 145-148° |
| 9.142 | $CF_3$ | 2 | $CH_3$ | $N(CH_3)_2$ | $OCH_3$ | N | 171-175° (Z) |

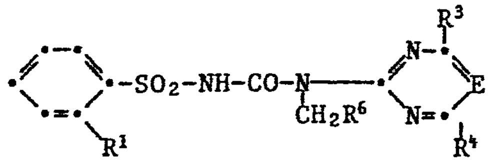

Tabelle 10

| No | $R^1$ | $R^6$ | $R^3$ | $R^4$ | E | Schmelzpunkt |
|---|---|---|---|---|---|---|
| 10.001 | $COOCH_3$ | Benztriazol-1-yl | $CH_3$ | $CH_3$ | CH | 178-180° |
| 10.002 | $COOCH_3$ | 1,2,4-Triazol-1-yl | $CH_3$ | $CH_3$ | CH | 164-167° |
| 10.003 | $COOCH_3$ | 2-Methylbenz-imidazol-1-yl | $CH_3$ | $CH_3$ | CH | |
| 10.004 | $COOCH_3$ | 2,3-Dimethylindol-1-yl | $CH_3$ | $CH_3$ | CH | . |
| 10.005 | Cl | Benztriazol-1-yl | $CH_3$ | $CH_3$ | CH | 122-128° Zers. |
| 10.006 | Cl | 1,2,4-Triazol-1-yl | $CH_3$ | $CH_3$ | CH | 152-157° Zers. |
| 10.007 | Cl | 2-Methylbenz-imidazol-1-yl | $CH_3$ | $CH_3$ | CH | |
| 10.008 | Cl | 2,3-Dimethylindol-1-yl | $CH_3$ | $CH_3$ | CH | |
| 10.009 | $OCHF_2$ | Benztriazol-1-yl | $CH_3$ | $CH_3$ | CH | |
| 10.010 | $OCHF_2$ | 1,2,4-Triazol-1-yl | $CH_3$ | $CH_3$ | CH | |
| 10.011 | $OCHF_2$ | 2-Methylbenz-imidazol-1-yl | $CH_3$ | $CH_3$ | CH | |
| 10.012 | $OCHF_2$ | 2,3-Dimethylindol-1-yl | $CH_3$ | $CH_3$ | CH | |
| 10.013 | $OCHF_2$ | Benztriazol-1-yl | $CH_3$ | $OCHF_2$ | CH | 109-111° |
| 10.014 | $COOCH_3$ | Benztriazol-1-yl | $OCH_3$ | $OCH_3$ | CH | |

Tabelle 10 (Fortsetzung)

| No | $R^1$ | $R^6$ | $R^3$ | $R^4$ | E | Schmelzpunkt |
|---|---|---|---|---|---|---|
| 10.015 | $COOCH_3$ | 1,2,4-Triazol-1-yl | $OCH_3$ | $OCH_3$ | CH | |
| 10.016 | $COOCH_3$ | 2-Methylbenz-imidazol-1-yl | $OCH_3$ | $OCH_3$ | CH | |
| 10.017 | $COOCH_3$ | 2,3-Dimethylindol-1-yl | $OCH_3$ | $OCH_3$ | CH | |
| 10.018 | Cl | Benztriazol-1-yl | $OCH_3$ | $OCH_3$ | CH | |
| 10.019 | Cl | 1,2,4-Triazol-1-yl | $OCH_3$ | $OCH_3$ | CH | |
| 10.020 | Cl | 2-Methylbenz-imidazol-1-yl | $OCH_3$ | $OCH_3$ | CH | |
| 10.021 | Cl | 2,3-Dimethylindol-1-yl | $OCH_3$ | $OCH_3$ | CH | |
| 10.022 | $OCHF_2$ | Benztriazol-1-yl | $OCH_3$ | $OCH_3$ | CH | 144-147° |
| 10.023 | $OCHF_2$ | 1,2,4-Triazol-1-yl | $OCH_3$ | $OCH_3$ | CH | |
| 10.024 | $OCHF_2$ | 2-Methylbenz-imidazol-1-yl | $OCH_3$ | $OCH_3$ | CH | |
| 10.025 | $OCHF_2$ | 2,3-Dimethylindol-1-yl | $OCH_3$ | $OCH_3$ | CH | |
| 10.026 | $OCHF_2$ | Benztriazol-1-yl | $OCH_3$ | $OCH_3$ | CH | |
| 10.027 | $COOCH_3$ | Benztriazol-1-yl | $OCH_3$ | $CH_3$ | CH | |
| 10.028 | $COOCH_3$ | 1,2,4-Triazol-1-yl | $OCH_3$ | $CH_3$ | CH | |
| 10.029 | $COOCH_3$ | 2-Methylbenz-imidazol-1-yl | $OCH_3$ | $CH_3$ | CH | |
| 10.030 | $COOCH_3$ | 2,3-Dimethylindol-1-yl | $OCH_3$ | $CH_3$ | CH | |
| 10.031 | Cl | Benztriazol-1-yl | $OCH_3$ | $CH_3$ | CH | |
| 10.032 | Cl | 1,2,4-Triazol-1-yl | $OCH_3$ | $CH_3$ | CH | |
| 10.033 | Cl | 2-Methylbenz-imidazol-1-yl | $OCH_3$ | $CH_3$ | CH | |

Tabelle 10 (Fortsetzung)

| No | $R^1$ ($R^2$) | $R^6$ | $R^3$ | $R^4$ | E | Schmelzpunkt |
|---|---|---|---|---|---|---|
| 10.034 | Cl | 2,3-Dimethylindol-1-yl | $OCH_3$ | $CH_3$ | CH | |
| 10.035 | $OCHF_2$ | Benztriazol-1-yl | $OCH_3$ | $CH_3$ | CH | 154-158° |
| 10.036 | $OCHF_2$ | 1,2,4-Triazol-1-yl | $OCH_3$ | $CH_3$ | CH | |
| 10.037 | $OCHF_2$ | 2-Methylbenz-imidazol-1-yl | $OCH_3$ | $CH_3$ | CH | |
| 10.038 | $OCHF_2$ | 2,3-Dimethylindol-1-yl | $OCH_3$ | $CH_3$ | CH | |
| 10.039 | $OCHF_2$ | Benztriazol-1-yl | $OCH_3$ | $OCHF_2$ | CH | |
| 10.040 | 2,6-$Cl_2$ | 1,2,4-Triazol-1-yl | $OCH_3$ | $OCH_3$ | CH | 156-159° |
| 10.041 | $COOCH_3$ | Benztriazol-1-yl | $OCH_3$ | $CH_2OCH_3$ | CH | 157-160° |
| 10.042 | Cl | 5(6)-Nitro-benz-triazol-1-yl (Isomeren-Gemisch) | $OCH_3$ | $OCH_3$ | CH | 152-155° |
| 10.043 | F | 5(6)-Nitro-benz-triazol-1-yl (Isomeren-Gemisch) | $OCH_3$ | $CH_3$ | CH | 147-149° |
| 10.044 | 2-Cl,6-$CH_3$ | 5(6)-Nitro-benz-triazol-1-yl (Isomeren-Gemisch) | $OCH_3$ | $CH_3$ | CH | 131-134° |

Formulierungsbeispiele:

Beispiel 14: Formulierungsbeispiele für Wirkstoffe der Formel I (% = Gewichtsprozent)

a) Spritzpulver

| | a) | b) | c) |
|---|---|---|---|
| Wirkstoff | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Aethylenglykol | 10 % | 10 % |
| Nonylphenolpolyäthylenglykoläther (15 MOl AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |

| | | |
|---|---|---|
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Biologische Beispiele:

Beispiel 15: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 xLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen, Spurenelemente enthaltenden Flüssigdüngers (®Greenzit ex. CIBA-GEIGY AG) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben

2-3: sehr starke Wirkung

4-6: mittlere Wirkung

7-8: schwache Wirkung

9: keine Wirkung (wie unbehandelte Kontrolle).

pre-emergente Wirkung:

Konzentration der Wirkstoffemulsion: 70,8 ppm

| Testpflanze / Wirkstoff Nr | Nasturtium | Stellaria | Agrostis | Digitaria |
|---|---|---|---|---|
| 5.001 | 2 | 2 | 2 | 2 |
| 5.004 | 1 | 1 | 1 | 1 |
| 5.007 | 1 | 1 | 1 | 1 |
| 5.022 | 2 | 2 | 2 | 2 |
| 5.051 | 2 | 2 | 2 | 2 |
| 5.061 | 2 | 2 | 2 | 2 |
| 5.064 | 1 | 1 | 1 | 1 |
| 5.066 | 1 | 1 | 1 | 1 |
| 5.089 | 2 | 1 | 1 | 2 |
| 5.102 | 2 | 1 | 1 | 2 |
| 5.105 | 1 | 1 | 1 | 1 |
| 5.107 | 1 | 1 | 1 | 1 |
| 5.121 | 2 | 2 | 2 | 2 |
| 5.124 | 2 | 2 | 2 | 2 |
| 5.130 | 2 | 2 | 2 | 2 |
| 5.133 | 2 | 2 | 2 | 2 |
| 5.142 | 2 | 2 | 2 | 2 |
| 5.165 | 1 | 2 | 1 | 2 |
| 5.170 | 2 | 2 | 2 | 2 |
| 5.229 | 2 | 2 | 2 | 2 |
| 5.230 | 2 | 2 | 2 | 2 |
| 5.231 | 2 | 2 | 2 | 2 |
| 5.232 | 2 | 1 | 1 | 2 |
| 5.235 | 2 | 2 | 2 | 2 |
| 5.236 | 2 | 1 | 1 | 2 |
| 5.237 | 2 | 2 | 2 | 2 |
| 5.238 | 2 | 1 | 2 | 2 |
| 5.239 | 2 | 1 | 1 | 1 |
| 5.240 | 2 | 2 | 2 | 2 |
| 5.241 | 2 | 2 | 2 | 2 |
| 5.242 | 2 | 2 | 2 | 2 |
| 5.243 | 2 | 3 | 2 | 3 |
| 5.244 | 2 | 3 | 2 | 3 |
| 5.246 | 3 | 4 | 3 | 5 |
| 5.247 | 3 | 5 | 3 | 5 |
| 5.252 | 2 | 2 | 2 | 2 |
| 6.001 | 2 | 2 | 2 | 2 |
| 6.010 | 1 | 1 | 1 | 1 |
| 6.011 | 1 | 1 | 1 | 1 |
| 6.013 | 2 | 2 | 2 | 2 |
| 6.021 | 2 | 2 | 2 | 2 |
| 6.040 | 2 | 2 | 2 | 2 |
| 6.041 | 2 | 2 | 2 | 2 |
| 6.061 | 2 | 1 | 2 | 1 |
| 6.230 | 2 | 2 | 3 | 3 |

| Testpflanze / Wirkstoff Nr | Nasturtium | Stellaria | Agrostis | Digitaria |
|---|---|---|---|---|
| 6.233 | 3 | 4 | 4 | 5 |
| 6.240 | 3 | 3 | 3 | 3 |
| 6.242 | 2 | 2 | 2 | 2 |
| 6.253 | 1 | 1 | 1 | 1 |
| 6.254 | 1 | 1 | 1 | 1 |
| 6.247 | 1 | 1 | 1 | 1 |
| 6.267 | 2 | 2 | 2 | 2 |
| 6.268 | 1 | 1 | 1 | 1 |
| 6.269 | 2 | 2 | 2 | 2 |
| 6.270 | 2 | 2 | 2 | 2 |
| 7.001 | 1 | 1 | 1 | 1 |
| 7.061 | 1 | 1 | 1 | 1 |
| 7.082 | 2 | 2 | 2 | 2 |
| 7.084 | 2 | 2 | 2 | 2 |
| 7.141 | 2 | 2 | 2 | 3 |
| 7.142 | 2 | 2 | 2 | 2 |
| 7.229 | 4 | 5 | 4 | 5 |
| 7.230 | 2 | 1 | 1 | 2 |
| 7.231 | 2 | 2 | 2 | 3 |
| 7.232 | 2 | 2 | 2 | 3 |
| 7.234 | 2 | 2 | 2 | 3 |
| 7.236 | 2 | 2 | 2 | 2 |
| 7.237 | 2 | 1 | 1 | 1 |
| 8.004 | 2 | 2 | 2 | 2 |
| 8.025 | 2 | 1 | 2 | 1 |
| 8.067 | 2 | 2 | 2 | 2 |
| 8.097 | 2 | 2 | 2 | 2 |
| 9.026 | 1 | 1 | 1 | 2 |
| 9.049 | 2 | 2 | 2 | 2 |
| 9.083 | 2 | 2 | 2 | 2 |
| 9.085 | 2 | 1 | 1 | 2 |
| 9.132 | 1 | 1 | 1 | 2 |
| 9.133 | 1 | 1 | 1 | 2 |
| 9.139 | 2 | 2 | 2 | 2 |
| 9.140 | 1 | 1 | 1 | 2 |
| 9.141 | 1 | 1 | 1 | 1 |
| 10.001 | 2 | 2 | 2 | 2 |
| 10.002 | 2 | 2 | 1 | 2 |
| 10.005 | 2 | 2 | 4 | 5 |
| 10.006 | 2 | 2 | 2 | 2 |
| 10.013 | 2 | 3 | 2 | 4 |
| 10.022 | 2 | 2 | 1 | 2 |
| 10.035 | 2 | 2 | 2 | 2 |
| 10.040 | 2 | 2 | 2 | 2 |
| 10.041 | 2 | 2 | 2 | 2 |
| 10.042 | 2 | 3 | 2 | 3 |
| 10.043 | 2 | 2 | 2 | 2 |
| 10.044 | 2 | 2 | 2 | 2 |

Beispiel 16: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 5-10 behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 17: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Dünger zugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die Wirkstoffe der Tabellen 5-10 eine merkliche Erhöhung der Anzahl und des Gewichts der Schoten im Haupttrieb.

Beispiel 18: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 2 Tage nach der Aussaat mit der wässrigen Spritzbrühe eines

Wirkstoffes der Tabellen 5-10 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 19: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 5-10 besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

Beispiel 20: Wurzelwachstum

Zur Bestimmung der Stimulierung des Wurzelwachstums werden Samen, welche durch Saatbeizung mit 45 - 500 mg Wirkstoff pro kg Saatgut gebeizt sind sowie zur Kontrolle auch unbehandelte Samen in Plastikzylindern von 5 cm Durchmesser und 30 cm Höhe angesät. Pro Zylinder kommen 10 Samen. Die Zylinder werden in Klimakammern unter kontrollierten Bedingungen gehalten. Die Bewertung des Versuches erfolgt 10 Tage nach der Aussaat, nachdem die Erde vorsichtig ausgewaschen wurde. Man misst die Wurzellänge und das Trockengewicht der Wurzeln und vergleicht mit den Werten, welche mit unbehandeltem Saatgut erzielt wurden.

Mit 100 und 200 mg Wirkstoff/pro kg gebeizte Weizensamen zeigten im Vergleich zu unbehandelten Keimlingen einen Längen- und Gewichtszuwachs der Wurzeln von ca. 10 %.

 

Patentansprüche

1. N-Sulfonyl-N'-pyrimidinyl und -triazinylharnstoffe der Formel I

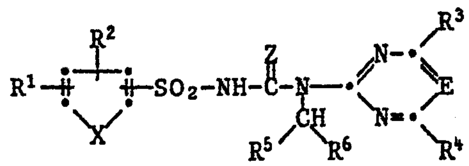

(I),

worin

E Stickstoff oder -CH=

X Sauerstoff, Schwefel, $-NR^7$, $-N=CR^7-$, -CH=CH- oder

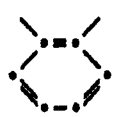

,

Y Sauerstoff oder einen Rest $S(O)_m$

m Null oder eine Zahl 1 bis 2,

Z Sauerstoff oder Schwefel,

$R^1$ Wasserstoff, Halogen, Nitro, $C_1$-$C_4$-Alkyl, $C_1$-$C_4$-Haloalkyl, $C_2$-$C_4$-Alkenyl, $C_2$-$C_4$-Haloalkenyl, $C_2$-$C_4$-Alkinyl, ein Aminorest $NR^{10}R^{11}$, ein Ketalrest $-CR^7(OC_1-C_4\text{-Alkyl})_2$ oder 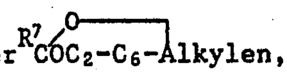, einen Rest Tetrahydrofuran-2-yloxy oder Tetrahydropyran-2-yloxy, $-OSO_2R^8$, $-COR^8$, $-\overset{R^7}{C}=N-OR^8$, $-SO_2NR^{10}R^{11}$, $SO_2R^8$, $YR^8$, Phenyl, Benzyl oder Phenoxy, wobei der Phenylkern unsubstituiert oder ein- oder mehrfach durch Halogen, Nitro, $C_1$-$C_4$-Alkyl, $C_1$-$C_4$-Halogenalkyl oder $C_1$-$C_4$-Alkoxy substituiert sein kann,

$R^2$ Wasserstoff, Halogen, $C_1$-$C_4$-Alkyl, $C_1$-$C_4$-Alkoxy, $C_1$-$C_4$-Halogenalkyl oder Nitro,

$R^3$ und $R^4$ unabhängig voneinander Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_2$-$C_4$-Alkoxyalkyl, $C_2$-$C_4$-Alkoxyalkoxy, Cyclopropyl, $NH_2$, $C_1$-$C_4$-Alkylamino, Di-$C_1$-$C_4$-alkylamino oder einen über das

Stickstoffatom gebundenen gesättigten 5- bis 7-gliedrigen Stickstoffheterocyclus, der noch ein weiteres Heteroatom enthalten kann, einen Rest

$-CH(OCH_3)_2$ oder 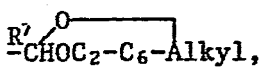,

$R^5$ Wasserstoff, $C_1$-$C_4$-Alkyl, $C_1$-$C_4$-Halogenalkyl, oder einen Phenyl-, Benzyl-, Furyl-, Thienyl- oder Naphthylrest, der unsubstituiert oder ein oder mehrfach durch Halogen, Nitro, $C_1$-$C_4$-Alkyl, $C_1$-$C_4$-Halogenalkyl oder $C_1$-$C_4$-Alkoxy, substituiert ist,

$R^6$ ist ein Rest $-S(O)_mR^9$ oder eine über ein Stickstoffatom gebundener 5-7 gliedriger heterocyclischer Rest, welcher noch weitere Heteroatome im Ring haben kann, benzanneliert sein kann und welcher unsubstituiert oder durch Nitro, Halogen und/oder $C_1$-$C_4$ Alkylreste ein- oder mehrfach substituiert sein kann

$R^7$ Wasserstoff, $C_1$-$C_4$-Alkyl, $C_1$-$C_4$-Halogenalkyl, $C_5$-$C_6$-Cycloalkyl, $C_4$-$C_7$-Cycloalkylalkyl, $C_2$-$C_4$-Alkoxyalkyl, $C_2$-$C_4$-Alkenyl, $C_2$-$C_4$-Halogenalkenyl $C_5$-$C_6$-Cycloalkenyl, $C_3$-$C_4$-Alkinyl, $C_1$-$C_4$-Cyanoalkyl, $C_1$-$C_4$-Alkyl-$NR^9R^{10}$ Benzyl oder durch Halogen substituiertes Benzyl,

$R^8$ dasselbe wie $R^7$ aber nicht Wasserstoff oder $C_1$-$C_4$ Alkoxy

$R^9$ einen $C_1$-$C_4$-Alkylrest, der durch Halogen, $C_2$-$C_4$-Alkoxy, Carboxyl, $C_1$-$C_4$-Alkylcarbonyl oder $-CONR^{10}R^{11}$ substituiert sein kann; einen Phenyl-, Benzyl- oder Naphthylrest, welcher einfach oder mehrfach durch Halogen, Nitro, Carboxyl, $C_1$-$C_4$-Alkyl, $C_1$-$C_4$-Halogenalkyl ,$C_1$-$C_4$-Alkoxy oder $C_1$-$C_4$-Halogenalkoxy substituiert sein kann, und in einem Rest $-SR^9$ kann $R^9$ auch einen Rest $-CZC_1$-$C_4$-Alkyl, $-CZOC_1$-$C_4$-Alkyl, $-CZNR^{10}R^{11}$ -CZ-Phenyl, CZ-Benzyl oder CZ-Naphthyl bedeuten, wobei diese Phenylring wie oben angegeben substituiert sein können,

$R^{10}$ und $R^{11}$ unabhängig voneinander je Wasserstoff $C_1$-$C_4$-Alkyl, $C_3$-$C_4$-Alkenyl, $C_3$-$C_4$-Alkinyl, $C_1$-$C_4$-Cyanoalkyl oder eines davon auch $C_1$-$C_4$-Alkoxy oder $C_2$-$C_4$-Alkoxyalkyl, Phenyl oder Benzyl

wobei der Phenylring ein- oder mehrfach durch Halogen, Nitro, $C_1$-$C_4$-Alkyl, $C_1$-$C_4$-Halogenalkyl oder $C_1$-$C_4$-Alkoxy substituiert sein kann,

$R^{10}$ und $R^{11}$ zusammen mit dem Stickstoffatom, an das sie gebunden sind auch einen 5-7 gliedrigen gesättigten oder teilweise gesättigten Heterocyclus, der im Ring noch ein Stickstoff oder Schwefel und/oder ein oder zwei weitere Stickstoffatome enthalten kann und der durch Halogen und/oder $C_1$-$C_4$-Alkyl substituiert sein kann

bedeuten, sowie deren Salze mit organischen oder anorganischen Basen.

2 . N-Sulfonyl-N'-pyrimidinyl- und -triazinyl-harnstoffe der Formel I, Anspruch 1, worin Z Sauerstoff bedeutet und E, $R^1$, $R^2$, $R^3$, $R^4$ ,$R^5$ und $R^6$ die in Anspruch 1 gegebene Bedeutung haben.

3. N-Sulfonyl-N'-pyrimidinyl- und -triazinyl-harnstoffe der Formel I, Anspruch 1, worin der Rest

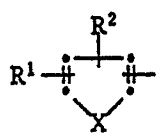 einen in ortho-Stellung durch $R^1$ substituierten Phenylrest bedeutet, während E, $R^3$, $R^4$, $R^5$, $R^6$ und Z die im Anspruch 1 gegebene Bedeutung haben.

4. N-Sulfonyl-N'-pyrimidinyl- und -triazinyl-harnstoffe der Formel I, Anspruch 1, in denen $R^5$ Wasserstoff und $R^6$ einen Rest $-SR^9$ bedeutet, während E, $R^1$, $R^2$, $R^3$, $R^4$, $R^9$ und Z die im Anspruch 1 gegebene Bedeutung haben.

5. N-Sulfonyl-N'-pyrimidinyl- und -triazinyl-harnstoffe gemäss Anspruch 1 der Formel Ia

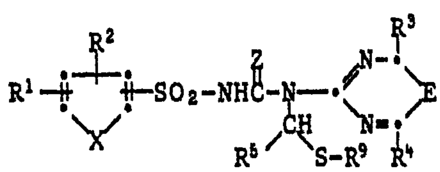

Ia

worin E, $R^1$, $R^2$, $R^3$, $R^4$, $R^5$, $R^9$ und Z die im Anspruch 1 gegebene Bedeutung haben.

6. N-Sulfonyl-N'-pyrimidinyl- und -triazinyl-harnstoffe der Formel I, Anspruch 1, worin $R^5$ Wasserstoff und $R^6$ einen Rest $-S(O)_nR^8$, darstellt, worin n die Zahl 1 oder 2 und E, $R^1$, $R^2$, $R^3$, $R^4$, $R^8$ und Z die im Anspruch 1 gegebene Bedeutung haben.

7. N-Sulfonyl-N'-pyrimidinyl- und -triazinyl-harnstoffe, Anspruch 1, der Formel Ib

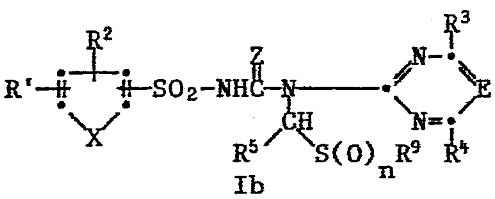

Ib

worin n die Zahl 1 oder 2 bedeutet, während E, $R^1$, $R^2$, $R^3$, $R^4$, $R^5$, $R^9$ und Z die im Anspruch 1 gegebene Bedeutung haben.

8. N-Sulfonyl-N'-pyrimidinyl- und -triazinyl-harnstoffe der Formel I, Anspruch 1, worin $R^5$ Wasserstoff und $R^6$ einen über das Stickstoffatom gebundenen 5-7 gliedrigen Heterocyclus bedeutet, welcher noch weitere Heteroatome im Ring enthalten kann, welcher benzanneliert sein kann und welcher unsubstituiert oder durch Nitro, Halogen und oder $C_1$-$C_4$-Alkylreste ein- oder mehrfach substituiert sein kann.

9. N-Sulfonyl-N'-pyrimidinyl- und -triazinyl-harnstoffe gemäss Anspruch 1 der Formel Ic

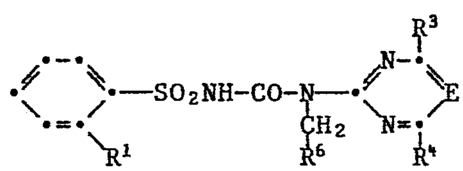

(Ic)

worin E, $R^1$, $R^3$, $R^4$ und $R^6$ die im Anspruch 1 gegebene Bedeutung haben.

10. N-Phenylsulfonyl-N'-pyrimidinyl- und -triazinyl-harnstoffe gemäss Anspruch 1 der Formel Id

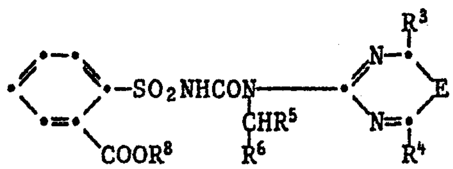

worin E, $R^3$, $R^4$, $R^5$, $R^6$ und $R^8$ die im Anspruch 1 gegebene Bedeutung haben.

11. N-(2-Chlorphenylsulfonyl)-N'-(4-chlorphenylthiomethyl)-N'-(4,6-dimethylpyrimidin-2-yl)-harnstoff gemäss Anspruch 1.

12. N-(2-Difluormethoxyphenylsulfonyl)-N'-(4-tolylsulfonylmethyl)-N'-(4-difluormethoxy-6-methylpyrimidin-2-yl)-harnstoff gemäss Anspruch 1.

13. N-(2-Chlorphenylsulfonyl)-N'-(4-tolylsulfonylmethyl)-N-(4,6-dimethylpyrimidin-2-yl)-harnstoff gemäss Anspruch 1.

14. N-(2-Methoxycarbonylphenylsulfonyl)-N-(4-tolylsulfonylmethyl)-N'-(4,6-dimethylpyrimidin-2-yl)-harnstoff gemäss Anspruch 1.

15. N-(2-Methoxycarbonylphenylsulfonyl)-N-(4-tolylsulfonylmethyl)-N'-(4-methoxy-6-methylpyrimidin-2-yl)-harnstoff gemäss Anspruch 1.

16. N-(2-Chlorphenylsulfonyl)-N'-(4-chlorphenylthiomethyl)-N'-(4,6-dimethylpyrimidin-2-yl)-harnstoff gemäss Anspruch 1.

17. N-(2-Methoxycarbonylphenylsulfonyl)-N'-(4-chlorphenylthiomethyl)-N'-(4,6-dimethylpyrimidin-2-yl)-harnstoff gemäss Anspruch 1.

18. N-(2-Difluormethoxyphenylsulfonyl)-N'-(naphthylthiomethyl)-N'-(4-methoxy-6-methylpyrimidin-2-yl)-harnstoff gemäss Anspruch 1.

19. N-(2-Chlorphenylsulfonyl)-N'-(pyrrolidinothiocarbonylthio-methyl)-N'-(4,6-dimethylpyrimidin-2-yl)-harnstoff gemäss Anspruch 1.

20. N-(2-Difluormethoxyphenylsulfonyl)-N'-(pyrrolidinothiocarbonyl-thio-methyl)-N'-(4,6-dimethylpyrimidin-2-yl)-harnstoff gemäss Anspruch 1.

21. N-(2-Chlorphenylsulfonyl)-N'-(1,3,4-triazol-1-ylmethyl)-N'-(2,4-dimethylpyrimidin-2-yl)-harnstoff gemäss Anspruch 1.

22. N-(2-Methoxycarbonylsulfonyl)-N'-(1,3,4-triazol-1-ylmethyl)-N'-(2,4-dimethylpyrimidin-2-yl)-harnstoff gemäss Anspruch 1.

23. N-(2-Chlorphenylsulfonyl)-N'-(benztriazol-1-ylmethyl)-N'-(2,4-dimethylpyrimidin-2-yl)-harnstoff gemäss Anspruch 1.

24. N-(2-Methoxycarbonylphenylsulfonyl)-N'-(benztriazol-1-yl-methyl)-N'-(2,4-dimethylpyrimidin-2-yl)-harnstoff gemäss Anspruch 1.

25. Verfahren zur Herstellung der N-Sulfonyl-N'-pyrimidinyl- und -triazinyl-harnstoffe der Formel I, Anspruch 1, dadurch gekennzeichnet, dass man ein Sulfonylisocyanat der Formel II

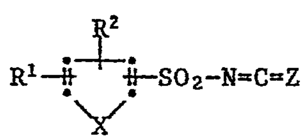

(II)

worin $R^1$, $R^2$, X und Z die unter Formel I gegebene Bedeutung haben, in einem inerten organischen Lösungs- oder Verdünnungsmittel, mit der äquivalenten Menge eines 2-Pyrimidin- oder 2-Triazinamines der Formel III umsetzt

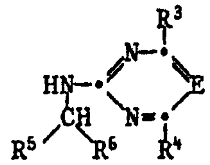

(III)

worin E, $R^3$, $R^4$, $R^5$ und $R^6$ die unter Formel I gegebene Bedeutung haben, und den Harnstoff gewünschtenfalls als Salz isoliert oder mit einer anorganischen oder organischen Base in ein Salz überführt.

26. Verfahren zur Herstellung der N-Sulfonyl-N'-pyrimidinyl- und -triazinyl-harnstoffe der Formel I, Anspruch 1, dadurch gekennzeichnet, dass man ein Sulfonylamid der Formel IV

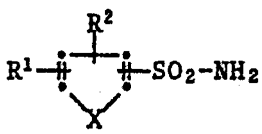

(IV)

worin $R^1$, $R^2$ und X die unter Formel I gegebene Bedeutung haben, in einem inerten organischen Lösungs- oder Verdünnungsmittel, in Gegenwart einer Base mit der äquivalenten Menge eines 2-Pyrimidinyl- oder 2-Triazinylcarbamoylhalides der Formel V umsetzt

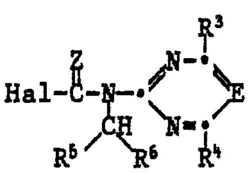

(V)

worin Hal für ein Halogenatom, vorzugsweise Chlor oder Brom steht und E, $R^3$, $R^4$, $R^5$, $R^6$ und Z die unter Formel I gegebene Bedeutung hat, und den Harnstoff gewünschtenfalls als Salz isoliert oder ihn mit einer anorganischen oder organischen Base in ein Salz überführt.

27. Verfahren zur Herstellung der N-Sulfonyl-N'-pyrimidinyl- und -triazinyl-harnstoffe der Formel I, Anspruch 1, dadurch gekennzeichnet, dass man einen Sulfonylharnstoff der Formel VI

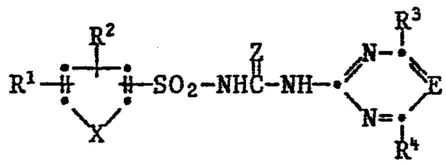 (VI)

worin E, $R^1$, $R^2$, $R^3$, $R^4$, X und Z die unter Formel I gegebene Bedeutung haben, in einem inerten organischen Lösungs- oder Verdünnungsmittel, in Gegenwart einer Base, mit der äquivalenten Menge eines Methylhalides der Formel VII umsetzt

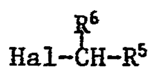 (VII)

worin Hal ein Halogenatom, vorzugsweise Chlor oder Brom bedeutet und $R^5$ und $R^6$ die unter Formel I gegebene Bedeutung haben, und den Harnstoff gewünschtenfalls als Salz isoliert oder ihn mit einer anorganischen oder organischen Base in ein Salz überführt.

28. Ein herbizides und den Pflanzenwuchs hemmendes Mittel, dadurch gekennzeichnet, dass es neben Träger- und/oder anderen Zuschlagstoffen als Wirkstoff mindestens einen substituierten N-Sulfonyl-N'-pyrimidinyl- oder -triazinyl-harnstoff der Formel I, Anspruch 1, oder ein Salz davon enthält.

29. Die Verwendung der Wirkstoffe der Formel I, gemäss Anspruch 1, oder sie enthaltender Mittel zur Bekämpfung unerwünschten Pflanzenwachstums.

30. Die Verwendung der Wirkstoffe der Formel I, gemäss Anspruch 1, oder sie enthaltender Mittel zur Hemmung des Pflanzenwachstums.

31. Die Verwendung der Wirkstoffe der Formel I, gemäss Anspruch 1, oder sie enthaltender Mittel zur Beeinflussung des Pflanzenwachstums zum Zweck der Ertragssteigerung.

32. Die Verwendung der Wirkstoffe der Formel I, gemäss Anspruch 1, oder sie enthaltender Mittel zur selektiven pre- oder post-emergenten Bekämpfung von Unkräutern in Nutzpflanzenkulturen.

33. Die Verwendung der Wirkstoffe der Formel I, gemäss Anspruch 1, oder sie enthaltender Mittel zur Unterdrückung des Pflanzenwachstums über das 2-Blattstadium hinaus, dadurch gekennzeichnet, dass die Wirkstoffe preemergent angewendet werden.

34. N-Pyrimidinyl- und triazinyl-N-thiomethylamine der Formel IIIa

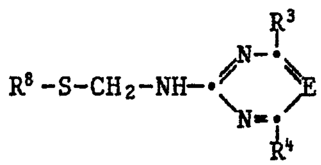

(IIIa)

als neue Zwischenprodukte, worin $R^3$, $R^4$ und $R^8$ die im Anspruch 1 gegebene Bedeutung haben.

35. N-Pyrimidinyl- und -triazinyl-N-sulfonylmethylamine der Formel IIIc

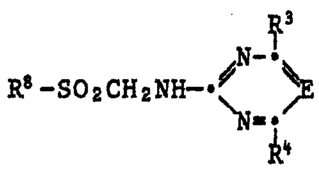

(IIIc)

als neue Zwischenprodukte, worin $R^3$, $R^4$ und $R^8$ die im Anspruch 1 gegebene Bedeutung haben.

36. N-Pyrimidinyl- und -triazinyl-N-heterocyclylaminomethylamine der Formel

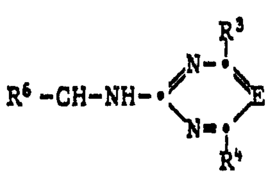

als Zwischenprodukt, worin $R^6$ eine über das Stickstoffatom gebundene 5-7 gliedrige heterocyclische Aminogruppe, welche noch weitere Heteroatome im Ring haben kann, benzanneliert sein kann und welche unsubstituiert oder durch Nitro, Halogen und/oder $C_1$-$C_4$ Alkylreste ein-oder mehrfach substituiert sein kann bedeuten, während $R^3$ und $R^4$ die im Anspruch 1 gegebene Bedeutung haben.

FO 7.5/NU/bg*